# EUROPEAN PATENT APPLICATION

(11) **EP 1 731 513 A1**
(43) Date of publication of application: **13.12.2006**
(21) Application number: 05727808.7
(22) Date of filing: 28.03.2005
(51) Int. Cl.: C07D 263/32, A61K 31/421, A61K 31/422, A61K 31/4245, A61K 31/427, A61K 31/4439, A61P 3/10, C07D 413/12, C07D 413/14, C07D 417/12, C07D 417/14

(54) **PHENOXYACETIC ACID DERIVATIVE AND MEDICINE CONTAINING THE SAME**

(30) Priority: 30.03.2004 JP 2004099201; 24.01.2005 JP 2005015954
(71) Applicant: DAIICHI PHARMACEUTICAL CO., LTD., Chuo-ku, Tokyo 103-8234 (JP)
(72) Inventor: KAGECHIKA, H., c/o DAIICHI PHARMACEUTICAL CO. LTD, Tokyo 1348630 (JP); SHIBATA Y., c/o DAIICHI PHARMACEUTICAL CO. LTD, Tokyo 1348630 (JP); OGURI, A., c/o DAIICHI PHARMACEUTICAL CO. LTD, Tokyo 1348630 (JP)
(74) Representative: Reitstötter - Kinzebach
(86) International application number: PCT/JP2005/005740
(87) International publication number: WO 2005/095364

(57) **Abstract**

Novel compounds having excellent PPAR α/γ agonist effects and having desirable properties for medicaments are provided.

Peroxisome proliferator-activated receptor α/γ agonists of the general formula (I): 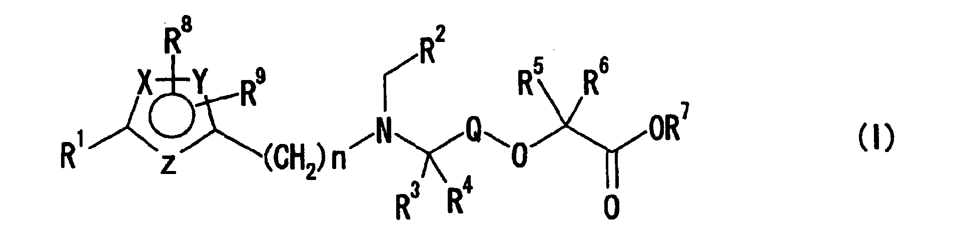 (wherein Q is an optionally-substituted benzene or pyridine ring; R¹ and R² each are an optionally-substituted phenyl group or 5- or 6-membered aromatic heterocyclic group; X Y, and Z are independently C, O, S or N; R³ to R⁹ each are a hydrogen atom, a lower alkyl group, etc.; n is an integer of 0 to 3).

## Description

### TECHNICAL FIELD

The present invention relates to a phenoxyacetic acid derivative, its salt and their solvates effective as a preventive/remedy for diabetes. More concretely, the invention relates to a peroxisome proliferator-activated receptor α/γ agonist (PPAR α/γ agonist).

### BACKGROUND ART

Diabetes is a disease to cause oneset and development of various acute or chronic complications such as typically ischemic heart diseases and cerebrovascular disorders, thereby bringing about an extreme disturbance in daily life. Accordingly, it is necessary to prevent the onset and development of such complications by early detection and strict blood sugar control.
Diabetes is grouped into type I diabetes of such that the production and secretion of insulin for blood sugar control is disordered, and type II diabetes of such that the production and secretion of insulin is within a normal range to a high level but the insulin sensitivity in the targeted organs and tissues is lowered (or that is, the insulin resistance is increased).
The insulin-targeted organs and tissues are principally muscles, adipose tissues and liver; and in muscles, insulin promotes intake of glucose and synthesis of glycogen, in adipose tissues, it promotes intake and consumption of glucose, and in liver, it inhibits gluconeogenesis and promotes synthesis of glycogen. In addition, not only controlling the glucose metabolism as above, but also insulin participates in lipid metabolism (promotion of lipid synthesis and inhibition of lipid degradation) in adipose tissues.

Recently, thiazolidinedione derivatives such as pioglitazone having the following structure have been developed as a drug for improving insulin resistance (Non-Patent Reference 1), and are widely used for treatment of type II diabetic patients, especially type II diabetic patients associated with obesity.

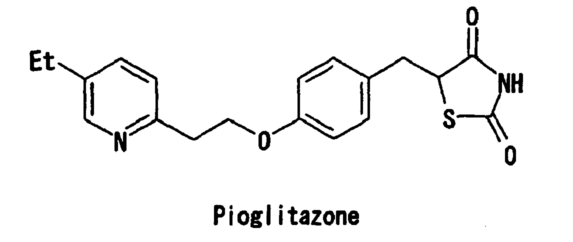

It has been revealed that these thiazolidinedione derivatives are an agonist for peroxisome proliferator-activated receptor γ (PPAR γ) (Non-Patent Reference 2). The mechanism of the PPAR γ to improve insulin resistance has not been yet fully clarified, but one promising theory for it is promotion of apoptosis of hypertrophic fat cells which produce and secrete free fatty acids that cause insulin resistance, and promotion of intake and storage of free fatty acids through promotion of differentiation from precursor fat cells to fat cells.

Pioglitazone, a PPAR γ agonist, has been administered specifically to patients associated with type II diabetes to achieve high therapeutic results, while body weight increase or fluid accumulation is observed in some patients (Non-Patent Reference 3). As so described hereinabove, since diabetes causes onset and development of complications such as ischemic heart diseases and cerebrovascular disorders, such body weight increase and fluid accumulation are not favorable. Recently, PPAR α/γ agonists obtained by adding PPAR α agonist effect to PPAR γ are actively studied, and in animal models, it is suggested that PPAR α/γ agonists exhibit more excellent properties as a remedy for diabetes than PPAR γ agonists. For example, in a test with db/db mice, it is shown that a PPAR α/γ agonist, KRP-297 significantly inhibits the body weight increase as compared with pioglitazone (Non-Patent Reference 4). In addition, it is shown that a PPAR α/γ agonist, LY465608 dose-dependently increases high-density lipoprotein (HDL) and lowers plasma triglyceride, thereby reducing the risk of ischemic heart diseases (Non-Patent Reference 5).
Typical PPAR α/γ agonists are the following compounds (Non-Patent References 6 and 7, and Patent References 1 and 2):

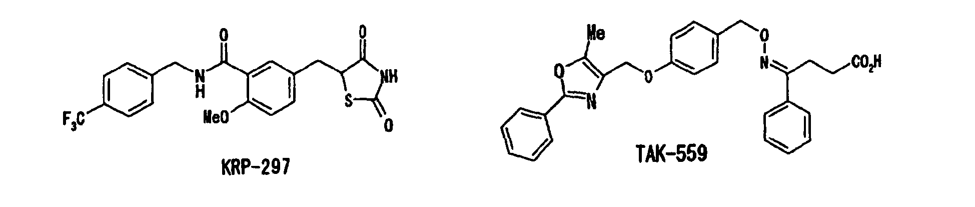

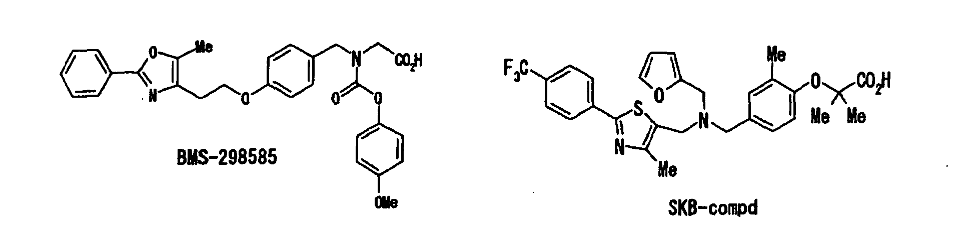

Non-Patent Reference 1: Chem. Pharm. Bull., 39, 1440-1445 (1991),
Non-Patent Reference 2: J. Biol. Chem., 270, 12953-12956 (1995),
Non-Patent Reference 3: Am. J. Med., 115 (8A), 111S-115S (2003),
Non-Patent Reference 4: Am. J. Physiol., 284, E966-E971 (2003),
Non-Patent Reference 5: Diabetes, 51, 1083-1087 (2002)
Non-Patent Reference 6: Bioorg. Med. Chem. Lett., 9, 533-538 (1999),
Non-Patent Reference 7: Chem. Pharm. Bull., 51, 138-151 (2003),
Patent Reference 1: WO2001-021602,
Patent Reference 2: WO2004-000785.

### Disclosure of the invention

### Problems that the Invention is to Solve

An object of the invention is to provide compounds which have different chemical structures from the above-mentioned known PPAR α/γ agonists and have excellent PPAR α/γ agonist effects and which have favorable properties for drugs.

### Means for Solving the Problems

The present inventors have variously studied and, as a result, have found that compounds of the following general formula (I) have excellent PPAR α/γ agonist effects and are useful as drugs for prevention/remedy of diabetes, and have completed the invention.

Specifically, the invention provides a compound of the general formula (I):

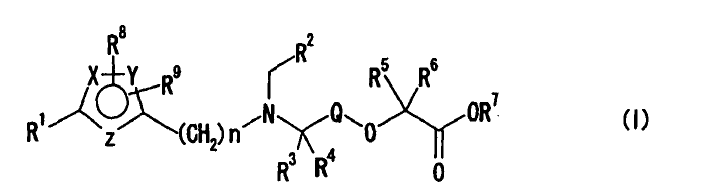

(wherein;
Q represents a benzene ring or a pyridine ring which may be substituted with one or two, the same or different groups selected from a hydroxyl group, a halogen atom, a lower alkenyl group, a lower alkoxy group, a substituted or unsubstituted lower alkyl group, a substituted or unsubstituted amino group, a pyrrolidinyl group, a piperidinyl group, a piperazinyl group, a morpholinyl group, a substituted or unsubstituted phenyl group and a substituted or unsubstituted pyridyl group;
R¹ represents a phenyl group, a pyridyl group, a pyrimidinyl group, a pyridazinyl group, a pyrazinyl group, a thienyl group, a furyl group, a pyrrolyl group, an imidazolyl group, a pyrazolyl group, a thiazolyl group, an oxazolyl group, an isothiazolyl group, an isoxazolyl group, an oxadiazolyl group or a triazolyl group, which may be substituted with one or two, the same or different groups selected from a halogen atom, a lower alkenyl group, a lower alkoxy group, a phenoxy group, a substituted or unsubstituted lower alkyl group and a substituted or unsubstituted amino group;
R² represents a pyridyl group, a pyrimidinyl group, a pyrazinyl group, a pyridazinyl group, an imidazolyl group, a pyrazolyl group, a thiazolyl group, an oxazolyl group, an isothiazolyl group, an isoxazolyl group, an oxadiazolyl group, a triazolyl group, a quinolyl group, an isoquinolyl group, a quinazolyl group, a cinnolyl group, a quinoxalyl group, a phthalazinyl group, a naphthyridinyl group, an indolyl group, a benzimidazolyl group, an indazolyl group, a benzothiazolyl group, a benzoxazolyl group, a benzisothiazolyl group, a benzisoxazolyl group or a benzotriazolyl group, which may be substituted with one or two, the same or different groups selected from a hydroxyl group, a halogen atom, a lower alkenyl group, a lower alkoxy group, a substituted or unsubstituted lower alkyl group, a substituted or unsubstituted amino group, a substituted or unsubstituted phenyl group and a substituted or unsubstituted pyridyl group, or represents a carbamoyl group which may be substituted with one or two, the same or different lower alkyl groups;
X, Y and Z each independently represent C, O, S or N (provided that at least any one of X, Y and Z is O, S or N);
R³ to R⁶ each independently represent a hydrogen atom or a lower alkyl group, or R³ and R⁴, or R⁵ and R⁶ may together form a 3- to 6-membered saturated ring along with the carbon atom substituted with them;
R⁷, R⁸ and R⁹ each independently represent a hydrogen atom or a lower alkyl group; n indicates an integer of from 0 to 3),
a salt thereof, or a solvate thereof.

The invention also provides a compound of the general formula (I) wherein the 5-membered ring containing X, Y and Z is an oxazole ring, a thiazole ring or an oxadiazole ring, a salt thereof, or a solvate thereof.
The invention also provides a compound of the general formula (I) wherein Q is a benzene ring which may be substituted with one or two, the same or different groups selected from a halogen atom, a lower alkenyl group, a lower alkoxy group and a substituted or unsubstituted lower alkyl group, a salt thereof, or a solvate thereof.
The invention also provides a compound of the general formula (I) wherein R¹ is a phenyl group which may be substituted with one or two, the same or different groups selected from a halogen atom and a substituted or unsubstituted lower alkyl group, a salt thereof, or a solvate thereof.
The invention also provides a compound of the general formula (1) wherein R² is a pyridyl group, an imidazolyl group, a pyrazolyl group, a thiazolyl group, an oxazolyl group, an isothiazolyl group, an isoxazolyl group, an oxadiazolyl group, a benzimidazolyl group, a benzothiazolyl group or a benzoxazolyl group which may be substituted with one or two, the same or different groups selected from a halogen atom, a lower alkoxy group, a substituted or unsubstituted lower alkyl group and a substituted or unsubstituted phenyl group, a salt thereof, or a solvate thereof.
The invention also provides a compound of the general formula (I) wherein R² is a carbamoyl group which may be substituted with one or two, the same or different lower alkyl groups, a salt thereof, or a solvate thereof.

The invention also provides a medicament comprising, as the active ingredient thereof, a compound of the general formula (I), a salt thereof, or a solvate thereof.
The invention also provides a drug composition comprising a compound of the general formula (I), a salt thereof, or a solvate thereof, and a pharmaceutically acceptable carrier.
The invention also provides use of a compound of the general formula (I), a salt thereof, or a solvate thereof for production of a medicament.
The invention also provides a method for treating insulin resistance-caused diseases, comprising administering an effective amount of a compound of the general formula (I), a salt thereof, or a solvate thereof.

### Effect of the Invention:

Compounds of the general formula (1) of the invention have excellent PPAR α/γ agonist effects and are useful as preventives/remedies for diabetes.

### Best Mode for Carrying out the Invention

Substituents in the general formula (I) are described below.

Halogen atom means a fluorine atom, a chlorine atom, a bromine atom and an iodine atom.
For the halogen atom, preferred are a fluorine atom and a chlorine atom.

Unsubstituted lower alkyl group means a linear, branched or cyclic alkyl group having from 1 to 6 carbon atoms, typically including, for example, a methyl group, an ethyl group, a propyl group, a butyl group, a pentyl group, a hexyl group, a 1-methylethyl group, a 1,1-dimethylethyl group, a 1-methylpropyl group, a 2-methylpropyl group, a 1,1-dimethylpropyl group, a 1,2-dimethylpropyl group, a 2,2-dimethylpropyl group, a 1-methylbutyl group, a 2-methylbutyl group, a 3-methylbutyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-methylpentyl group, a 2-methylpentyl group, a 3-methylpentyl group, a 4-methylpentyl group, a cyclopropyl group, a cyclobutyl group, a cyclopentyl group, a cyclohexyl group, a cyclopropylmethyl group, a cyclobutylmethyl group, and a cyclopentylmethyl group. Of those, preferred are a methyl group, an ethyl group and a propyl group.

Substituted lower alkyl group means a lower alkyl group substituted with from 1 to 3, the same or different groups selected from a hydroxyl group, a halogen atom, an amino group, an alkylamino group, a dialkylamino group, a lower alkoxy group, a carboxyl group, a lower alkoxycarbonyl group, a carbamoyl group, an alkylcarbamoyl group, a dialkylcarbamoyl group, a carbamoylamino group, an alkylcarbamoyl group, a dialkylcarbamoylamino group, an alkylsulfonylamino group, a lower alkoxycarbonylamino group and a lower alkanoylamino group; and typically it includes, for example, a trifluoromethyl group, a hydroxymethyl group, a 2-hydroxyethyl group, a 3-hydroxypropyl group, a 2-fluoroethyl group, a 2-chloroethyl group, a 3-fluoropropyl group, an aminomethyl group, a 2-aminoethyl group, a 3-aminopropyl group, a methylaminomethyl group, a 2-methylaminoethyl group, a 3-methylaminopropyl group, a dimethylaminomethyl group, a 2-dimethylaminoethyl group, a 3-dimethylaminopropyl group, a methoxymethyl group, a 2-methoxyethyl group, a 3-methoxypropyl group, a carboxymethyl group, a 2-carboxyethyl group, a 3-carboxypropyl group, a methoxycarbonylmethyl group, a 2-methoxycarbonylethyl group, a 3-methoxycarbonylpropyl group, a carbamoylmethyl group, a 2-carbamoylethyl group, a 3-carbamoylpropyl group, a methylcarbamoylmethyl group, a 2-methylcarbamoylethyl group, a 3-methylcarbamoylpropyl group, an ethylcarbamoylmethyl group, a 2-ethylcarbamoylethyl group, a 3-ethylcarbamoylpropyl group, a dimethylcarbamoylmethyl group, a 2-dimethylcarbamoylethyl group, a 3-dimethylcarbamoylpropyl group, a diethylcarbamoylmethyl group, a 2-diethylcarbamoylethyl group, a 3-diethylcarbamoylpropyl group, a carbamoylaminomethyl group, a 2-carbamoylaminoethyl group, a 3-carbamoylaminopropyl group, a methylcarbamoylaminomethyl group, a 2-methylcarbamoylaminoethyl group, a 3-methylcarbamoylaminopropyl group, an ethylcarbamoylaminomethyl group, a 2-ethylcarbamoylaminoethyl group, a 3-ethylcarbamoylaminopropyl group, a dimethylcarbamoylaminomethyl group, a 2-dimethylcarbamoylaminoethyl group, a 3-dimethylcarbamoylaminopropyl group, a diethylcarbamoylaminomethyl group, a 2-diethylcarbamoylaminoethyl group, a 3-diethylcarbamoylaminopropyl group, a methylsulfonylaminomethyl group, a 2-methylsulfonylaminoethyl group, a 3-methylsulfonylaminopropyl group, a methoxycarbonylaminomethyl group, a 2-methoxycarbonylaminoethyl group, a 3-methoxycarbonylaminopropyl group, an ethoxycarbonylaminomethyl group, a 2-ethoycarbonylaminoethyl group, a 3-ethoxycarbonylaminopropyl group, an acetylaminomethyl group, a 2-acetylaminoethyl group, and a 3-acetylaminopropyl group. Preferred are a trifluoromethyl group, a hydroxymethyl group, a 2-hydroxyethyl group, a 2-fluoroethyl group, a 2-chloroethyl group, an aminomethyl group, a 2-aminoethyl group, a methylaminomethyl group, a 2-methylaminoethyl group, a dimethylaminomethyl group, a 2-dimethylaminoethyl group, a methoxymethyl group, a 2-methoxyethyl group, a carboxymethyl group, a 2-carboxyethyl group, a methoxycarbonylmethyl group, a 2-methoxycarbonylethyl group, a carbamoylmethyl group, a 2-carbamoylethyl group, a methylcarbamoylmethyl group, a 2-methylcarbamoylmethyl group, an ethylcarbamoylmethyl group, a 2-ethylcarbamoylethyl group, a dimethylcarbamoylethyl group, a 2-dimethylcarbamoylethyl group, a diethylcarbamoylmethyl group, a 2-diethylcarbamoylethyl group, a carbamoylaminomethyl group, a 2-carbamoylaminoethyl group, a methylcarbamoylaminomethyl group, a 2-methylcarbamoylaminoethyl group, an ethylcarbamoylaminomethyl group, a 2-ethylcarbamoylaminoethyl group, a dimethylcarbamoylaminomethyl group, a 2-dimethylcarbamoylaminoethyl group, a diethylcarbamoylaminomethyl group, a 2-diethylcarbamoylaminoethyl group, a methylsulfonylaminomethyl group, a 2-methylsulfonylaminoethyl group, a methoxycarbonylaminomethyl group, a 2-methoxycarbonylaminoethyl group, an ethoxycarbonylaminomethyl group, a 2-ethoxycarbonylaminoethyl group, an acetylaminomethyl group, and a 2-acetylaminoethyl group; more preferred are a trifluoromethyl group, a hydroxymethyl group, a 2-hydroxyethyl group, a 2-fluoroethyl group, an aminomethyl group, a 2-aminoethyl group, a methylaminomethyl group, a 2-methylaminoethyl group, a dimethylaminomethyl group, a 2-dimethylaminoethyl group, a methoxymethyl group, a 2-methoxyethyl group, a carbamoylmethyl group, a 2-carbamoylethyl group, a methylcarbamoylmethyl group, a 2-methylcarbamoylethyl group, a dimethylcarbamoylmethyl group, a 2-dimethylcarbamoylethyl group, a diethylcarbamoylmethyl group, a carbamoylaminomethyl group, a methylcarbamoylaminomethyl group, an ethylcarbamoylaminomethyl group, a dimethylcarbamoylaminomethyl group, a diethylcarbamoylaminomethyl group and a methylsulfonylaminomethyl group; and especially preferred are a trifluoromethyl group, a hydroxymethyl group, a 2-hydroxyethyl group and a 2-fluoroethyl group.

Lower alkenyl group means a linear or branched alkenyl group having from 2 to 6 carbon atoms, typically including, for example, a vinyl group, an allyl group and a butenyl group.

Lower alkoxy group means a linear, branched or cyclic alkyl group having from 1 to 6 carbon atoms, typically including, for example, a methoxy group, an ethoxy group, a propoxy group, an isopropoxy group, a butoxy group, an isobutoxy group, a pentoxy group, and a cyclopentyloxy group. Of those, preferred are a methoxy group and an ethoxy group; and more preferred is a methoxy group.

Substituted amino group means an alkylamino group, a dialkylamino group, a lower alkoxycarbonylamino group, a carbamoylamino group, an alkylcarbamoylamino group, a dialkylcarbamoylamino group, an alkylsulfonylamino group, and a lower alkanoylamino group, typically including, for example, a methylamino group, an ethylamino group, a propylamino group, a butylamino group, a pentylamino group, a hexylamino group, a 1-methylethylamino group, a 1,1-dimethylethylamino group, a 1-methylpropylamino group, a dimethylamino group, a diethylamino group, a dipropylamino group, a dibutylamino group, a dipentylamino group, a dihexylamino group, a di(1-methylethyl)amino group, a methylethylamino group, a methoxycarbonylamino group, an ethoxycarbonylamino group, a methylcarbamoylamino group, an ethylcarbamoylamino group, a dimethylcarbamoylamino group, a diethylcarbamoylamino group, a methylsulfonylamino group, an ethylsulfonylamino group, an acetylamino group, and a propionylamino group. Above all, preferred are a methylamino group, an ethylamino group, a 1-methylethylamino group, a dimethylamino group, a diethylamino group, a dipropylamino group, a methylethylamino group, a methoxycarbonylamino group, an ethoxycarbonylamino group, a methylcarbamoylamino group, an ethylcarbamoylamino group, a dimethylcarbamoylamino group, a diethylcarbamoylamino group, a methylsulfonylamino group, an ethylsulfonylamino group, an acetylamino group.

Substituted phenyl group means a phenyl group substituted with one or two, the same or different groups selected from an alkyl group, a hydroxyl group, a halogen atom, an amino group, an alkylamino group, a dialkylamino group, a lower alkoxy group, a phenoxy group and a substituted or unsubstituted amino group. Examples of the mono-substituted phenyl group include a methylphenyl group, a trifluoromethylphenyl group, an ethylphenyl group, a hydroxyphenyl group, a fluorophenyl group, a chlorophenyl group, a bromophenyl group, an aminophenyl group, a methylaminophenyl group, an ethylaminophenyl group, a diethylaminophenyl group, a methoxyphenyl group, a phenoxyphenyl group, a methoxycarbonylaminophenyl group, a carbamoylaminophenyl group, a methylcarbamoylaminophenyl group, a dimethylcarbamoylaminophenyl group, a methylsulfonylaminophenyl group and an acetylaminophenyl group; and examples of the di-substituted phenyl group include a fluoro-methylphenyl group, a chloro-methylphenyl group, a fluoro-hydroxyphenyl group, a chloro-hydroxyphenyl group, a difluorophenyl group, a dichlorophenyl group, a chloro-fluorophenyl group, an aminofluorophenyl group, an aminochlorophenyl group, a fluoro-methylaminophenyl group, a chloro-methylaminophenyl group, a dimethylamino-fluorophenyl group, a dimethylamino-chlorophenyl group, a diethylamino-fluorophenyl group, a chloro-diethylaminophenyl group, a fluoro-methoxyphenyl group, a chloro-methoxyphenyl group, a fluoro-methoxycarbonylaminophenyl group, a chloro-methoxycarbonylaminophenyl group, a carbamoylamino-fluorophenyl group, a carbamoylamino-chlorophenyl group, a fluoro-methylcarbamoylaminophenyl group, a chloro-inethylcarbamoylaminophenyl group, a dimethylcarbamoylamino-fluorophenyl group, a chloro-dimethylcarbamoylaminophenyl group, a fluoro-methylsulfonylaminophenyl group, a chloro-methylsulfonylaminophenyl group, an acetylamino-fluorophenyl group and an acetylamino-chlorophenyl group. For the mono-substituted phenyl group, preferred are a methylphenyl group, a trifluoromethylphenyl group, a methoxyphenyl group, a phenoxyphenyl group, a fluorophenyl group, a chlorophenyl group, and a bromophenyl group; and more preferred are a methylphenyl group, a trifluoromethylphenyl group, a methoxyphenyl group, a phenoxyphenyl group, a fluorophenyl group and a chlorophenyl group. For the di-substituted phenyl group, preferred are a fluoro-methylphenyl group, a chloro-methylphenyl group, a difluorophenyl group, a dichlorophenyl group, a chloro-fluorophenyl group, a fluoro-methoxyphenyl group, and a chloro-methoxyphenyl group; and more preferred are a difluorophenyl group, a dichlorophenyl group, and a chloro-fluorophenyl group.

Substituted pyridyl group means one substituted with one or two, the same or different groups selected from an alkyl group, a hydroxyl group, a halogen atom, an amino group, an alkylamino group, a dialkylamino group, a lower alkoxy group, a phenoxy group, a substituted or unsubstituted amino group. Regarding its examples, examples of the mono-substituted pyridyl group include a methylpyridyl group, a trifluoromethylpyridyl group, an ethylpyridyl group, a hydroxypyridyl group, a fluoropyridyl group, a chloropyridyl group, a bromopyridyl group, an aminopyridyl group, a methylaminopyridiyl group, an ethylaminopyridyl group, a dimethylaminopyridyl group, a diethylaminopyridyl group, a methoxypyridyl group, a phenoxypyridyl group, a methoxycarbonylaminopyridyl group, a carbamoylaminopyridyl group, a methylcarbamoylaminopyridyl group, a dimethylcarbamylaminopyridyl group, a methylsulfonylaminopyridyl group, and an acetylaminopyridyl group; and examples of the di-substituted pyridyl group include a fluoro-methylpyridyl group, a chloro-methylpyridyl group, a fluoro-hydroxypyridyl group, a chloro-hydroxypyridyl group, a difluoropyridyl group, a dichloropyridyl group, a chloro-fluoropyridyl group, an aminofluoropyridyl group, an aminochloropyridyl group, a fluoro-methylaminopyridyl group, a chloro-methylaminopyridyl group, a dimethylamino-fluoropyridyl group, a dimethylamino-chloropyridyl group, a diethylamino-fluoropyridyl group, a chloro-diethylaminopyridyl group, a fluoro-methoxypyridyl group, a chloro-methoxypyridyl group, a fluoro-methoxycarbonylaminopyridyl group, a chloro-methoxycarbonylaminopyridyl group, a carbamoylamino-fluoropyridyl group, a carbamoylamino-chloropyridyl group, a fluoro-methylcarbamoylaminopyridyl group, a chloro-methylcarbamoylaminopyridyl group, a dimethylcarbamoylamino-fluoropyridyl group, a chloro-dimethylcarbamoylaminopyridyl group, a fluoro-methylsulfonylaminopyridyl group, a chloro-methylsulfonylaminopyridiyl group, an acetylamino-fluoropyridyl group, and an acetylamino-chloropyridyl group. For the mono-substituted pyridyl group, preferred are a methylpyridyl group, a trifluoromethylpyridyl group, a methoxypyridyl group, a phenoxypyridyl group, a fluoropyridyl group, a chloropyridyl group, and a bromopyridyl group; and more preferred are a methylpyridyl group, a trifluoromethylpyridyl group, a methoxypyridyl group, a phenoxypyridyl group, a fluoropyridyl group and chloropyridyl group. For the di-substituted pyridyl group, preferred are a fluoro-methylpyridyl group, a chloro-methylpyridyl group, a difluoropyridyl group, a dichloropyridyl group, a chloro-fluoropyridyl group, a fluoro-methoxypyridyl group, and a chloro-methoxypyridyl group; and more preferred are a difluoropyridyl group, a dichloropyridyl group, an a chloro-fluoropyridyl group.

R¹ and R² are described below.
R¹ is preferably a phenyl group, a pyridyl group or a thienyl group which may be substituted with one or two, the same or different groups selected from a halogen atom, a lower alkenyl group, a lower alkoxy group, a phenoxy group, a substituted or unsubstituted lower alkyl group and a substituted or unsubstituted amino group. Concretely, its preferred examples are a phenyl group, a fluorophenyl group, a chlorophenyl group, a bromophenyl group, a trifluoromethylphenyl group, a methylphenyl group, a methoxyphenyl group, a phenoxyphenyl group and a fluoro-methylphenyl group, and a methylthienyl group.

R² is preferably a pyridyl group, an imidazolyl group, an oxadiazolyl group, a pyrazolyl group, a thiazolyl group, an oxazolyl group, an isothiazolyl group, an isoxazolyl group, a benzimidazolyl group, an indazolyl group, a benzothiazolyl group, a benzoxazolyl group, a benzisothiazolyl group or a benzisoxazolyl group which may be substituted with one or two, the same or different groups selected from a hydroxyl group, a halogen atom, a lower alkenyl group, a lower alkoxy group, a substituted or unsubstituted lower alkyl group, a substituted or unsubstituted amino group, a substituted or unsubstituted phenyl group and a substituted or unsubstituted pyridyl group; or a carbamoyl group optionally substituted with one or two, the same or different lower alkyl groups. Concretely, its preferred examples are a pyridyl group, an imidazolyl group, a methylimidazolyl group, a pyrazolyl group, a methylpyrazolyl group, a thiazolyl group, a methylthiazolyl group, an oxazolyl group, a methyloxazolyl group, a methoxyoxazolyl group, a phenyloxazolyl group, a methyl-phenyloxazolyl group, a hydroxyoxadiazolyl group, a methyloxadiazolyl group, an isothiazolyl group, a methylisothiazolyl group, a dimethylisothiazolyl group, an isoxazolyl group, a methylisoxazolyl group, a dimethylisoxazolyl group, a benzimidazolyl group, a methylbenzimidazolyl group, an indazolyl group, a methylindazolyl group, a benzothiazolyl group, a benzoxazolyl group, a benzisothiazolyl group, a benzisoxazolyl group, a carbamoyl group a methylcarbamoyl group, an ethylcarbamoyl group, an isopropylcarbamoyl group, a butylcarbamoyl group, a cyclopropylcarbamoyl group, a cyclopropylmethylcarbamoyl group, a dimethylcarbamoyl group, a methyl-ethylcarbamoyl group and a diethylcarbamoyl group; and its more preferred examples are a pyridyl group, an imidazolyl group, a methylimidazolyl group, a thiazolyl group, a methylthiazolyl group, an oxazolyl group, a methyloxazolyl group, a methoxyoxazolyl group, a phenyloxazolyl group, a methyl-phenyloxazolyl group, a hydroxyoxadiazolyl group, a methyloxadiazolyl group, an isoxazolyl group, a methylisoxazolyl group, a dimethylisoxazolyl group, a benzimidazolyl group, a methylbenzimidazolyl group, a carbamoyl group, a methylcarbamoyl group, an ethylcarbamoyl group, an isopropylcarbamoyl group, a butylcarbamoyl group, a cyclopropylcarbamoyl group, a cyclopropylmethylcarbamoyl group, a dimethylcarbamoyl group, a diethylcarbamoyl group, and a methyl-ethylcarbamoyl group.

Q is described below.
Examples of Q are mono-substituted benzene rings such as a benzene ring, a hydroxybenzene ring, a fluorobenzene ring, a chlorobenzene ring, a bromobenzene ring, a vinylbenzene ring, an allylbenzene ring, a methoxybenzene ring, an ethoxybenzene ring, a methylbenzene ring, an ethylbenzene ring, a methoxymethylbenzene ring, an aminobenzene ring, a methylaminobenzene ring, a dimethylaminobenzene ring, a pyrrolidinylbenzene ring, a piperidinylbenzene ring, a piperazinylbenzene ring, a morpholinylbenzene ring, a phenylbenzene ring, a pyridylbenzene ring; di-substituted benzene rings such as a dimethylbenzene ring, a methyl-ethylbenzene ring, a methyl-methoxybenzene ring, a methoxy-fluorobenzene ring, a difluorobenzene ring, a dimethoxybenzene ring; and a pyridine ring, a hydroxypyridine ring, a fluoropyridine ring, a chloropyridine ring, a bromopyridine ring, a vinylpyridine ring, an allylpyridine ring, a methoxypyridine ring, an ethoxypyridine ring, a methylpyridine ring, an ethylpyridine ring, a methoxymethylpyridine ring, an aminopyridine ring, a methylaminopyridine ring, a dimethylaminopyridine ring, a pyrrolidinylpyridine ring, a piperidinylpyridine ring, a piperazinylpyridine ring, a morpholinylpyridine ring, a phenylpyridine ring, and a pyridylpyridine ring. Preferred examples are a benzene ring, a hydroxybenzene ring, a fluorobenzene ring, a chlorobenzene ring, an allylbenzene ring, a methoxybenzene ring, a methylbenzene ring, a phenylbenzene ring, a pyridylbenzene ring, a pyridine ring, a hydroxypyridine ring, a fluoropyridine ring, a chloropyridine ring, an allylpyridine ring, a methoxypyridine ring, and a methylpyridine ring. Of those, more preferred examples are a benzene ring, a fluorobenzene ring, a chlorobenzene ring, a bromobenzene ring, an allylbenzene ring, a methoxybenzene ring, a methylbenzene ring, a difluorobenzene ring, a dimethylbenzene ring, a methyl-ethylbenzene ring, a methyl-methoxybenzene ring, a methoxy-fluorobenzene ring, a dimethoxybenzene ring, and a pyridine ring. Among these, especially preferred are a benzene ring, an allylbenzene ring, a methoxybenzene ring, a methylbenzene ring, a dimethylbenzene ring, a methyl-ethylbenzene ring, a methyl-methoxybenzene ring, a dimethoxybenzene ring.

R³ to R⁹ and n are described below.
Preferably, R³ and R⁴ each are a hydrogen atom, a methyl group or an ethyl group, more preferably a hydrogen atom or a methyl group, even more preferably a hydrogen atom.
Preferably, R⁵ and R⁶ each are a hydrogen atom, a methyl group or an ethyl group. Also preferably, they form a 3- to 6- membered saturated ring along with the hydrogen atom to which R⁵ and R⁶ bond. More preferably, R⁵ and R⁶ each are a hydrogen atom or a methyl group, even more preferably a methyl group. Especially preferably, R⁵ and R⁶ are both methyl groups.
R⁷ is preferably a hydrogen atom, a methyl group, an ethyl group or a tert-butyl group.
Preferably, R⁸ and R⁹ each are a hydrogen atom, a methyl group or an ethyl group, more preferably a hydrogen atom or a methyl group.
n is an integer of from 0 to 3, preferably from 0 to 2, more preferably from 1 to 2.

The ring containing X, Y and Z in the general formula (I) means a 5-membered hetero ring, and its examples are a thiophene ring, a furan ring, a pyrrole ring, an imidazole ring, a pyrazole ring, a thiazole ring, an oxazole ring, an isothiazole ring, an isoxazole ring, an oxadiazole ring, and a triazole ring. When the 5-membered hetero ring is a thiazole ring, an oxazole ring, an isothiazole ring, an isoxazole ring or a triazole ring, then the hetero ring is not substituted with R⁹; and when the hetero ring is an oxadiazole ring, then, it is not substituted with R⁸ and R⁹.

Preferred examples of the 5-membered hetero ring substituted with R⁸ and R⁹ are a thiophene ring, a furan ring, a pyrrole ring, an imidazole ring, a pyrazole ring, a thiazole ring, an oxazole ring, an isothiazole ring, an isoxazole ring and a triazole ring that are substituted with one methyl or ethyl group; and a thiophene ring, a furan ring, a pyrrole ring, an imidazole ring and a pyrazole ring that are substituted with two methyl groups. Of those, more preferred are a thiophene ring, a furan ring, a pyrrole ring, an imidazole ring, a pyrazole ring, a thiazole ring, an oxazole ring, an isothiazole ring, an isoxazole ring and a triazole ring that are substituted with one methyl group; and even more preferred are a thiazole ring and an oxazole ring that are substituted with one methyl group. A preferred 5-membered hetero ring not substituted with R⁹ or with R⁸ and R⁹ is an oxadiazole ring.

The compounds of the general formula (I) of the invention include optical isomers resulting from stereoisomers or asymmetric carbon atom-derived optical isomers, and such stereoisomers, optical isomers and their mixtures are all within the scope of the invention.

Salts of the compounds of the general formula (I) of the invention are not specifically defined so far as they are pharmaceutically-acceptable salts. Concretely, they include mineral acid salts such as hydrochlorides, hydrobromides, hydroiodides, phosphates, nitrates and sulfates; benzoates; organic sulfonic acid salts such as methanesulfonates, 2-hydroxyethanesulfonates and p-toluenesulfonates; as well as organic carboxylic acid salts such as acetates, propanoates, oxalates, malonates, succinates, glutarates, adipates, tartrates, maleates, malates and mandelates.

In case where the compounds of the general formula (I) have an acidic group, then they may be in the form of salts with an alkali metal ion or an alkaline earth metal ion. The solvates are not specifically defined so far as they are pharmaceutically-acceptable ones, and concretely includes hydrates, ethanolates.

As so described hereinabove, PPAR γ agonists are known to have an unfavorable effect of body weight increase, etc. On the other hand, the compounds of the invention may prevent excessive body weight increase. The reason may be because the compounds of the invention have a strong agonist effect for both PPAR α receptor and PPAR γ receptor, therefore improving insulin resistance owing to its PPAR γ receptor-agonist effect and inhibiting excessive body weight increase owing to its PPAR α receptor-agonist effect. To that effect, the compounds of the invention have an excellent nature to be a remedy for diabetes.
In case where a compound having a low solubility in water is orally administered, then its absorption through gastrointestinal tract is generally low owing to the low solubility thereof. Even though the absorption of the compound through gastrointestinal tract is relatively high, there may be individual variation in the absorbability thereof in animals and humans administered with the compound owing to the low solubility thereof. In this point, the compounds of the invention are highly soluble in water under acidic to neutral conditions, and therefore they have an excellent nature as drugs.
Compounds having a high lipid solubility may often lack metabolic stability, and may have some undesirable influences on liver functions. However, since the compounds of the invention have a moderate lipid solubility, they may have an excellent nature as drugs.

Typical methods for producing the compounds of the invention are described below.
First described is a method for producing Ia and Ib-type compounds (where R² is an optionally-substituted pyridyl, pyrimidinyl, pyrazinyl, pyridazinyl, imidazolyl, pyrazolyl, thiazolyl, oxazolyl, isothiazolyl, isoxazolyl, oxadiazolyl, triazolyl, quinolyl, isoquinolyl, quinazolyl, cinnolyl, quinoxalyl, phthalazinyl, naphthyridinyl, indolyl, benzimidazolyl, indazolyl, benzothiazolyl, benzoxazolyl, benzisothiazolyl, benzisoxazolyl or benzotriazolyl group).

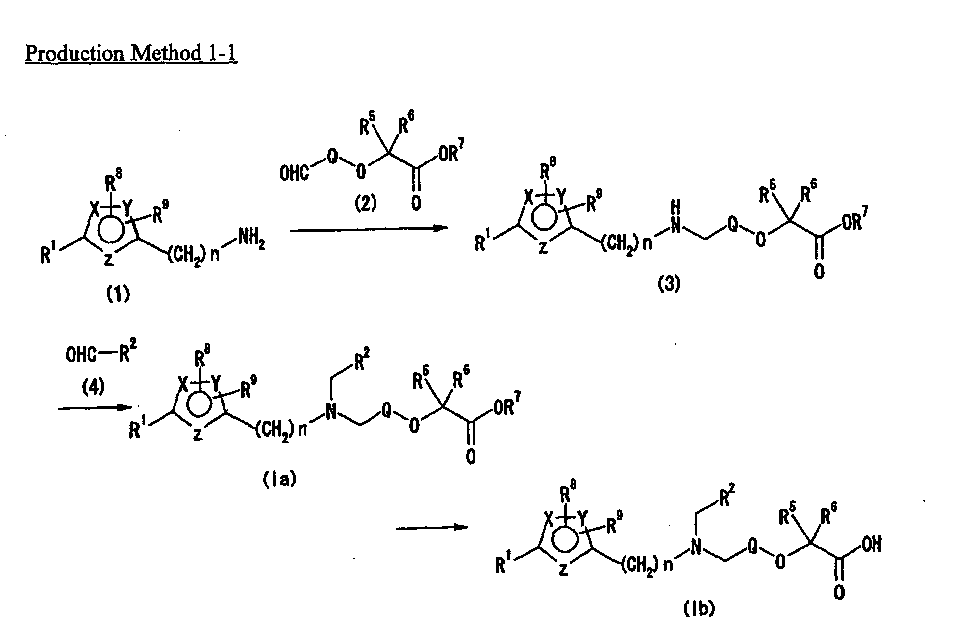

(In the above formulae, R¹, R², R⁵ to R⁹, Q, X, Y, Z and n have the same meanings as above.)

Compound 3 is produced by reacting compound 1 with aldehyde 2 in the presence of a reducing agent. In the presence or absence of an acid such as acetic acid, a Schiff base is formed from compound 1 and aldehyde 2, and then reacted with a reducing agent to obtain compound 3. In this case, compound 1 and aldehyde 2 may be dissolved in a solvent, and then may be reacted with a reducing agent without confirming the formation of a Schiff base to give compound 3. In general, an equimolar or excessive molar amount of aldehyde 2 is used relative to compound 1. The reducing agent may be a metal hydride complex such as sodium borohydride, sodium cyanoborohydride, sodium triacetoxyborohydride, and it may be used generally in an equimolar or excessive molar amount relative to compound 1. Preferably, from 3 mols to 5 mols of the reducing agent is used. The reaction solvent includes alcohols such as methanol, ethanol; ether solvents such as tetrahydrofuran; halogenoalkanes such as dichloromethane, chloroform. The reaction temperature may be from -20°C to the boiling point of the solvent used, preferably from 0°C to 50°C; and the reaction time may be from 15 minutes to 24 hours, preferably from 30 minutes to 10 hours or so.

Producing compound Ia from compound 3 may be attained by reacting compound 3 with aldehyde 4 in the presence of a reducing agent. In general, an equimolar or excessive molar amount of aldehyde 4 is used relative to compound 3. The reducing agent may be a metal hydride complex such as sodium borohydride, sodium cyanoborohydride, sodium triacetoxyborohydride, preferably sodium triacetoxyborohydride, and it may be used generally in an equimolar or excessive molar amount relative to compound 3. Preferably, from 2 mols to 3 mols of the reducing agent is used. The reaction solvent may be a halogenoalkane such as dichloromethane or chloroform. The reaction temperature may be from 0°C to 40°C, preferably from 0°C to 30°C; and the reaction time may be from 1 to 48 hours, preferably from 1 to 30 hours or so.

Though varying depending on the type of the ester, compound Ib may be produced from compound Ia according to a methods described in literature [see Protective Groups in Organic Synthesis), 2nd Ed., written by T. W. Green and P. G. M. Wuts, published by John Wiley & Son (1991)] or methods similar to them. Employable are a method of hydrolysis with acid or base, a method of hydrogenation in the presence of a catalyst such as palladium-carbon, or a method of using trifluoroacetic acid. For example, in a method of hydrolysis with a base, an equimolar or excess molar amount of a metal hydroxide such as lithium hydroxide or sodium hydroxide, or a carbonate such as sodium carbonate or potassium carbonate is reacted with compound Ia. The solvent includes alcohols such as methanol, ethanol; ether solvents such as tetrahydrofuran; water; and their mixed solvents. The reaction temperature may be from 0°C to 100°C, preferably from 0°C to 60°C. The reaction time may vary depending on the type of the ester, but may be generally from 1 hour to 72 hours, preferably from 1 hour to 24 hours or so. In case where R⁷ in compound Ia is a tert-butyl group, then the compound may be reacted with an acid such as trifluoroacetic acid or hydrochloric acid to give the intended compound. An excess molar amount of trifluoroacetic acid or hydrochloric acid may be used. The solvent includes dichloromethane and dioxane; the reaction temperature may be from 0°C to the boiling point of the solvent used, preferably from 0°C to 30°C; and the reaction time may be from 1 hour to 48 hours, preferably from 1 hour to 24 hours.

In the production method 1-1, compound Ia may be produced from compound 3 according to the following production method 1-2 where compound 5 is reacted with compound 3.

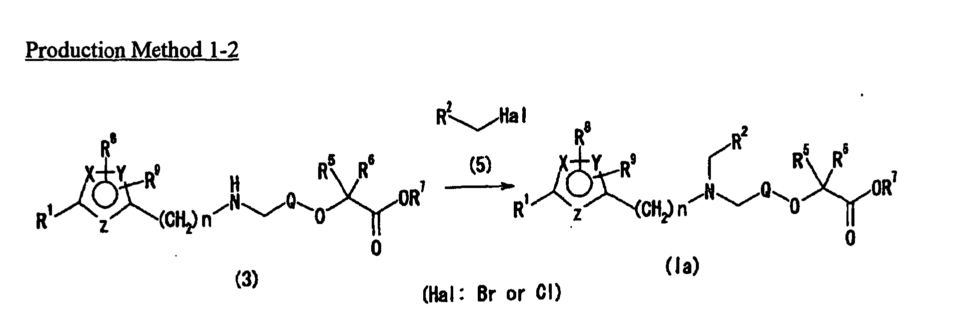

(In the formulae, R¹, R², R⁵ to R⁹, Q, X, Y, Z and n have the same meanings as above.)

For producing compound Ia from compound 3, compound 3 is reacted with an equimolar or excessive molar amount, preferably from an equimolar amount to 2 mols of compound 5 in the presence of a base, for which, if desired, a reaction promoter such as tetrabutylammonium iodide or potassium iodide may be used. The base includes tertiary amines such as triethylamine, and carbonates such as potassium carbonate, cesium carbonate, and its amount may be an equimolar to excessive molar amount. The solvent includes alcohols such as methanol, ethanol; ether solvents such as tetrahydrofuran; N,N-dimethylformamide, acetonitrile. The reaction temperature may be from 20°C to the boiling point of the solvent used, preferably from room temperature to 80°C; and the reaction time may be from 1 hour to 7 days, preferably from 1 hour to 48 hours or so.
For producing compound la, preferred is the production method 1-1.

Next described is a method for producing Ic and Id-type compounds (where R² is a carbamoyl group optionally substituted with 1 or 2 lower alkyl groups).

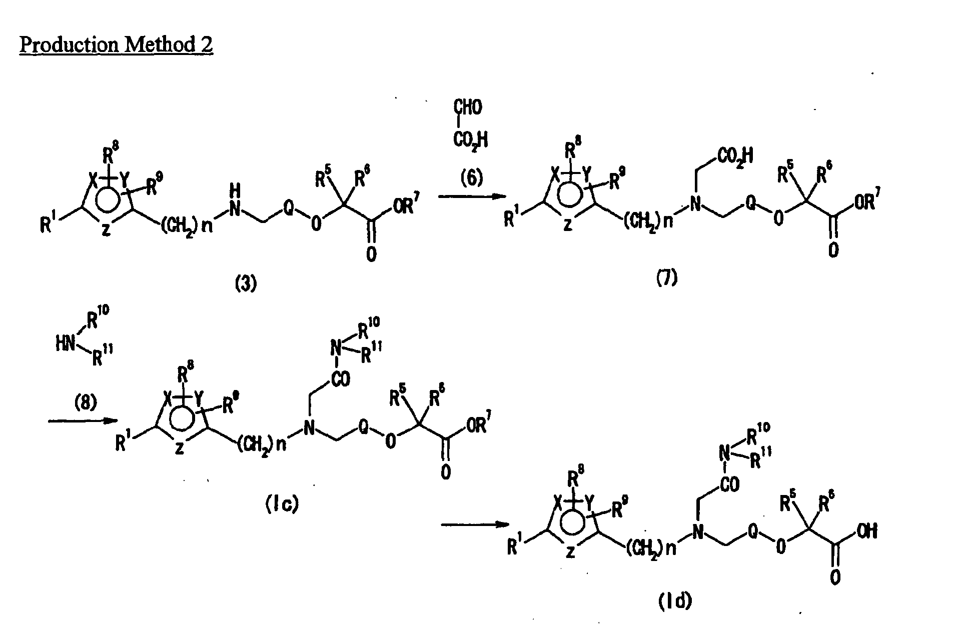

(In the formulae, R¹, R², R⁵ to R⁹, X, Y, Z and n have the same meanings as above; and R¹⁰ and R¹¹ each represent a hydrogen atom or a lower alkyl group.)

For producing compound 7 from compound 3, a metal hydride complex such as sodium triacetoxyborohydride, sodium borohydride or sodium cyanoborohydride, preferably sodium triacetoxyborohydride is reacted with compound 3 and glyoxylic acid 6. In general, an equimolar to excessive molar amount of glyoxylic acid is used relative to compound 3. The metal hydride complex is used generally in an equimolar to excessive molar amount, preferably in an amount of from 2 mols to 3 mols or so relative to compound 3. The reaction solvent may be an inert solvent such as tetrahydrofuran, dichloromethane, chloroform; the reaction temperature may be from 0°C to 40°C, preferably from 0°C to 30°C or so; and the reaction time may be from 1 hour to 48 hours, preferably from 1 hour to 10 hours or so.

For producing compound Ic from compound 7, amine 8 is reacted with compound 7 in the presence of a condensing agent. For example, an equimolar to excessive molar amount of amine 8 is reacted with compound 7 in an inert solvent at a temperature of from -50°C to the boiling point of the solvent used for the reaction, preferably from 0°C to 30°C, in the presence of a condensing agent. The reaction time may be from 10 minutes to 48 hours, preferably from 30 minutes to 12 hours or so. The condensing agent includes N,N'-dicyclohexylcarbodiimide, 1-ethyl-3-(3-dimethylaminopropyl)carbodiimide, diethyl cyanophosphate, benzotriazolyloxy-tris[pyrrolidino]-phosphonium hexafluorophosphate, 2-(1H-benzotriazol-1-yl)-1,1,3,3-tetramethyluronium tetrafluoroborate. It is used in an equimolar to excessive molar amount, preferably in an amount of from 1 to 5 mols relative to compound 7. The inert solvent includes dichloromethane, N-dimethylformamide, tetrahydrofuran, ethyl acetate, or their mixtures. If desired, the reaction may also be effected in the presence of a base such as triethylamine, diisopropylethylamine, N-methylmorpholine or 4-dimethylaminopyridine. Further, an N-hydroxy compound such as 1-hydroxybenzotriazole, N-hydroxysuccinimide, N-hydroxyphthalimide, or a phenol compound such as 4-nitrophenol, 2,4-dinitrophenol, 2,4,5-trichlorophenol, pentachlorophenol may also be added to the reaction system as a reaction promoter.

Compound Id may be produced from compound Ic in the same manner as in the production method 1-1 for producing compound Ib from compound Ia.

Compound 2 used in the production method 1-1 may be produced according to the following production method 3.

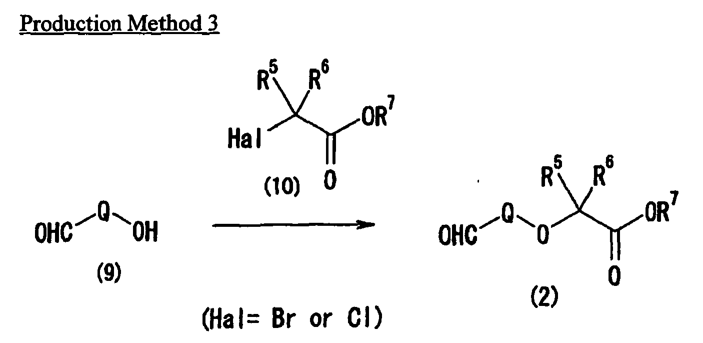

(In the formulae, R⁵ to R⁷, and Q have the same meanings as above.)

For producing compound 2, compound 10 is reacted with compound 9 in the presence of a base. Compound 10 is reacted with compound 9 in the presence of an excessive molar amount of a carbonate such as cesium carbonate, potassium carbonate, or tertiary amine such as triethylamine. The solvent may be an inert solvent such as N,N-dimethylformamide, dichloromethane. The reaction temperature may be from room temperature to the boiling point of the solvent used; and the reaction time may be from 1 hour to 3 days, preferably from 1 hour to 1 day or so.

For administering the compound of the invention as a preventive/remedy for diabetes, various modes such as oral administration may be employed. For oral administration, the compound may be in a free form or a salt thereof. In preparing the pharmaceutical composition containing the compound of the invention, suitable composition forms are selected in accordance with the administration mode for them, and they may be prepared in various methods generally employed for producing pharmaceutical preparations. In case where the compound of the general formula (I), its salt and their solvates of the invention are used in preparing pharmaceutical compositions, then the forms of the compositions may be oral preparations including, for example, tablets, pills, powders, granules, capsules. Above all, tablets are preferred. The solid preparations contain pharmaceutically-acceptable additives along with the active compound. For example, the additives may be suitably selected from fillers, expander, binders, disintegrators, dissolution promoters, moistening agents and lubricants, and mixed in pharmaceutical preparations.

The dose of the compound for administration as a preventive or remedy for diabetes is preferably from 0.1 mg to 1500 mg/case/day, more preferably from 1 mg to 500 mg/case/day.
The dose may be administered once a day, or it may be divided into two or three portions so as to be separately administered in a day.

### EXAMPLES

The invention is described with reference to the following Examples.

### [Reference Example 1]

### (1) 4-Azidomethyl-2-(3-bromophenyl)-5-methyloxazole

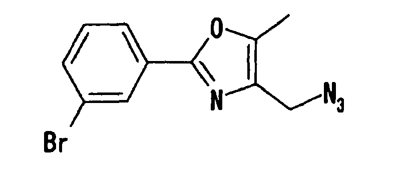

2-(3-Bromophenyl)-4-chloromethyl-5-methylphenyloxazole (5.0 g) was dissolved in dimethylformamide (30 ml), and sodium azide (1.71 g) and potassium iodide (2.17 g) were added thereto at room temperature, and the resulting solution was heated at 80°C and stirred for 7 hours.
After cooled to 0°C, water was added to the reaction solution, which was stirred. The precipitated solid was collected by filtration to obtain the entitled compound (5.3 g) as a pale yellow solid.

¹H-NMR (400 MHz, CDCl₃) δ :2.43 (3H, s), 4.28 (2H, s), 7.32 (1H, t, J=7.8 Hz), 7.54-7.58 (1H, m), 7.94 (1H, dd, J=7.9 Hz, 1.3 Hz), 8.16 (1H, s).
MS m/z: 293(M+H)⁺.

(2) tert-Butyl [2-(3-bromophenyl)-5-methyloxazol-4-ylmethyl]carbamate

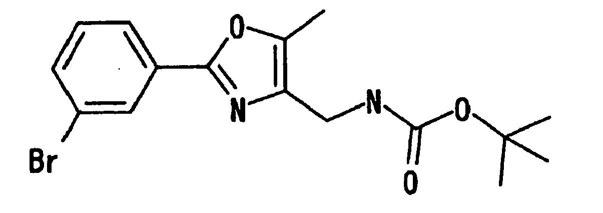

The compound (5.3 g ) of Reference Example 1-(1) was dissolved in tetrahydrofuran (150 ml), and triphenylphosphine (6.0 g) and water (1.0 ml) were added thereto at 0°C, and the resulting solution was stirred for a day at room temperature. The solvent was evaporated under reduced pressure, and the residue was dissolved in dichloromethane (100 ml), and di-tert-butyl dicarbonate (8.0 ml) and an aqueous saturated sodium bicarbonate solution (50 ml) were added thereto and the resulting solution was stirred at room temperature. After extraction with dichloromethane, the organic layer was washed with saturated brine, then dried over anhydrous sodium sulfate, and the solvent was evaporated under reduced pressure. The residue was purified by silica gel column chromatography (hexane-ethyl acetate) to obtain the entitled compound (6.0 g) as a colorless solid.

¹H-NMR (400 MHz, CDCl₃) δ:1.48 (9H, s), 2.41 (3H, s), 4.19 (2H, d, J=5.4 Hz), 7.31 (1H, t, J=8.0 Hz), 7.52-7.56 (1H, m), 7.8 8-7.92 (1H, m), 8.13 (1H, s).
MS m/z: 369(M+H)⁺.

(3) C-[2-(3-Bromophenyl)-5-methyloxazol-4-yl]methylamine

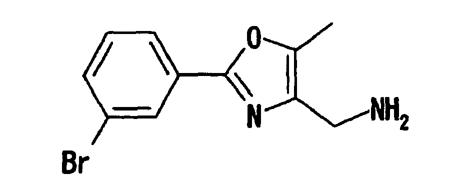

The compound (6.0 g) of Reference Example 1-(2) was dissolved in dichloromethane (100 ml), and trifluoroacetic acid (10 ml) was added thereto and the resulting solution was stirred for a day at room temperature. The solvent was evaporated under reduced pressure, and an aqueous saturated sodium bicarbonate solution was added to the residue, then extracted with ethyl acetate, and the organic layer was washed with saturated brine, then dried over anhydrous sodium sulfate, and the solvent was evaporated under reduced pressure to obtain the entitled compound (4.12 g) as a pale yellow solid.
MS m/z: 296(M+H)⁺.

### [Reference Example 2]

### tert-Butyl 2-(4-formylphenoxy)-2-methylpropionate

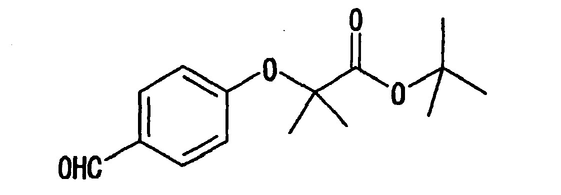

4-Hydroxybenzaldehyde (20 g) and tert-butyl 2-bromo-2-methylpropanoate (40 ml) were dissolved in dimethylformamide (10 ml), and cesium carbonate (100 g) and triethylamine (50 ml) were added thereto and the resulting solution was stirred for a day at 80°C. The reaction solution was diluted with ethyl acetate, then washed with water and saturated brine in that order, dried over anhydrous sodium sulfate, and the solvent was evaporated under reduced pressure. The residue was purified by silica gel column chromatography (hexane-ethyl acetate) to obtain the entitled compound (15.4 g) as a pale yellow oily substance.

¹H-NMR (400 MHz, CDCl₃) δ: 1.41 (9H, s), 1.64 (6H, s), 6.91 (2H, d, J=8.82 Hz), 7.78 (2H, d, J=8.82 Hz), 9.88 (1H, s).

### [Reference Example 3]

### tert-Butyl 2-[4-[[[2-(3-bromophenyl-5-methyloxazol-4-ylmethyl]amino]methyl]phenoxy]-2-methylpropionate

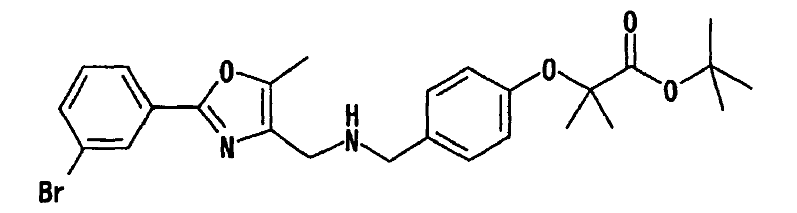

The compound (2.0 g) of Reference Example 1-(3) and the compound (2.4 g) of Reference Example 2 were dissolved in chloroform (30 ml) and the resulting solution was heated under reflux for 5 hours. The solvent was evaporated under reduced pressure, and the residue was dissolved in methanol (40 ml), then sodium borohydride (0.85 g) was added thereto at 0°C, and the solution was stirred for 1 hour, allowing the temperature to elevate to room temperature. The solvent was evaporated under reduced pressure, water was added thereto and the solution was extracted with ethyl acetate. The organic layer was washed with an aqueous saturated sodium bicarbonate solution and saturated brine in that order, then dried over anhydrous sodium sulfate, and the solvent was evaporated under reduced pressure. The residue was purified by silica gel column chromatography (chloroform-methanol) to obtain the entitled compound (4.21 g) as an yellow oily substance.

¹H-NMR (400 MHz, CDCl₃) δ: 1.44 (9H, s), 1.56 (6H, s), 2.32 (3H, s), 3.66 (2H, s), 3.75 (2H, s), 6.82 (2H, d, J=8.55 Hz), 7.20 (2H, d, J=8.79 Hz), 7.30 (1H, t, J=7.81 Hz), 7.52 (1H, d, J=8.06 Hz), 7.91 (1H, d, J=7.81 Hz), 8.15 (1H, s).
MS m/z: 517(M+H)⁺.

### [Reference Example 4]

### Ethyl 2-(4-formylphenoxy)-2-methylpropionate

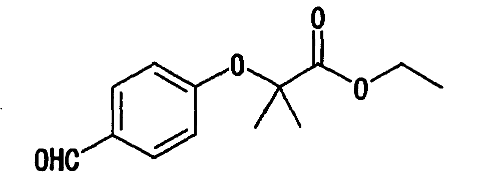

In the same manner as in Reference Example 2, the entitled compound (8.91 g) was obtained as a pale yellow oily substance from 4-hydroxybenzaldehyde (10 g) and ethyl 2-bromo-2-methylpropanoate (18 ml).

¹H-NMR (400 MHz, CDCl₃) δ: 1.21 (3H, t, J=7.1 Hz), 1.67 (6H, s), 4.23 (2H, q, J=7.1 Hz), 6.90 (2H, d, J=8.8 Hz), 7.79 (2H, d, J=8.8 Hz), 9.88 (1H, s).

### [Reference Example 5]

### Ethyl 2-4-[[[2-(3-bromophenyl)-5-methyloxazol-4-ylmethyl]amino]methyl]phenoxy]-2-methylpropionate

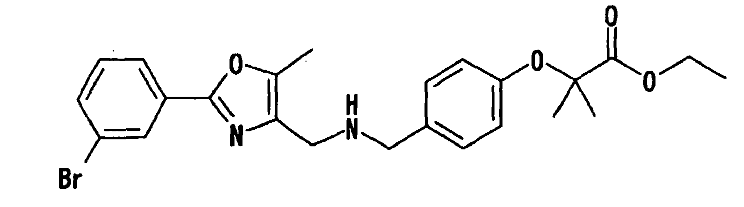

In the same manner as in Reference Example 3, the entitled compound (3.02 g) was obtained as an yellow oily substance from the compound (1.42 g) of Reference Example 1-(3) and the compound (1.51 g) of Reference Example 4.

¹H-NMR (400 MHz, CDCl₃) δ: 1.25 (3H, t, J=7.1 Hz), 1.58 (6H, s), 2.31 (3H, s), 3.66 (2H, s), 3.75 (2H, s), 4.24 (2H, q, J=7.2 Hz), 4.61 (1H, s), 6.81 (2H, d, J=8.8 Hz), 7.21 (2H, d, J=8.6 Hz), 7.30 (1H, t, J=7.8 Hz), 7.51-7.55 (1H, m), 7.89-7.93 (1H, m), 8.15 (1H, s). MS m/z: 489(M+H)⁺.

### [Reference Example 6]

### tert-Butyl 2-[4-[[[2-(3-bromophenyl)-5-methyloxazol-4-ylmethyl]carboxymethylamino]methyl]phenoxy]-2-methylpropionate

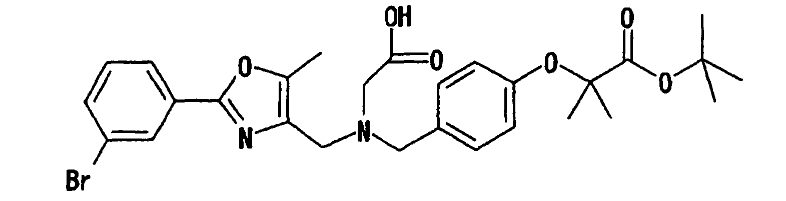

The compound (3.0 g) of Reference Example 3 and glyoxylic acid (696 mg) were dissolved in tetrahydrofuran (30 ml), and sodium triacetoxyborohydride (2.47 g) was added thereto and the resulting solution was stirred for a day. The solvent was evaporated under reduced pressure, the residue was diluted with ethyl acetate, then washed with water and saturated brine in that order, dried over anhydrous sodium sulfate, and the solvent was evaporated under reduced pressure. The residue was purified by silica gel column chromatography (chloroform-methanol) and recrystallized from acetone-hexane to obtain the entitled compound (2.1 g) as a colorless solid.

¹H-NMR (400 MHz, CDCl₃) δ: 1.43 (9H, s), 1.56 (6H, s), 2.44 (3H, s), 3.59 (2H, s), 4.24 (2H, s), 4.37 (2H, s), 6.89 (2H, d, J=8.6 Hz), 7.43 (1H, t, J=8.0 Hz), 7.51 (2H, d, J=8.6 Hz), 7.65 (1H, d, J=6.8 Hz), 7.98 (1H, d, J=7.8 Hz), 8.16 (1H, s).
MS m/z: 575(M+H)⁺.

### [Reference Example 7]

### tert-Butyl 2-(2-allyl-4-formylphenoxy)-2-methylpropanoate

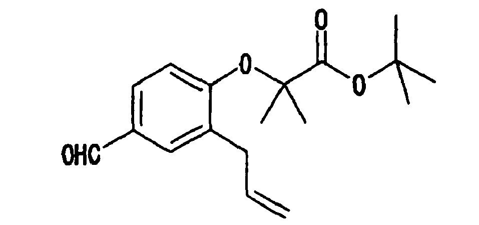

In the same manner as in Reference Example 2, the entitled compound (3.6 g) was obtained as an oily substance from 3-allyl-4-hydroxybenzaldehyde (5.0 g) and tert-butyl 2-bromo-2-methylpropanoate (10 ml).

¹H-NMR (400 MHz, CDCl₃) δ: 1.39 (9H, s), 1.68 (6H, s), 3.43 (2H, d, J=6.6 Hz), 5.05-5.13 (2H, m), 5.93-6.05 (1H, m), 6.76 (1H, d, J=8.3 Hz), 7.64 (1H, dd, J=2.2, 8.3 Hz), 7.70 (1H, d, J=2.2 Hz), 9.87 (1H, s).

### [Reference Example 8]

### tert-Butyl 2-[2-allyl-4-[[(5-methyl-2-phenyloxazol-4-ylmethyl)amino]methyl]phenoxy]-2-methylpropanoate

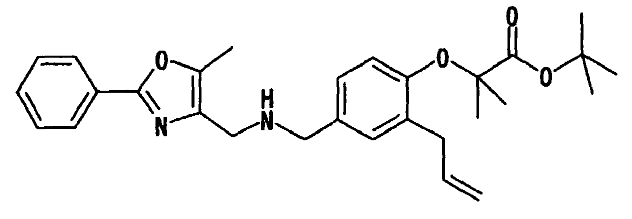

In the same manner as in Reference Example 3, the entitled compound (420.1 mg) was obtained as an oily substance from the compound (304.4 mg) of Reference Example 7 and 5-methyl-2-phenyloxazol-4-ylmethylamine (188.2 mg).

¹H-NMR (400 MHz, CDCl₃) δ: 1.39 (9H, s), 1.56 (6H, s), 1.55-1.70 (1H, brs), 2.30 (3H, s), 3.35 (2H, d, J=6.6 Hz), 3.62 (2H, s), 3.75 (2H, s), 4.97-5.01 (2H, m), 5.85-6.00 (1H, m), 6.65 (1H, d, J=8.5 Hz), 7.02 (1H, dd, J=2.2, 8.5 Hz), 7.07 (1H, d, J=8.5 Hz), 7.40-7.50 (3H, m), 7.97-8.03 (2H, m).

### [Reference Example 9]

### tert-Butyl 2-[2-allyl-4-[[carboxymethyl-(5-methyl-2-phenyloxazol-4-ylmethyl)amino]methyl]phenoxy]-2-methylpropanoate

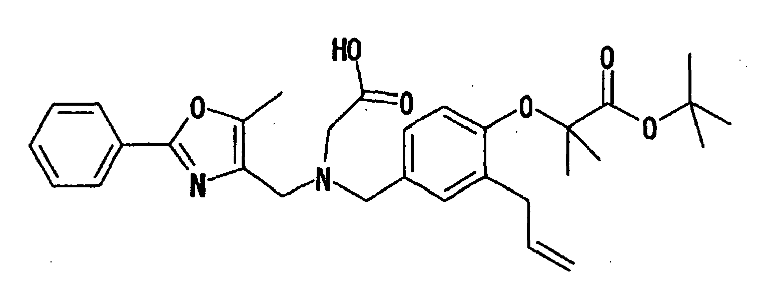

In the same manner as in Reference Example 6, the entitled compound was obtained from the compound (420.1 mg) of Reference Example 8 and glyoxylic acid (81.0 mg), and this was directly used in the next reaction as it was.

### [Reference Example 10]

### tert-Butyl 2-[2-allyl-4-[[[5-methyl-2-(3-bromophenyl)oxazol-4-ylmethyl]amino]methyl]phenoxy]-2-methylpropanoate

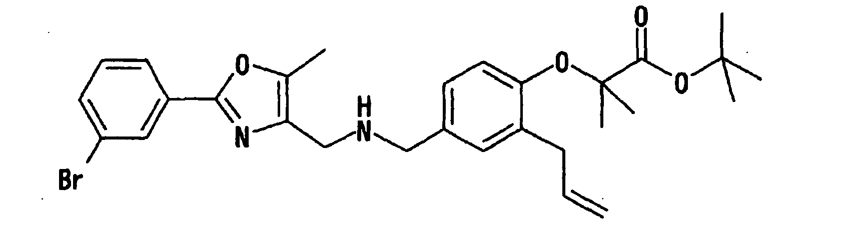

In the same manner as in Reference Example 3, the entitled compound (212.0 mg) was obtained as an oily substance from the compound (304.4 m) of Reference Example 7 and the compound (267.1 mg) of Reference Example 1-(3).

¹H-NMR (400 MHz, CDCl₃) δ: 1.39 (9H, s), 1.56 (6H, s), 1.55-1.70 (1H, brs), 2.30 (3H, s), 3.35 (2H, d, J=6.6 Hz), 3.62 (2H, s), 3.75 (2H, s), 4.97-5.01 (2H, m), 5.85-6.00 (1H, m), 6.65 (1H, d, J=8.5 Hz), 7.02 (1H, dd, J=2.2, 8.5 Hz), 7.07 (1H, d, J=8.5 Hz), 7.25-7.30 (1H, m), 7.50-7.60 (1H, m), 7.90-8.00 (1H, m), 8.15-8.17 (1H, m).

### [Reference Example 11]

### tert-Butyl 2-[2-allyl-4-[[carboxymethyl-[5-methyl-2-(3-bromophenyl)oxazol-4-ylmethyl]amino]methyl]phenoxy-2-methylpropanoate

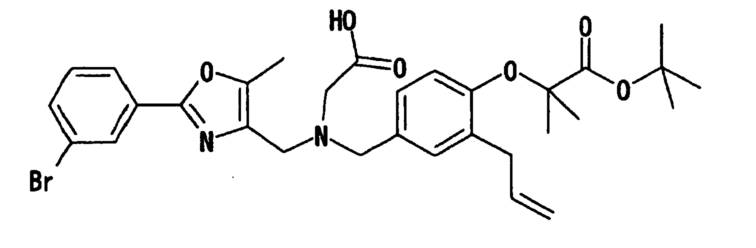

In the same manner as in Reference Example 6, the entitled compound was obtained from the compound of Reference Example 10 and glyoxylic acid (81.0 mg), and this was directly used in the next reaction as it was.

### [Reference Example 12]

### Ethyl 2-(4-formyl-2,6-dimethylphenoxy)-2-methylpropanoate

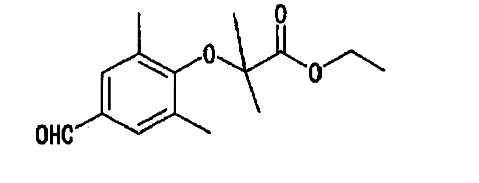

3,5-Dimethyl-4-hydroxybenzaldehyde (5.0 g) was dissolved in N,N-dimethylformamide (20 ml), and cesium carbonate (10 g) and ethyl 2-bromo-2-methylpropanoate (10 ml) were added thereto and the resulting solution was stirred at 80°C for 12 hours. The temperature was allowed to elevate to room temperature, and ethyl acetate (200 ml) and water (40 ml) were added to the solution. The organic layer separated was collected, washed with saturated brine, and dried over anhydrous sodium sulfate. This was concentrated under reduced pressure, and the residue was purified by silica gel column chromatography (ethyl acetate/hexane = 1/3) to obtain the entitled compound (6.2 g) as a pale yellow oily substance.

¹H-NMR (400 MHz, CDCl₃) δ: 1.35 (3H, t, J=7.2 Hz), 1.50 (6H, s), 2.31 (6H, s), 4.30 (2H, q, J=7.2 Hz), 7.52 (2H, s), 9.85 (1H, s).

### [Reference Example 13]

### (1) Oxazole-2-carbaldehyde

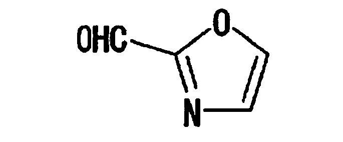

Oxazole (3.0 g) was dissolved in tetrahydrofuran (120 ml), and with stirring with cooling at -78°C, n-butyllithium (1.6 M hexane solution, 41 ml) was dropwise added thereto. The reaction solution was stirred at -10°C for 10 minutes, then again cooled to -78°C and stirred for 6 hours. 4-Formylmorpholine (22 ml) was dropwise added to the reaction solution, which was stirred at room temperature for 15 hours. With cooling with ice in water, an aqueous saturated ammonium chloride solution was added to the reaction solution, then diluted with ethyl acetate, and an aqueous potassium sodium tartrate solution was added thereto, and the organic layer was taken out. The organic layer was washed with saturated brine, then dried over anhydrous sodium sulfate to obtain an ethyl acetate solution of the entitled compound, which was directly used in the next reaction as it was.

(2) tert-Butyl (5-methyl-2-phenyloxazol-4-ylmethyl)oxazol-2-ylmethylcarbamate

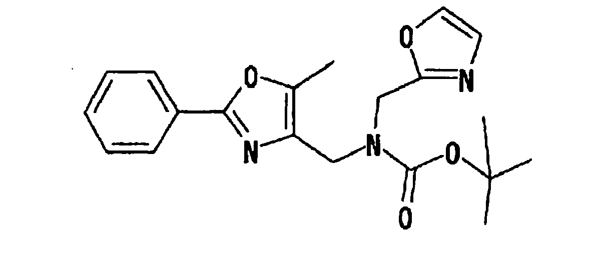

C-(5-Methyl-2-phenyloxazol-4-yl)methylamine (2.05 g) was dissolved in tetrahydrofuran (30 ml), and oxazole-2-carbaldehyde (ethyl acetate solution) obtained in Reference Example 13-(1) was added thereto and the resulting solution was heated under reflux for 6 hours. The solvent was evaporated under reduced pressure, then the residue was dissolved in methanol, and sodium borohydride (1.24 g) was added thereto with cooling with ice in water, and then stirred at room temperature for 5 hours. Water was added to the reaction solution, which was concentrated under reduced pressure. The residue was dissolved in ethyl acetate, and washed with an aqueous saturated sodium bicarbonate solution and saturated brine in that order, and dried over anhydrous sodium sulfate. The solvent was evaporated under reduced pressure. The residue was dissolved in dichloromethane (50 ml), and with cooling with ice in water, di-tert-butyl dicarbonate (2.86 g) and an aqueous saturated sodium bicarbonate solution (50 ml) were added thereto and the mixture was stirred at room temperature for 12 hours. The reaction solution was extracted with ethyl acetate, washed with water and saturated brine in that order, and dried over anhydrous sodium sulfate, and the solvent was evaporated under reduced pressure. The residue was purified by silica gel column chromatography (hexane/ethyl acetate = 1/2) to obtain the entitled compound (1.51 g) as a colorless solid.

¹H-NMR (400 MHz, CDCl₃) δ: 1.56 (9H, s), 2.35-2.39 (3H, m), 4.45-4.49 (2H, m), 4.64-4.73 (2H, m), 7.06 (1H, s), 7.41-7.44 (3H, m), 7.59 (1H, s), 7.96-7.98 (2H, m).
MS m/z: 370(M+H)⁺.

(3) (5-Methyl-2-phenyloxazol-4-ylmethyl)oxazol-2-ylmethylamine

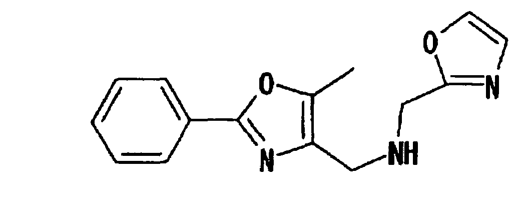

The compound (1.51 g) of Reference Example 13-(2) was dissolved in dichloromethane (20 ml), and with cooling with ice in water, trifluoroacetic acid (10 ml) was added thereto and the resulting solution was stirred at room temperature for 12 hours. With cooling with ice in water, the reaction solution was made alkaline by addition of 50% sodium hydroxide solution, and then extracted with ether. The organic layer was washed with water and saturated brine in that order, then dried over anhydrous sodium sulfate, and the solvent was evaporated under reduced pressure to obtain the entitled compound (0.87 g) as a pale yellow solid.

¹H-NMR (400 MHz, CDCl₃) δ: 2.36 (3H, s), 3.74 (2H, s), 3.97 (2H, s), 7.07 (1H, s), 7.40-7.45 (3H, m), 7.61 (1H, d, J=0.7 Hz), 7.98-8.01 (2H, m).
MS m/z: 270(M+H)⁺.

### [Reference Example 14]

### (1) Ethyl 2-[4-[(tert-butoxycarbonylmethylamino)methyl]-2,6-dimethylphenoxy]-2-methylpropanoate

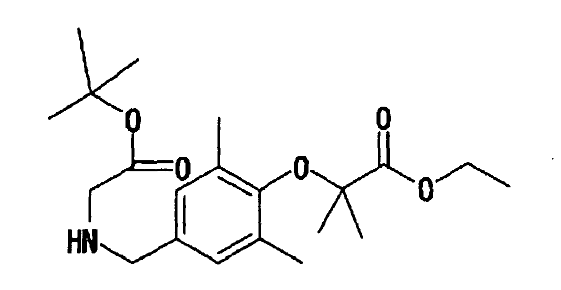

The compound (15.8 g) of Reference Example 12 was dissolved in tetrahydrofuran (300 ml), and tert-butyl glycinate (9 ml) and magnesium sulfate (50 g) were added thereto and the resulting solution was heated under reflux for 4 hours. The reaction solution was restored to room temperature, filtered through Celite, and the filtrate was concentrated under reduced pressure. The residue was dissolved in methanol (100 ml), and with cooling with ice in water, sodium borohydride (2.3 g) was added thereto and then the solution was stirred at room temperature for 4 hours. Water was added to the reaction solution, which was concentrated under reduced pressure, and the residue was diluted with ethyl acetate, then washed with an aqueous saturated sodium bicarbonate solution and saturated brine in that order, and dried over anhydrous sodium sulfate, and the solvent was evaporated under reduced pressure. The residue was purified by silica gel column chromatography (chloroform/methanol = 19/1) to obtain the entitled compound (21.6 g) as a pale yellow oily substance.

¹H-NMR (400 MHz, CDCl₃) δ: 1.35 (3H, t, J=7.1 Hz), 1.46 (6H, s), 1.48 (9H, s), 2.18 (6H, s), 3.30 (2H, s), 3.65 (2H, s), 4.29 (2H, q, J=7.1 Hz), 6.93 (2H, s).
MS m/z: 380(M+H)⁺.

### (2) Ethyl 2-[4-[[tert-butoxycarbonylmethyl-(5-methyl-2-phenyloxazol-4-ylmethyl)amino]methyl]-2,6-dimethylphenoxy]-2-methylpropanoate

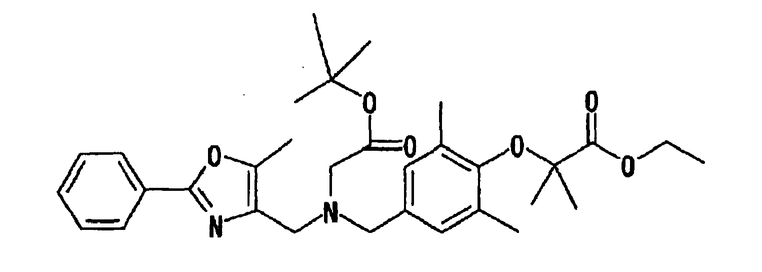

4-Chloromethyl-5-methyl-2-phenyloxazole (1.87 g) and the compound (3.9 g) of Reference Example 14-(1) were dissolved in acetonitrile (30 ml), and potassium carbonate (2.5 g) was added thereto and the resulting solution was heated under reflux for 24 hours. The reaction solution was concentrated under reduced pressure, and the residue was diluted with ethyl acetate, then washed with water and saturated brine in that order, dried over anhydrous sodium sulfate, and the solvent was evaporated under reduced pressure. The residue was purified by silica gel column chromatography (hexane/ethyl acetate = 2/1) to obtain the entitled compound (4.0 g) as an yellow oily substance.

¹H-NMR (400 MHz, CDCl₃) δ: 1.35 (3H, t, J=7.1 Hz), 1.45 (6H, s), 1.48 (9H, s), 2.17 (6H, s), 2.30 (3H, s), 3.30 (2H, s), 3.71 (2H, s), 3.75 (2H, s), 4.28 (2H, q, J=7.1 Hz), 6.99 (2H, s), 7.38-7.46 (3H, m), 8.00-8.02 (2H, m).
MS m/z: 551(M+H)⁺.

### (3) Ethyl 2-[4-[[carboxymethyl-(5-methyl-2-phenyloxazol-4-ylmethyl)ainino]methyl]-2,6-dimethylphenoxy]-2-methylpropanoate

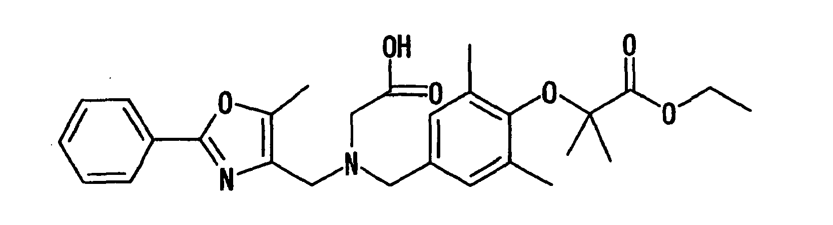

The compound (4.0 g) of Reference Example 14-(2) was dissolved in dichloromethane (30 ml), and with cooling with ice in water, 4 N hydrochloric acid-dioxane solution (30 ml) was added thereto and the resulting solution was stirred at room temperature for 14 hours. The reaction solution was concentrated under reduced pressure, and the residue was crystallized from hexane to obtain a hydrochloride of the entitled compound (3.17 g) as a pale yellow solid.
MS m/z: 495(M+H)⁺.

### [Reference Example 15]

### (5-Methyl-2-phenyloxazol-4-ylmethyl)thiazol-2-ylmethylamine

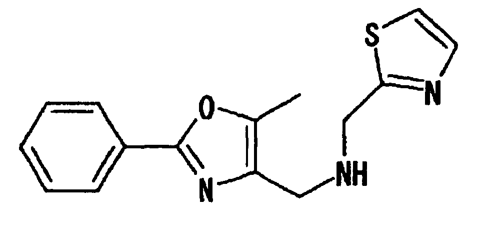

C-(5-Methyl-2-phenyloxazol-4-yl)methylamine (5.17 g) and thiazole-2-carbaldehyde (3.11 g) were dissolved in chloroform (50 ml) and the reaction solution was concentrated under reduced pressure. The residue was dissolved in methanol (100 ml), and sodium borohydride (1.5 g) was added thereto and the reaction solution was stirred at room temperature for 10 hours. Ethyl acetate and water were added, and the organic layer was separated and dried over anhydrous sodium sulfate. The solvent was evaporated under reduced pressure, and the residue was subjected to silica gel column chromatography (methanol/dichloromethane = 1/30) to obtain the entitled compound (4.8 g) as a pale yellow oily substance.

### [Reference Example 16]

### (1) Ethyl 2-[4-[[[2-(N'-tert-butoxycarbonylhydrazino)-2-oxoethyl]-(5-methyl-2-phenyloxazol-4-ylmethyl)amino]methyl]-2,6-dimethylphenoxy]-2-methylpropanoate

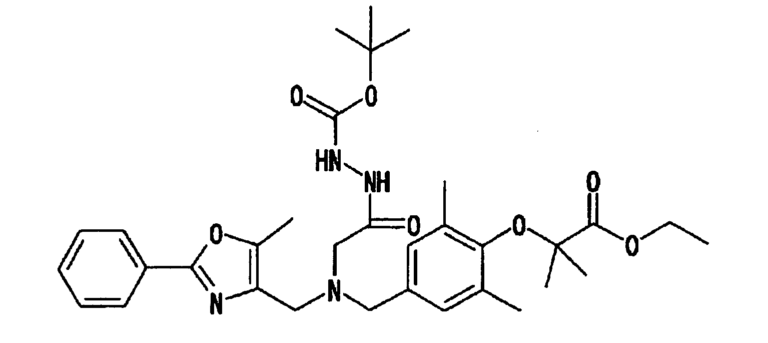

The compound (1.0 g) of Reference Example 14-(3) was dissolved in N,N-dimethylformamide (3 ml), and tert-butyl hydrazinecarboxylate (400 mg), 1-(3-dimethylaminopropyl)-3-ethylcarbodiimide hydrochloride (776 mg) and 1-hydroxybenzotriazole (622 mg) were added thereto and the resulting solution was stirred overnight at room temperature. The reaction solution was concentrated under reduced pressure, diluted with ethyl acetate, and washed with water, an aqueous 10% citric acid solution, an aqueous saturated sodium bicarbonate solution and saturated brine in that order. This was dried over anhydrous sodium sulfate, and the solvent was evaporated under reduced pressure to obtain the entitled compound as an yellow oily substance, which was used in the next reaction directly as it was.
MS m/z: 609(M+H)⁺.

### (2) Ethyl 2-[4-[[hydrazinocarbonylmethyl-(5-methyl-2-phenyloxazol-4-ylmethyl)amino]methyl]-2,6-dimethylphenoxy]-2-methylpropanoate

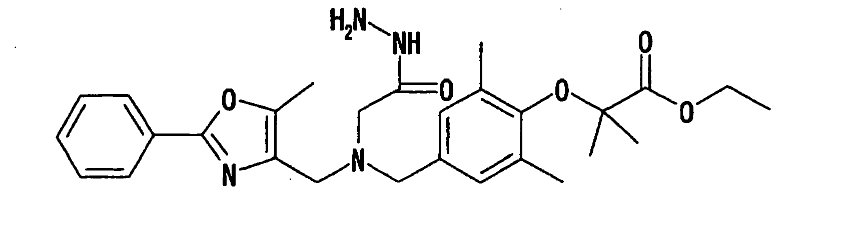

The compound of Reference Example 16-(1) was dissolved in dichloromethane (5 ml), and trifluoroacetic acid (5 ml) was added thereto and the resulting solution was stirred at room temperature for 14 hours. The solvent was evaporated under reduced pressure, and the residue was dissolved in ethyl acetate, and washed with an aqueous saturated sodium bicarbonate solution and then with saturated brine. This was dried over anhydrous sodium sulfate, and the solvent was evaporated under reduced pressure to obtain the entitled compound (1.14 g) as an yellow oily substance.
MS m/z: 509(M+H)⁺.

### [Reference Example 17]

### Ethyl 2-[4-[[[2-(N'-acetylhydrazino)-2-oxoethyl]-(5-methyl-2-phenyloxazol-4-ylmethyl)amino]methyl]-2,6-dimethylphenoxy]-2-methylpropanoate

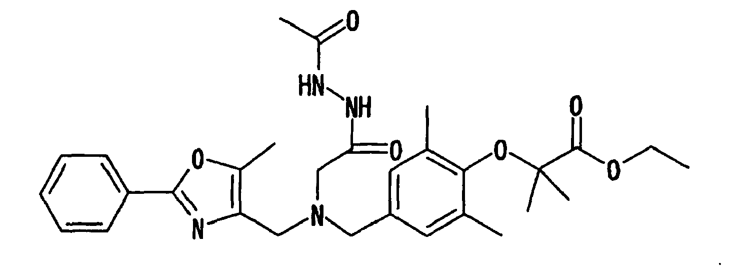

In the same manner as in Reference 16-(1), the entitled compound (503 mg) was obtained as a pale yellow oily substance from the compound (400 mg) of Reference Example 14-(3) and acetic acid hydrazide (77 mg).
¹H-NMR (400 MHz, CDCl₃) δ: 1.35 (3H, t, J=7.1 Hz), 1.45 (6H, s), 2.02 (3H, s), 2.18 (6H, s), 2.23 (3H, s), 3.40 (2H, s), 3.60 (2H, s), 3.65 (2H, s), 4.28 (2H, q, J=7.1 Hz), 6.98 (2H, s), 7.42-7.45 (3H, m), 8.00-8.02 (2H, m).
MS m/z: 551(M+H)⁺.

### [Reference Example 18]

### Ethyl 2-(4-formylphenoxy)hexanoate

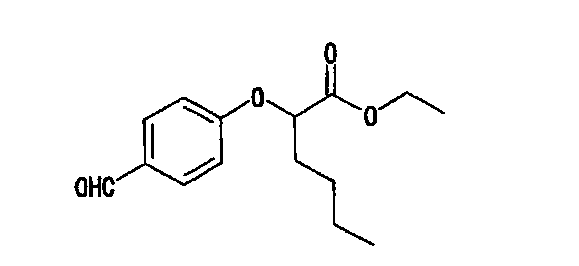

In the same manner as in Reference Example 12, the entitled compound (2.24 g) was obtained as a colorless oily substance from 2-hydroxybenzaldehyde (1.22 g) and ethyl 2-bromohexanoate (2.23 g).
¹H-NMR (400 MHz, CDCl₃) δ: 0.93 (3H, t, J=7.2 Hz), 1.23(3H, t, J=7.1 Hz), 1.25-1.60 (6H, m), 4.24 (2H, q, J=7.1 Hz), 4.50-4.42 (1H, m), 6.70, (2H, d, J=8.1 Hz), 7.65 (2H, d, J=8.1 Hz), 9.85 (1H, s).

### [Reference Example 19]

### Ethyl 2-[2,6-dimethyl-4-[[2-(5-methyl-2-phenyloxazol-4-yl)ethylamino]methyl]phenoxy]-2-methyl-propionate

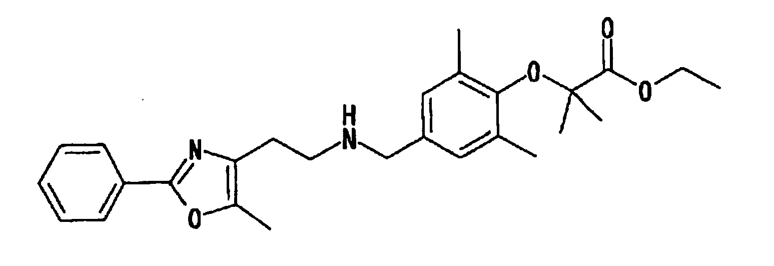

2-(5-Methyl-2-phenyloxazol-4-yl)ethylamine (400 mg) and the compound (523 mg) of Reference Example 12 were dissolved in tetrahydrofuran (10 ml), and magnesium sulfate (5 g) was added thereto and the resulting mixture was stirred at room temperature for 17 hours. This was filtered through Celite, and the filtrate was concentrated under reduced pressure. The residue was dissolved in methanol (10 ml), and with cooling with ice in water, sodium borohydride (225 mg) was added thereto, and the solution was stirred at room temperature for 3 hours. The reaction solution was concentrated under reduced pressure, water was added to the residue, extracted with ethyl acetate, and washed with an aqueous saturated sodium bicarbonate solution and saturated brine. This was dried over anhydrous sodium sulfate, the solvent was evaporated under reduced pressure, and the residue was subjected to silica gel column chromatography (methanol/chloroform = 1/9) to obtain the entitled compound (1.0 g) as a pale yellow oily substance.

¹H-NMR (400 MHz, CDCl₃) δ: 1.35 (3H, t, J=7.2 Hz), 1.45 (6H, s), 2.17 (6H, s), 2.33 (3H, s), 2.71 (2H, t, J=6.9 Hz), 2.95 (2H, t, J=6.9 Hz), 3.70 (2H, s), 4.28 (2H, q, J=7.2 Hz), 6.91 (2H, s), 7.40-7.44 (3H, m), 7.96-7.99 (2H, m).

### [Reference Example 20]

### Ethyl 2-[2,6-dimethyl-4-[[(5-methyl-2-phenoxyoxazol-4-ylmethyl)amino]methyl]phenoxy]-2-methylpropionate

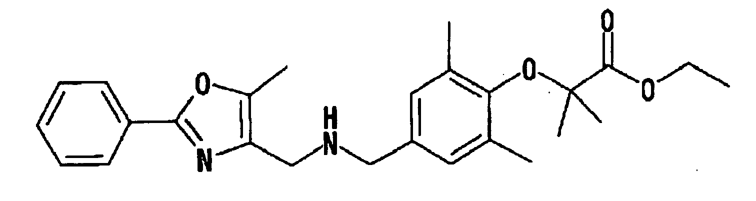

In the same manner as in Reference Example 19, the entitled compound (2.9 g) was obtained as an yellow oily substance from C-(5-methyl-2-phenyloxazol-4-yl)methylamine (1.43 g) and the compound (2.0 g) of Reference Example 12.
¹H-NMR (400 MHz, CDCl₃) δ: 1.35 (3H, t, J=7.2 Hz), 1.45 (6H, s), 2.18 (6H, s), 2.31 (3H, s), 3.67 (2H, s), 3.70 (2H, s), 4.29 (2H, q, J=7.2 Hz), 6.95 (2H, s), 7.40-7.45 (3H, m), 7.98-8.00 (2H, m).
MS m/z: 437(M+H)⁺.

### [Reference Example 21]

### (1) Ethyl 2-(4-hydroxyiminomethyl-2,6-dimethylphenoxy)-2-methylpropionate

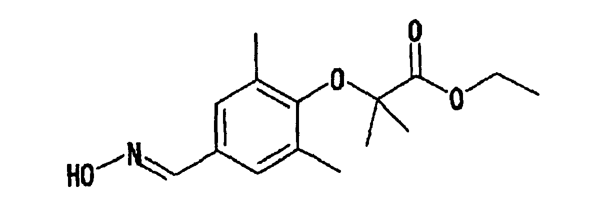

The compound (3.0 g) of Reference Example 12, hydroxylamine hydrochloride (3.15 g) and N-methylmorpholine (5 ml) were dissolved in methanol (100 ml), and magnesium sulfate (20 g) was added thereto and the resulting mixture was heated under reflux for 5 hours. After cooled, the mixture was filtered through Celite, and the filtrate was concentrated under reduced pressure. The residue was dissolved in ethyl acetate, and washed with an aqueous 0.5N hydrochloric acid solution and then with saturated brine.

This was dried over anhydrous sodium sulfate, the solvent was evaporated under reduced pressure, and the residue was purified by silica gel column chromatography (ethyl acetate/hexane = 1/1) to obtain the entitled compound (2.8 g) as a pale yellow oily substance.
MS m/z: 280(M+H)⁺.

### (2) Ethyl 2-(4-aminomethyl-2,6-dimethylphenoxy)-2-methylpropionate

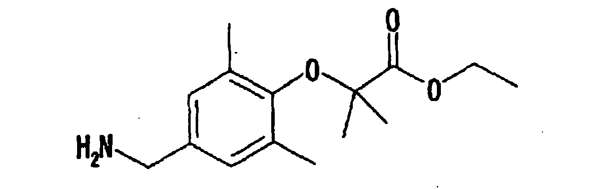

The compound (2.8 g) of Reference example 21-(1) was dissolved in a mixture of ethanol (50 ml) and tetrahydrofuran (10 ml), and 10% palladium-carbon (2 g) was added thereto, and the mixture was left at room temperature under about 30 hydrogen atmospheres for 12 hours. The catalyst was removed by filtration, the filtrate was concentrated under reduced pressure, then ethyl acetate and hexane were added to the residue, and a solid was collected by filtration to obtain the entitled compound (1.72 g) as a colorless solid.
¹H-NMR (400 MHz, CDCl₃) δ: 1.26 (3H, t, J=7.2 Hz), 1.38 (6H, s), 2.13 (6H, s), 3.88(2H, brs), 4.19 (2H, q, J=7.1 Hz), 7.15 (2H, s), 8.37 (2H, brs).

### [Reference Example 22]

### (1) Ethyl 2-[4-[[[(2-hydroxy-1-methylethylcarbamoyl)methyl](5-methyl-2-phenyloxazol-4-yhnethyl)amino]methyl]-2,6-dimethylphenoxy]-2-methylpropanoate

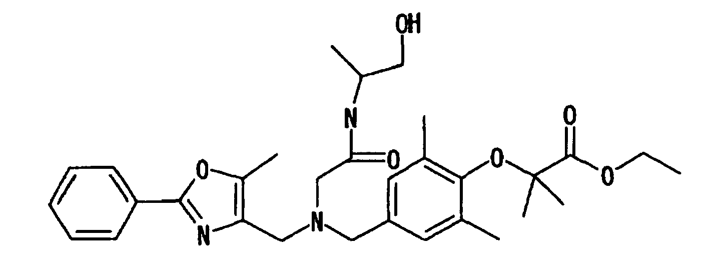

The compound (0.200 g) obtained in Reference Example 14-(3) and 2-amino-1-propanol (0.063 ml) were dissolved in methanol (3 ml), and 4-(4,6-dimethoxy-1,3,5-triazin-2-yl)-4-methylmorpholinium chloride n-hydrate (0.224 g) was added thereto and the resulting solution was stirred at room temperature for 24 hours. Again, 2-amino-1-propanol (0.063 ml) and 4-(4,6-dimethoxy-1,3,s-triazin-2-yl)-4-methylmorpholinium chloride n-hydrate (0.224 g) were added thereto and the solution was stirred for further 16 hours. The reaction solution was diluted with ethyl acetate, washed with saturated brine, and dried over anhydrous sodium sulfate. The solvent was evaporated under reduced pressure, and the resulting residue was purified by silica gel column chromatography (methanol/chloroform = 1/9) to obtain the entitled compound (0.292 g) as a colorless oily substance.
¹H-NMR (400 MHz, CDCl₃) δ: 1.19 (3H, d, J=6.9 Hz), 1.34 (3H, t, J=7.1 Hz), 1.43 (6H, s), 2.14 (6H, s), 2.32 (3H, s), 3.02-4.01 (9H, m), 4.10 (1H, s), 4.27 (2H, q, J=7.2 Hz), 4.59 (1H, s), 6.85 (2H, s), 7.45-7.52 (3H, m), 8.00-8.05 (3H, m).
MS m/z: 552(M+H)⁺.

### (2) Ethyl 2-[2,6-dimethyl-4-[[[(1-methyl-2-oxoethylcarbamoyl)methyl](s-methyl-2-phenyloxazol-4-ylmethyl)amino]methyl]phenoxy]-2-methylpropanoate

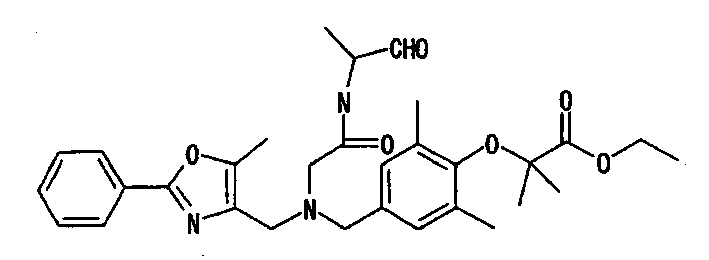

To a solution of oxalyl chloride (0.45 ml) in dichloromethane (10 ml) was cooled and stirred at -78°C, a solution of dimethylsulfoxide (0.52 ml) in dichloromethane (1 ml) was dropwise added. After 10 minutes, a solution of the compound (0.292 g) obtained in Reference Example 22-(1) in dichloromethane (5 ml) was added to it, and stirred for 1 hour. Triethylamine (1.7 ml) was added to it, then the resulting solution was warmed up to room temperature, and stirred for 1 hour. Water was added to the reaction solution, which was extracted with ethyl acetate and washed with an aqueous saturated sodium bicarbonate solution and saturated brine in that order. This was dried over anhydrous sodium sulfate, and the solvent was evaporated under reduced pressure. The resulting residue was purified by silica gel column chromatography (methanol/chloroform = 1/19) to obtain the entitled compound (0.247 g) as an yellow oily substance.
¹H-NMR (400 MHz, CDCl₃) δ: 1.31-1.36 (6H, m), 1.44 (6H, s), 2.17 (6H, s), 2.29 (3H, s), 3.29 (2H, s), 3.54 (1H, d, J=13.9 Hz), 3.59 (1H, d, J=13.9 Hz), 3.61 (2H, s), 4.27 (2H, q, J=7.1 Hz), 4.43 (1H, quin, J=7.3 Hz), 6.95 (2H, s), 7.42-7.45 (3H, m), 7.95-7.97 (2H, m), 8.34 (1H, d, J=7.3 Hz), 9.51 (1H, s).
MS m/z: 550(M+H)⁺.

### [Reference Example 23]

### Ethyl 2-[2,6-dimethyl-4-[[(oxazol-2-ylmethyl)amino]methyl]phenoxy]-2-methylpropanoate

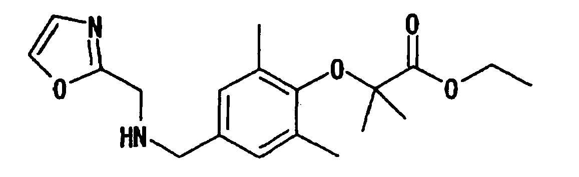

The compound (2.70 g) obtained in Reference Example 21-(2) and N-methylmorpholine (1.7 ml) were dissolved in a mixture of methanol (10 ml) and tetrahydrofuran (20 ml). Ethyl acetate solution of oxazole-2-carbaldehyde, which had been produced from oxazole (1.73 g) in the same manner as in Reference Example 13-(1), and magnesium sulfate (30 g) were added to it, and the resulting mixture was heated under reflux for 14 hours. After cooled, the insoluble material was removed by filtration through Celite, and the filtrate was concentrated under reduced pressure. The residue was dissolved in methanol (30 ml), and with cooling with ice in water, sodium borohydride (2.84 g) was added thereto, and stirred at room temperature for 2 hours. The reaction solution was concentrated under reduced pressure, the residue was dissolved in ethyl acetate, and washed with an aqueous saturated sodium bicarbonate solution and then with saturated brine. This was dried over anhydrous sodium sulfate, and the solvent was evaporated under reduced pressure. The resulting residue was purified by silica gel column chromatography (ethyl acetate) to obtain the entitled compound (2.35 g) as an yellow oily substance.
¹H-NMR (400 MHz, CDCl₃) δ: 1.35 (3H, t, J=7.2 Hz), 1.45 (6H, s), 2.18 (6H, s), 3.71 (2H, s), 3.93 (2H, s), 4.29 (2H, q, J=7.2 Hz), 6.93 (2H, s), 7.07 (1H, s), 7.61 (1H, d, J=1.0 Hz).
MS m/z: 347(M+H)⁺.

### [Reference Example 24]

### (1) Ethyl 2-[4-[(carboxymethyloxazol-2-ylmethylamino)methyl]-2,6-dimethylphenoxy]-2-methylpropanoate

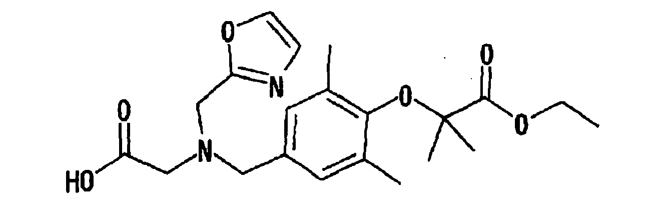

In the same manner as in Reference Example 6, the entitled compound was obtained from the compound (0.5 g) obtained in Reference Example 23 and glyoxylic acid (0.2 g). This was dissolved in a small amount of dichloromethane, and an excessive amount of 4 N hydrochloric acid-dioxane solution was added thereto and the resulting solution was concentrated under reduced pressure. The residue was crystallized from a mixed solvent of ether/ethyl acetate/hexane to obtain a hydrochloride of the entitled compound (0.43 g) as an yellow solid.
¹H-NMR (400 MHz, CDCl₃) δ: 1.32 (3H, t, J=7.1 Hz), 1.47 (6H, s), 2.23 (6H, s), 4.27-4.21 (4H, m), 4.43 (2H, s), 4.60 (2H, s), 7.24 (2H, s), 7.33 (1H, s), 8.07 (1H, d, J=0.7 Hz).
MS m/z: 405(M+H)⁺.

### (2) Ethyl 2-[4-[[[2-(N'-benzoylhydrazino)-2-oxoethyl]oxazol-2-ylmethylamino]methyl]-2,6-dimethylphenoxy]-2-methylpropanoate

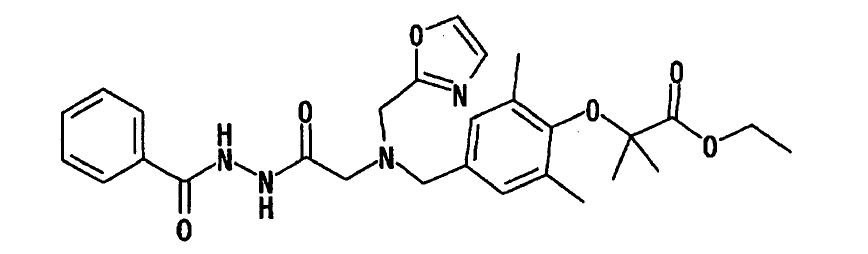

The compound (0.15 g) obtained in Reference Example 24-(1), benzoic acid hydrazide (0.070 g) and 1-hydroxybenzotriazole (0.079 g) were dissolved in N,N-dimethylformamide (2 ml), and 1-ethyl-3-(3-dimethylaminopropyl)carbodiimide hydrochloride (0.098 g) was added thereto and the resulting solution was stirred at room temperature for 5 hours. The reaction solution was concentrated under reduced pressure, the residue was dissolved in ethyl acetate, and then washed with water, an aqueous citric acid solution, an aqueous saturated sodium bicarbonate solution and saturated brine in that order. This was dried over anhydrous sodium sulfate, and the solvent was evaporated under reduced pressure. The resulting residue was purified by silica gel column chromatography (methanol/chloroform = 1/9) to obtain the entitled compound (0.115 g) as a pale yellow oily substance.
¹H-NMR (400 MHz, CDCl₃) δ: 1.34 (3H, t, J=7.3 Hz), 1.45 (6H, s), 2.20 (6H, s), 3.45 (2H, s), 3.71 (2H, s), 3.95 (2H, s), 4.27 (2H, q, J=7.2 Hz), 7.03 (2H, s), 7.12 (1H, s), 7.47 (2H, t, J=7.7 Hz), 7.52-7.58 (1H, m), 7.67 (1H, s), 7.83 (2H, d, J=7.1 Hz), 8.54 (1H, br s), 10.00 (1H, br s).
MS m/z: 523(M+H)⁺.

### [Reference Example 25]

### Ethyl 2-[2,6-dimethyl-4-[[oxazol-2-ylmethyl-[(2-oxo-2-phenylethylcarbamoyl)methyl]amino]methyl]phenoxy]-2-methylpropanoate

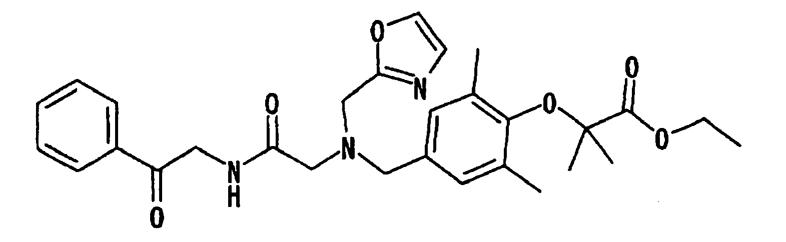

In the same manner as in Reference Example 24-(2), the entitled compound (0.122 g) was obtained as a pale yellow oily substance from the compound (0.15 g) obtained in Reference Example 24-(1), 2-aminoacetophenone hydrochloride (0.064 g) and N-methylmorpholine (0.083 ml).
¹H-NMR (400 MHz, CDCl₃) δ: 1.34 (3H, t, J=7.6 Hz), 1.45 (6H, s), 2.21 (6H, s), 3.35 (2H, s), 3.68 (2H, s), 3.91 (2H, s), 4.28 (2H, q, J=7.1 Hz), 4.76 (2H, d, J=4.7 Hz), 7.08 (2H, s), 7.09 (1H, s), 7.51 (2H, t, J=8.0 Hz), 7.62 (1H, t, J=6.7 Hz), 7.65 (1H, s), 7.99 (2H, d, J=8.3 Hz).
MS m/z: 522(M+H)⁺.

### [Reference Example 26]

### Ethyl 2-[2,6-dimethyl-4-[[oxazol-2-ylmethyl-[[2-oxo-2-[N'-(thiophene-2-carbonyl)hydrazino]ethyl]amino]methyl]amino]methyl]phenoxy-2-methylpropanoate

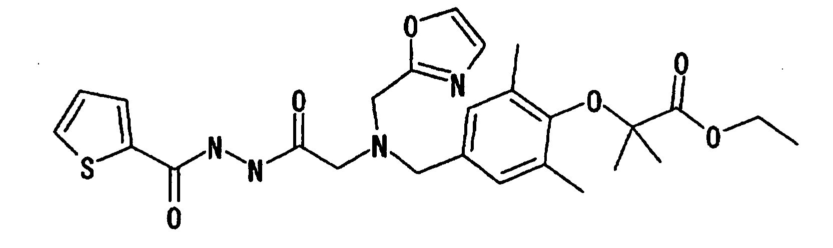

In the same manner as in Reference Example 24-(2), the entitled compound (0.12 g) was obtained as an yellow oily substance from the compound (0.15 g) obtained in Reference Example 24-(1) and 2-thiophenecarboxylic acid hydrazide (0.053 g).
¹H-NMR (400 MHz, CDCl₃) δ: 1.34 (3H, t, J=7.1 Hz), 1.45 (6H, s), 2.20 (6H, s), 3.43 (2H, s), 3.69 (2H, s), 3.94 (2H, s), 4.28 (2H, q, J=7.1 Hz), 6.99 (2H, s), 7.12-7.10 (2H, m), 7.55 (1H, d, J=4.9 Hz), 7.62 (1H, d, J=3.7 Hz), 7.66 (1H, s), 8.31 (1H, s), 9.81 (1H, s).
MS m/z: 529(M+H)⁺.

### [Reference Example 27]

### (1) N-Hydroxythiophene-2-carboxyamidine

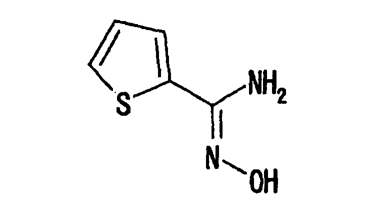

2-Thiophenecarbonitrile (1.0 g) was dissolved in ethanol (20 ml), and 50% hydroxylamine (1.2 ml) was added thereto and the resulting solution was heated under reflux for 18 hours. After cooled, the reaction solution was concentrated under reduced pressure, then ether-hexane was added to the residue, and the insoluble material was collected by filtration to obtain the entitled compound (1.1 g) as a colorless solid.
¹H-NMR (400 MHz, CD₃OD) δ: 7.05-7.02 (1H, m), 7.37-7.40 (2H, m).
MS m/z: 143(M+H)⁺.

### (2) Ethyl 2-[2,6-dimethyl-4-[[oxadiazol-2-ylmethyl-[2-oxo-2-[(thiophene-2-carboximidoyl)aminoxy]ethyl]amino]methyl]phenoxy]-2-methylpropanoate

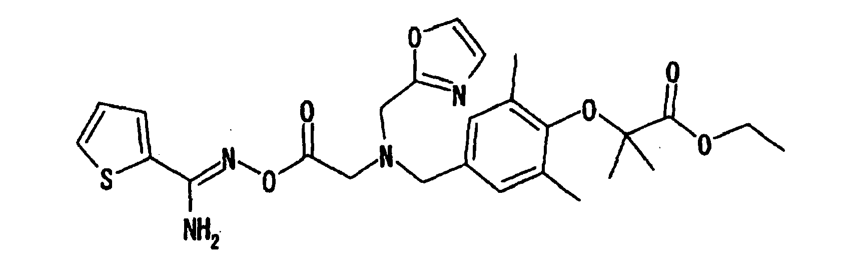

In the same manner as in Reference Example 24-(2), the entitled compound was obtained as a brown oily substance from the compound (0.15 g) obtained in Reference Example 24-(1) and the compound (0.053 g) obtained in Reference Example 27-(1), and this was used in the next reaction directly as it was.
MS m/z: 529(M+H)⁺.

### [Reference Example 28]

### Ethyl 2-(2-fluoro-4-formyl-6-methoxyphenoxy)-2-methylpropanoate

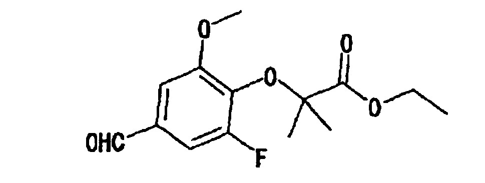

In the same manner as in Reference Example 12, the entitled compound (0.78 g) was obtained as a colorless oily substance from 3-fluoro-4-hydroxy-5-methoxybenzaldehyde (0.50 g) and ethyl 2-bromo-2-methylpropanoate (1.8 g).
¹H-NMR (400 MHz, CDCl₃) δ: 1.32 (3H, t, J=7.1 Hz), 1.55 (6H, s), 3.86 (3H, s), 4.26 (2H, q, J=7.1 Hz), 7.22-7.26 (2H, m), 9.85 (1H, d, J=1.2 Hz).
MS m/z: 285(M+H)⁺.

### [Reference Example 29]

### Ethyl 2-(4-formyl-2-methoxy-6-methylphenoxy)-2-methylpropanoate

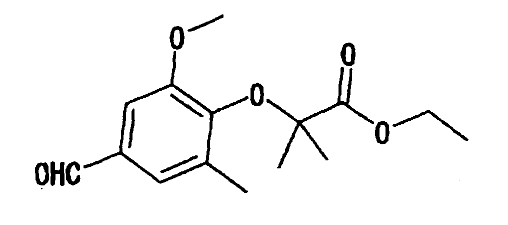

In the same manner as in Reference Example 12, the entitled compound (21.5 g) was obtained as a colorless solid from 4-hydroxy-3-methoxy-5-methylbenzaldehyde (11.5 g) and ethyl 2-bromo-2-methylpropanoate (30 ml).
¹H-NMR (400 MHz, CDCl₃) δ: 1.32 (3H, t, J=7.3 Hz), 1.50 (6H, s), 2.30 (3H, s), 3.78 (3H, s), 4.28 (2H, q, J=7.3 Hz), 7.22 (1H, s), 7.30 (1H, s), 9.78 (1H, s).
MS m/z: 281(M+H)⁺.

### [Reference Example 30]

### (1) Ethyl 2-[4-[[tert-butoxycarbonylmethyl-(9H-fluoren-9-ylmethoxycarbonyl)amino]methyl]-2,6-dimethylphenoxy]-2-methylpropanoate

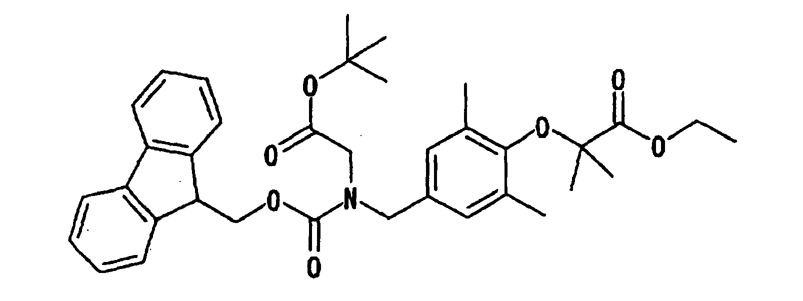

The compound (3.5 g) obtained in Reference Example 14-(1) was dissolved in acetonitrile (30 ml), and with cooling with ice in water, N-(9-fluorenylmethoxycarbonyloxy)succinimide (3.74 g) was added thereto, and the resulting solution was stirred at room temperature for 4 hours. The reaction solution was concentrated under reduced pressure, then the residue was extracted with ethyl acetate, and the organic layer was washed with water, an aqueous saturated sodium bicarbonate solution, an aqueous citric acid solution, saturated brine in that order. This was dried over anhydrous sodium sulfate, the solvent was evaporated under reduced pressure, and the resulting residue was purified by silica gel column chromatography (ethyl acetate/hexane = 1/1) to obtain the entitled compound (5.24 g) as a pale yellow oily substance.
¹H-NMR (400 MHz, CDCl₃) δ: 1.34-1.38 (3H, m), 1.43-1.54 (15H, m), 2.17 (3H, s), 2.18 (3H, s), 3.78 (1H, s), 3.84 (1H, s), 4.26-4.30 (3H, m), 4.44-4.49 (4H, m), 6.77 (1H, s), 6.85 (1H, s), 7.25-7.31 (2H, m), 7.41-7.35 (2H, m), 7.53 (1H, d, J=7.4 Hz), 7.59 (1H, d, J=7.4 Hz), 7.73 (1H, d, J=7.6 Hz), 7.76 (1H, d, J=7.6 Hz).
MS m/z: 624(M+Na)⁺.

### (2) Ethyl 2-[4-[[carboxymethyl-(9H-fluoren-9-ylmethoxycarbonyl)amino]methyl]-2,6-dimethylphenoxy]-2-methylpropanoate

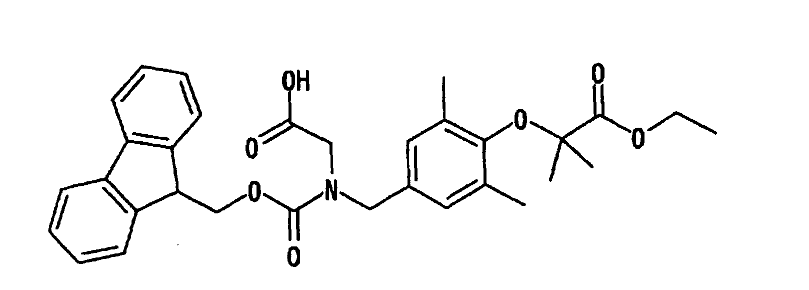

The compound (5.24 g) obtained in Reference Example 30-(1) was dissolved in dichloromethane (60 ml), and with cooling with ice in water, trifluoroacetic acid (20 ml) was added thereto, and the resulting solution was stirred at room temperature for 16 hours. The reaction solution was concentrated under reduced pressure, and the residue was extracted with ethyl acetate. The organic layer was washed with water and saturated brine in that order. This was dried over anhydrous sodium sulfate, and the solvent was evaporated under reduced pressure. The resulting residue was purified by silica gel column chromatography (methanol/chloroform = 1/9) to obtain the entitled compound (5.70 g) as an yellow oily substance.
¹H-NMR (400 MHz, CDCl₃) δ: 1.33-1.38 (3H, m), 1.45 (3H, s), 1.46 (3H, s), 2.17 (6H, s), 3.77 (1H, s), 3.98 (1H, s), 4.25-4.32 (3H, m), 4.44 (2H, s), 4.52-4.55 (2H, m), 6.75 (1H, s), 6.82 (1H, s), 7.23-7.31 (2H, m), 7.38 (2H, t, J=7.4 Hz), 7.56-7.53 (2H, m), 7.74 (2H, d, J=7.3 Hz).
MS m/z: 568(M+H)⁺.

### (3) Ethyl 2-[4-[[[2-(N'-acetylhydrazino)-2-oxoethyl]-(9H-fluoren-9-ylmethoxycarbonyl)amino]methyl]-2,6-dimethylphenoxy]-2-methylpropanoate

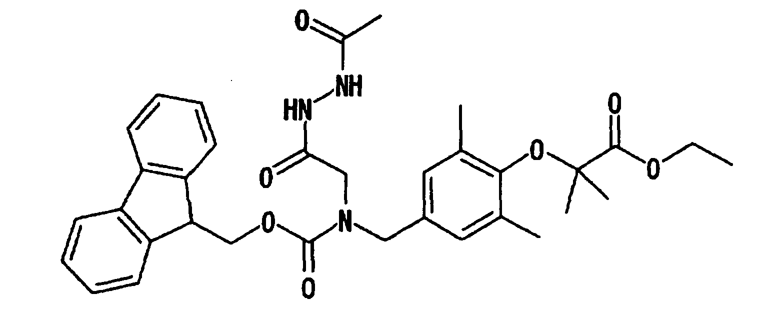

In the same manner as in Reference Example 17, the entitled compound (1.1 g) was obtained as a colorless solid from the compound (1.5 g) obtained in Reference Example 30-(2) and acetic acid hydrazide (0.31 g).
MS m/z: 602(M+H)⁺.

### (4) Ethyl 2-[4-[[(9H-fluoren-9-ylmethoxycarbonyl)-(5-methyl-[1,3,4]oxadiazol-2-ylmethyl)amino]methyl]-2,6-dimethylphenoxy]-2-methylpropanoate

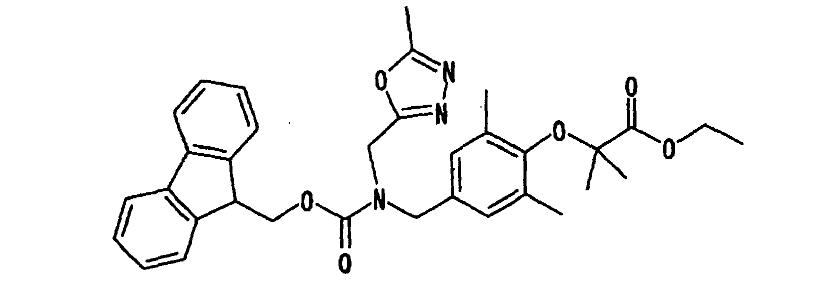

In the same manner as in Reference Example 18-(1), the entitled compound (1.0 g) was obtained as a colorless solid from the compound (1.1 g) obtained in Reference Example 30-(3).
¹H-NMR (400 MHz, CDCl₃) δ: 1.36 (3H, t, J=7.1 Hz), 1.46 (6H, s), 2.16 (6H, s), 2.46,2.49 (3H, each s), 4.26-4.31 (3H, m), 4.39-4.44 (3H, m), 4.55-4.59 (3H, m), 6.73,6.85 (2H, each s), 7.36-7.40 (2H, m), 7.46-7.49 (1H, m), 7.53-7.57 (3H, m), 7.65-7.70 (1H, m), 7.75-7.73 (2H, m).
MS m/z: 584(M+H)⁺.

### (5) Ethyl 2-[2,6-dimethyl-4-[[(5-methyl-[1,3,4]oxadiazol-2-ylmethyl)axnino]methyl]phenoxy-2-methylpropanoate

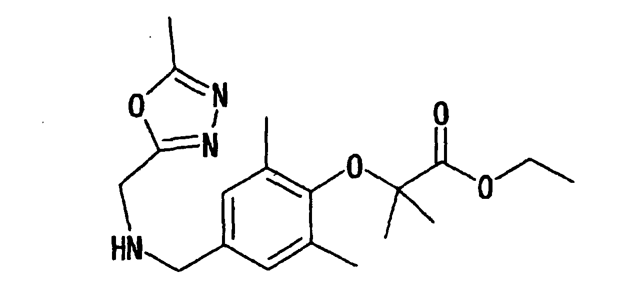

The compound (1.0 g) obtained in Reference Example 30-(4) was dissolved in tetrahydrofuran (50 ml), and with cooling with ice in water, 1,8-diazabicyclo[5.4.0]undec-7-ene (2% tetrahydrofuran solution, 42.2 ml) was added thereto, and the resulting solution was stirred at room temperature for 2 hours, and then the reaction solution was concentrated under reduced pressure. The residue was extracted with ethyl acetate, and then washed with an aqueous saturated sodium bicarbonate solution and saturated brine in that order. This was dried over anhydrous sodium sulfate, and the solvent was evaporated under reduced pressure. The resulting residue was purified by silica gel column chromatography (methanol/chloroform = 1/9) to obtain the entitled compound (0.54 g) as an yellow oily substance.
¹H-NMR (400 MHz, CDCl₃) δ: 1.35 (3H, t, J=7.1 Hz), 1.46 (7H, s), 2.19 (6H, s), 2.53 (3H, s), 3.73 (2H, s), 3.98 (2H, s), 4.29 (2H, q, J=7.1 Hz), 6.92 (2H, s).
MS m/z: 362(M+H)⁺.

### [Reference Example 31]

### Ethyl 2-[2,6-dimethyl-4-[[(thiazol-2-ylmethyl)amino]methyl]phenoxy]-2-methylpropanoate

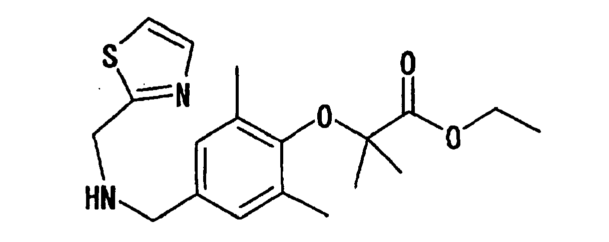

The compound (hydrochloride, 1.0 g) obtained in Reference Example 21-(2), 2-thiazolecarbaldehyde (0.46 g) and N-methylmorpholine (0.40 ml) were added to tetrahydrofuran (20 ml), and the resulting solution was heated under reflux for 2 hours. After cooled, the solvent was evaporated under reduced pressure, the resulting residue was dissolved in methanol (30 ml), and sodium borohydride (0.38 g) was added thereto. The solution was stirred at room temperature for 18 hours. The reaction solution was concentrated under reduced pressure. The residue was extracted with ethyl acetate, and washed with water and saturated brine in that order. This was dried over anhydrous sodium sulfate, and the solvent was evaporated under reduced pressure. The resulting residue was purified by silica gel column chromatography (methanol/chloroform = 1/9) to obtain the entitled compound (1.2 g) as a pale yellow oily substance.
¹H-NMR (400 MHz, CDCl₃) δ: 1.35 (3H, t, J=7.1 Hz), 1.46 (7H, s), 2.19 (7H, s), 3.76 (2H, s), 4.14 (2H, s), 4.29 (2H, q, J=7.1 Hz), 6.95 (2H, s), 7.28 (1H, d, J=3.4 Hz), 7.73 (1H, d, J=3.2 Hz).
MS m/z: 363(M+H)⁺.

### [Reference Example 32]

### 4-Chloromethyl-5-methyl-2-(5-methylthiophen-2-yl)oxazole

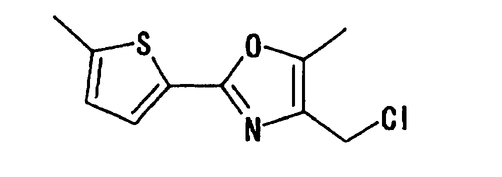

5-Methyl-2-thiophenecarbaldehyde (1.07 ml) and 2,3-butanedione monoxide (2.0 g) were mixed, and 4 N hydrochloric acid-dioxane solution (10 ml) was added thereto and the resulting solution was stirred at room temperature for 14 hours. 4 N hydrochloric acid-dioxane solution (10 ml) was added thereto and the solution was further stirred for 3 days. With cooling with ice in water, diethyl ether was added to it, and the solid was collected by filtration to obtain 4,5-dimethyl-2-(2-methylthiophen-5-yl)oxazole 3-oxide hydrochloride (2.3 g) as a brown solid. This was dissolved in chloroform (30 ml), and phosphorus oxychloride (2 ml) was added thereto and the resulting solution was heated under reflux for 4 hours. After cooled and diluted with chloroform, the solution was washed with water and saturated brine, and then dried over anhydrous sodium sulfate. The solvent was evaporated under reduced pressure, and the resulting residue was purified by silica gel column chromatography (ethyl acetate/hexane = 1/4) to obtain the entitled compound (1.34 g) as a pale yellow solid.
¹H-NMR (400 MHz, CDCl₃) δ: 2.39 (3H, s), 2.52 (3H, s), 4.51 (2H, s), 6.74 (1H, d, J=3.7 Hz), 7.42 (1H, d, J=3.7 Hz).
MS m/z: 228(M+H)⁺.

### [Reference Example 33]

### (1) Ethyl 2-[4-[[(9H-fluoren-9-ylmethoxycarbonyl)-[(2-hydroxypropylcarbamoyl)methyl]amino]methyl]-2,6-dimethylphenoxy]-2-methylpropanoate

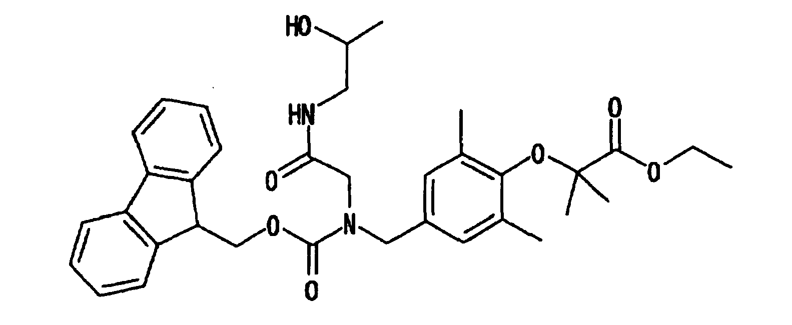

The compound (1.70 g) obtained in Reference Example 30-(2) was dissolved in methanol (30 ml), and 4-(4,6-dimethoxy-1,3,5-triazin-2-yl)-4-methylmorpholinium chloride n-hydrate (1.29 g) and 1-amino-2-propanol (0.36 m) were added thereto and the resulting solution was stirred at room temperature for 20 hours. The reaction solution was concentrated under reduced pressure, and the residue was dissolved in ethyl acetate. The solution was washed with water, an aqueous 10% citric acid solution, an aqueous saturated sodium bicarbonate solution and saturated brine in that order, and dried over anhydrous sodium sulfate. The solvent was evaporated under reduced pressure, and the resulting residue was purified by silica gel column chromatography (methanol/chloroform = 1/9) to obtain the entitled compound (1.89 g) as a pale yellow oily substance.
MS m/z:603(M+H)⁺.

### (2) Ethyl 2-[4-[[(9H-fluoren-9-ylmethoxycarbonyl)-[(2-oxopropylcarbamoyl)methyl]amino]methyl]-2,6-dimethylphenoxy]-2-methylpropanoate

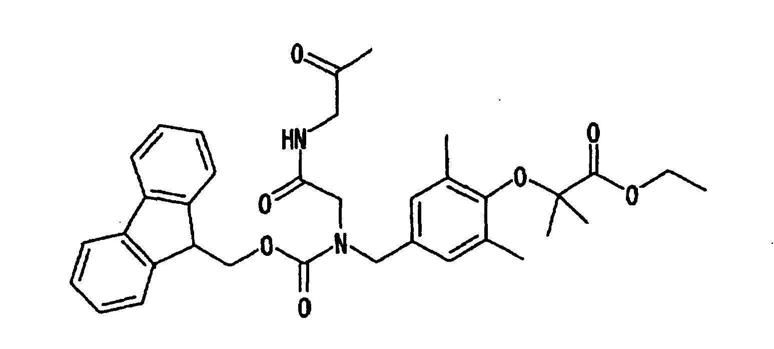

In the same manner as in Reference Example 22-(2), the entitled compound (1.1 g) was obtained as a pale yellow solid from the compound (1.89 g) obtained in Reference Example 33-(1).
¹H-NMR (400 MHz, CDCl₃) δ: 1.35 (3H, t, J=7.1 Hz), 1.45 (6H, s), 2.16-2.18 (9H, m), 3.69-4.13 (4H, m), 4.26-4.31 (3H, m), 4.42 (2H, s), 4.56 (2H, d, J=6.6 Hz), 6.73 (1H, s), 6.83 (1H, s), 7.23-7.29 (9H, m), 7.38 (2H, t, J=7.5 Hz), 7.56-7.54 (2H, m), 7.74 (2H, d, J=7.6 Hz).
MS m/z: 601(M+H)⁺.

### (3) Ethyl 2-[4-[[(9H-fluoren-9-ylmethoxycarbonyl)-(5-methyloxazol-2-ylmethyl)amino]methyl]-2,6-dimethylphenoxy]-2-methylpropanoate

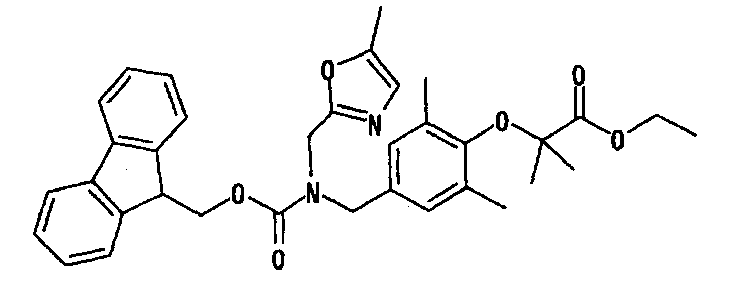

Triphenylphosphine (1.01 g) was dissolved in dichloromethane (50 ml), and with cooling with ice in water, hexachloroethane (0.78 g) and triethylamine (0.51 ml) were added thereto. Then, the compound (1.10 g) obtained in Reference Example 33-(2) was added thereto and the resulting solution was stirred for 1 hour, and then further stirred at room temperature for 2 days. The reaction solution was concentrated under reduced pressure, and the residue was dissolved in ethyl acetate. The solution was washed with water, an aqueous 10% citric acid solution, an aqueous saturated sodium bicarbonate solution and saturated brine in that order, and dried over anhydrous sodium sulfate. The solvent was evaporated under reduced pressure, and the resulting residue was purified by silica gel column chromatography (ethyl acetate/hexane = 3/1) to obtain the entitled compound (0.71 g) as a pale yellow oily substance.
MS m/z: 583(M+H)⁺.

### (4) Ethyl 2-[2,6-dimethyl-4-[[(5-methyloxazol-2-ylmethyl)amino]methyl]phenoxy]-2-methylpropanoate

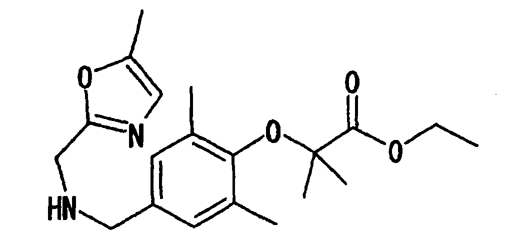

The compound (0.71 g) obtained in Reference Example 33-(3) was dissolved in tetrahydrofuran (10 ml), and with cooling with ice in water, diethylamine (1.26 ml) was added thereto, and the resulting solution was stirred at room temperature for 23 hours. The solvent was evaporated under reduced pressure, and the resulting residue was purified by silica gel column chromatography (methanol/chloroform = 1/9) to obtain the entitled compound (0.44 g) as a pale yellow oily substance.
MS m/z: 361(M+H)⁺.

### [Reference Example 34]

### (1) Ethyl 2-[4-[[tert-butoxycarbonylmethyl-5-(methyl-2-p-tolyloxazol-4-ylmethyl)amino]methyl]-2,6-dimethylphenoxy]-2-methylpropanoate

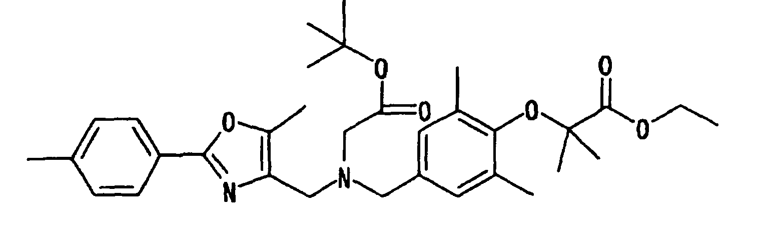

4-Chloromethyl-5-methyl-2-p-tolyloxazole (0.127 g) and the compound (0.198 g) obtained in Reference Example 14-(1) were dissolved in acetonitrile (5 ml), and potassium carbonate (0.087 g) was added thereto and the resulting mixture was stirred overnight at 50°C and then at 70°C for 4 days. After cooled, the insoluble material was removed by filtration, and the filtrate was concentrated under reduced pressure. The residue was purified by silica gel column chromatography (13% ethyl acetate-hexane) to obtain the entitled compound (0.258 g) as a colorless oily substance.
¹H-NMR (400 MHz, CDCl₃) δ: 1.35 (3H, t, J=7.2 Hz), 1.45 (6H, s), 1.47 (9H, s), 2.17 (6H, s), 2.29 (3H, s), 2.38 (3H, s), 3.30 (2H, s), 3.71 (2H, br s), 3.74 (2H, br s), 4.28 (2H, q, J=7.2 Hz), 6.99 (2H, s), 7.23 (2H, d, J=8.3 Hz), 7.89 (2H, d, J=8.3 Hz).
MS m/z: 565(M+H)⁺.

### (2) Ethyl 2-[4-[[carboxymethyl-(5-methyl-2-p-tolyloxazol-4-ylmethyl)amino]methyl]-2,6-dimethylphenoxy]-2-methylpropanoate

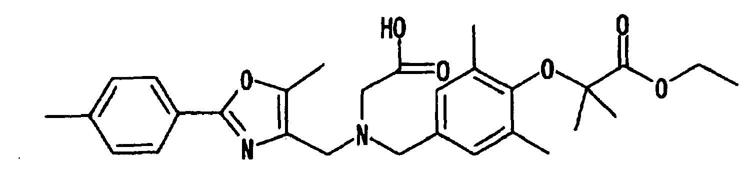

The compound (0.258 g) obtained in Reference Example 34-(1) was dissolved in dichloromethane (5.2 ml), and 4 N hydrochloric acid-dioxane solution (5.2 ml) was added thereto and the resulting solution was stirred at room temperature for 2 days. This was concentrated under reduced pressure to obtain the entitled compound (0.232 g) as a colorless oily substance.

### [Reference Example 35]

### (1) Ethyl 2-[4-[[tert-butoxycarbonylmethyl-[2-(4-chlorophenyl)-5-methyloxazol-4-ylmethyl]amino]methyl]-2,6-dimethylphenoxy]-2-methylpropanoate

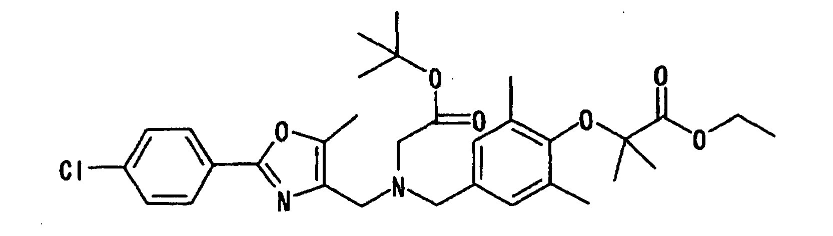

In the same manner as in Reference 34-(1), the entitled compound (0.289 g) was obtained as a colorless oily substance from 4-chloromethyl-2-(4-chlorophenyl)-5-methyloxazole (0.146 g) and the compound (0.208 g) obtained in Reference Example 14-(1).
¹H-NMR (400 MHz, CDCl₃) δ: 1.34 (3H, t, J=7.1 Hz), 1.44 (6H, s), 1.47 (9H, s), 2.17 (6H, s), 2.29 (3H, s), 3.29 (2H, s), 3.71 (2H, br s), 3.73 (2H, br s), 4.27 (2H, q, J=7.1 Hz), 6.98 (2H, s), 7.36-7.42 (2H, m), 7.90-7.96 (2H, m).
MS m/z: 585(M+H)⁺.

### (2) Ethyl 2-[4-[[carboxymethyl-[2-(4-chlorophenyl)-5-methyloxazol-4-ylmethyl]wnino]methyl]-2,6-dimethylphenoxy]-2-methylpropanoate

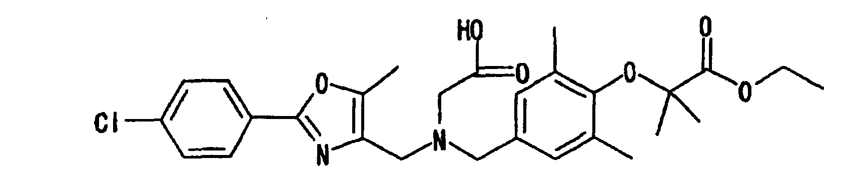

In the same manner as in Reference Example 34-(2), the entitled compound (0.261 g) was obtained as a colorless oily substance from the compound (0.289 g) obtained in Reference Example 35-(1).

### [Reference Example 36]

### (1) Ethyl 2-[4-[[tert-butoxycarbonylmethyl-[2-(3-chlorophenyl)-5-methyloxazol-4-ylmethyl]amino]methyl]-2,6-dimethylphenoxy]-2-methylpropanoate

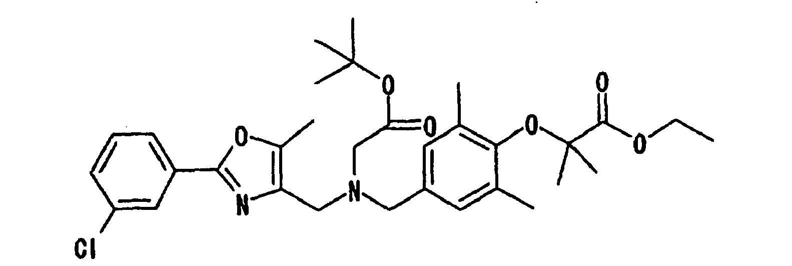

In the same manner as in Reference Example 34-(1), the entitled compound (0.236 g) was obtained as a colorless oily substance from 4-chloromethyl-2-(3-chlorophenyl)-5-methyloxazole (0.134 g) and the compound (0.190 g) obtained in Reference Example 14-(1).
¹H-NMR (400 MHz, CDCl₃) δ: 1.35 (3H, t, J=7.2 Hz), 1.45 (6H, s), 1.48 (9H, s), 2.18 (6H, s), 2.30 (3H, s), 3.30 (2H, s), 3.71 (2H, br s), 3.75 (2H, br s), 4.28 (2H, q, J=7.2 Hz), 6.99 (2H, s); 7.34-7.38 (2H, m), 7.85-7.91 (1H, m), 8.00-8.02 (1H, m).
MS m/z: 585(M+H)⁺.

### (2) Ethyl 2-[4-[[carboxymethyl-[2-(3-chlorophenyl)-5-methyloxazol-4-ylmethyl]amino]methyl]-2,6-dimethylphenoxy]-2-methylpropanoate

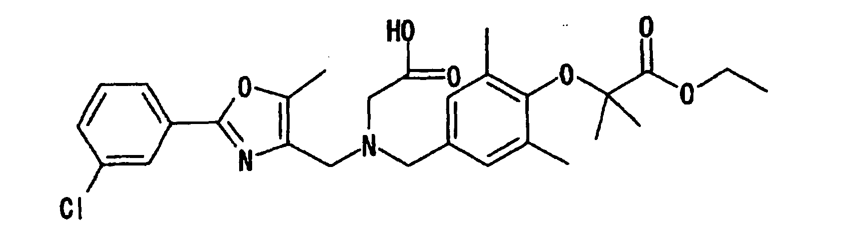

In the same manner as in Reference Example 34-(2), the entitled compound (0.214 g) was obtained as a colorless oily substance from the compound (0.236 g) obtained in Reference Example 36-(1).

### [Reference Example 37]

### (1) tert-Butyl 2-(4-formyl-2-methoxy-6-methylphenoxy)-2-methylpropanoate

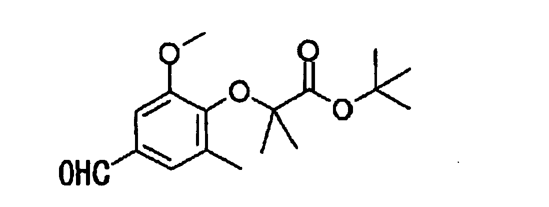

4-Hydroxy-3-methyl-5-methoxybenzaldehyde (7.0 g) was dissolved in N,N-dimethylformamide (110 ml), and potassium carbonate (17.4 g) and tert-butyl 2-bromo-2-methylpropanoate (23.5 ml) were added thereto and the resulting mixture was stirred at 80°C for 27 hours. After cooled and filtered through Celite, the filtrate was diluted with ethyl acetate, and washed with water. The aqueous layer was extracted with ethyl acetate, the organic layers were combined, washed twice with saturated brine, and dried over anhydrous sodium sulfate. The solvent was evaporated under reduced pressure, and the resulting residue was purified by silica gel column chromatography (ethyl acetate/hexane = 1/4) to obtain the entitled compound (4.25 g) as a pale yellow oily substance.
¹H-NMR (400 MHz, CDCl₃) δ: 1.47 (6H, s), 2.31 (3H, s), 3.80 (3H, s), 7.24-7.30 (2H, m), 9.84 (1H, s).
MS m/z: 309(M+H)⁺.

### (2) tert-Butyl 2-[2-methoxy-6-methyl-4-[[(5-methyl-2-phenyloxazol-4-ylmethyl)amino]methyl]phenoxy]-2-methylpropanoate

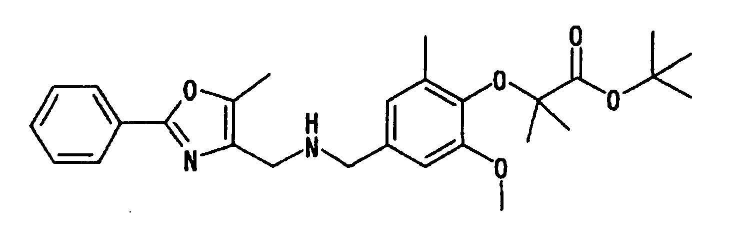

In the same manner as in Reference Example 19, the entitled compound (2.3 g) was obtained as a brown oily substance from C-[5-methyl-2-phenyloxazol-4-yl]methylamine (1.0 g) and the compound (1.63 g) obtained in Reference Example 37-(1).

¹H-NMR (400 MHz, CDCl₃) δ: 1.40 (6H, s), 1.51 (9H, s), 2.21 (3H, s), 2.31 (3H, s), 3.67 (2H, s), 3.70 (3H, s), 3.73 (2H, s), 6.70-6.73 (2H, m), 7.38-7.49 (3H, m), 8.01-7.97 (2H, m).
MS m/z: 481(M+H)⁺.

### (3) tert-Butyl 2-[4-[[carboxymethyl-(5-methyl-2-phenyloxazol-4-ylmethyl)amino]methyl]-2-methoxy-6-methylphenoxy-2-methylpropanoate

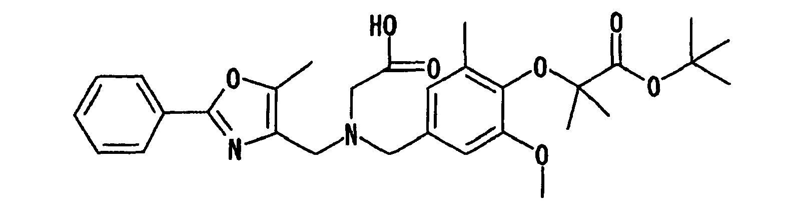

In the same manner as in Reference Example 6, the entitled compound (0.12 g) was obtained as a pale yellow oily substance from the compound (0.20 g) obtained in Reference Example 37-(2).
¹H-NMR (400 MHz, CDCl₃) δ: 1.40 (6H, s), 1.50 (9H, s), 2.21 (3H, s), 2.27 (3H, s), 3.44 (2H, s), 3.67 (2H, s), 3.68 (3H, s), 3.76 (2H, s), 6.67 (1H, s), 6.69 (1H, s), 7.43-7.47 (3H, m), 7.97-8.01 (2H, m).
MS m/z: 539(M+H)⁺.

### [Reference Example 38]

### Ethyl 2-(2-fluoro-4-formylphenoxy)-2-methylpropanoate

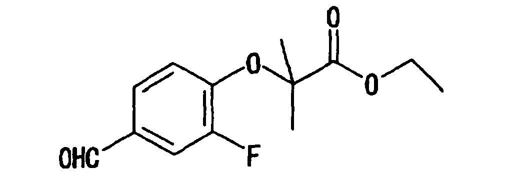

In the same manner as in Reference Example 2, the entitled compound (1.54 g) was obtained as a colorless oily substance from 3-fluoro-4-hydroxybenzaldehyde (2.5 g) and ethyl 2-bromo-2-methylpropanoate (7.0 g).
MS m/z: 255(M+H)⁺.

### [Reference Example 39]

### 5-Phenylisoxazol-3-ylmethyl methanesulfonate

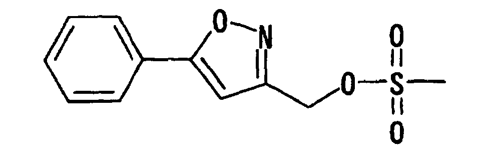

3-Hydroxymethyl-S-phenylisoxazole (0.101 g) was dissolved in dichloromethane (4 ml), and with cooling with ice in water, methanesulfonyl chloride (0.048 ml) and triethylamine (0.094 ml) were added thereto and the resulting solution was stirred for 1 hour. An aqueous saturated sodium bicarbonate solution was added to it, then extracted three times with dichloroethane, and washed with saturated brine. This was dried over anhydrous sodium sulfate, and the solvent was evaporated under reduced pressure to obtain the entitled compound as an oily substance. The entitled compound was used in the next reaction without purification.

### [Reference Example 40]

### Ethyl 2-(2-ethyl-4-formyl-6-methylphenoxy)-2-methylpropanoate

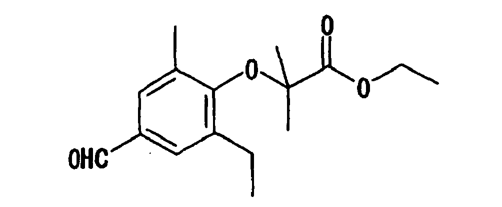

In the same manner as in Reference Example 12, the entitled compound (2.46 g) was obtained as a pale yellow oily substance from 3-ethyl-4-hydroxy-5-methylbenzaldehyde (2.08 g) and ethyl 2-bromo-2-methylpropanoate (7.0 g).
¹H-NMR (400 MHz, CDCl₃) δ: 1.24 (3H, t, J=7.4 Hz), 1.36 (3H, t, J=7.1 Hz), 1.50 (6H, s), 2.28 (3H, s), 2.65 (2H, q, J=7.4 Hz), 4.30 (2H, q, J=7.1 Hz), 7.53 (1H, d, J=2.2 Hz), 7.58 (1H, d, J=2.2 Hz), 9.90 (1H, s).
MS m/z: 279(M+H)⁺.

### [Reference Example 41]

### (1) tert-Butyl 2-(4-formyl-2,6-dimethylphenoxy)-2-methylpropanoate

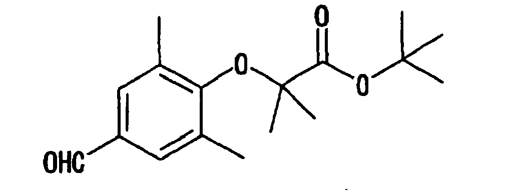

In the same manner as in Reference Example 37-(1), the entitled compound (4.47 g) was obtained as a pale yellow oily substance by processing 3,5-dimethyl-4-hydroxybenzaldehyde (15.0 g) and tert-butyl 2-bromo-2-methylpropanoate (56 ml) in the presence of potassium carbonate (55.3 g) at 80°C for 2 days.
¹H-NMR (400 MHz, CDCl₃) δ: 1.41 (6H, s), 1.47 (9H, s), 2.25 (6H, s), 7.48 (2H, s), 9.83 (1H, s).

### (2) tert-Butyl 2-[2,6-dimethyl-4-[[(5-methyl-2-phenyloxazol-4-ylmethyl)amino]methyl]phenoxy]-2-methylpropanoate

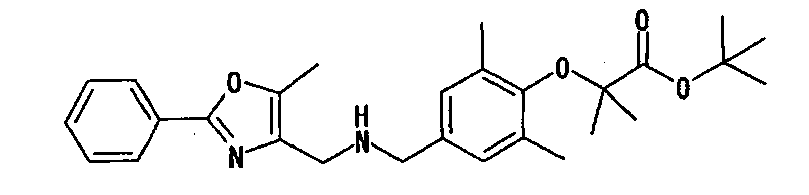

In the same manner as in Reference Example 19, the entitled compound (5.8 g) was obtained as a pale yellow oily substance from C-(5-methyl-2-phenyloxazol-4-yl)methylamine (1.94 g) and the compound (3.0 g) obtained in Reference Example 41-(1).
MS m/z: 465(M+H)⁺.

### (3) tert-Butyl 2-[4-[[carboxymethyl-(5-methyl-2-phenyloxazol-4-ylmethyl)amino]methyl]-2,6-dimethylphenoxy]-2-methylpropanoate

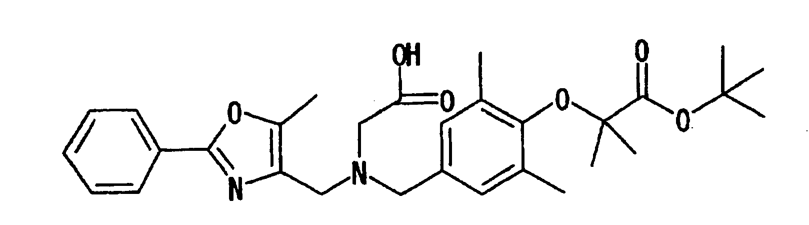

In the same manner as in Reference Example 6, the entitled compound (0.90 g) was obtained as a colorless oily substance from the compound (1.00 g) obtained in Reference Example 41-(2).
¹H-NMR (400 MHz, CDCl₃) δ: 1.42 (6H, s), 1.51 (9H, s), 2.21 (6H, s), 2.25 (3H, s), 3.45 (2H, s), 3.67 (2H, s), 3.74 (2H, s), 6.92 (2H, s), 7.44-7.47 (3H, m), 8.01-7.98 (2H, m).
MS m/z: 523(M+H)⁺.

### (4) tert-Butyl 2-[2,6-dimethyl4-[[(5-methyl-2-phenyloxazol4-ylmethyl)-[N'-(2,2,2-trifluoroacetyl)-hydrazinocarbonylmethyl]amino]methyl]phenoxy]-2-methylpropanoate

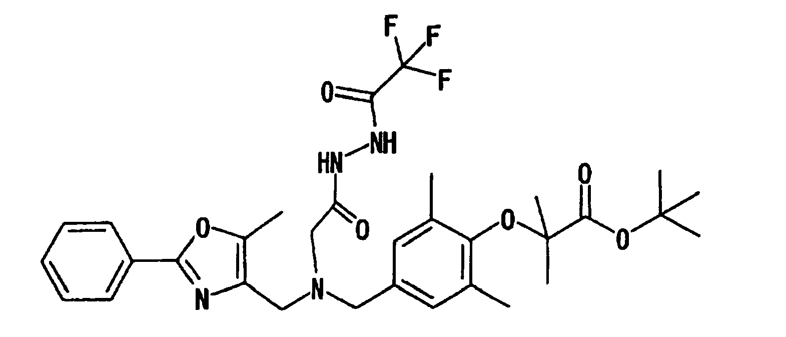

In the same manner as in Reference Example 16-(1), the entitled compound (0.135 g) was obtained as a pale yellow oily substance from the compound (0.150 g) obtained in Reference Example 41-(3) and trifluoroacetic acid hydrazide (0.046 g).
MS m/z: 633(M+H)⁺.

### [Reference Example 42]

### (1) tert-Butyl 2-[4-[[[2-[N'-(9H-fluoren-9-ylmethoxycarbonyl)hydrazino]-2-oxoethyl]-(5-methyl-2-phenyloxazol-4-ylmethyl)amino]methyl]-2,6-dimethylphenoxy]-2-methylpropanoate

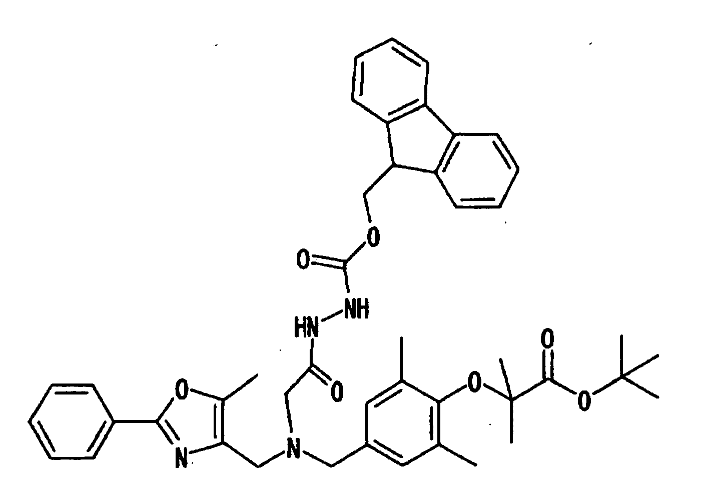

In the same manner as in Reference Example 16-(1), the entitled compound (0.48 g) was obtained as an yellow oily substance from the compound (0.350 g) obtained in Reference Example 41-(3) and 9H-fluoren-9-ylmethyl hydrazinecarboxylic acid (0.24 g).
¹H-NMR (400 MHz, CDCl₃) δ: 1.26 (3H, t, J=7.2 Hz), 1.40 (6H, s), 1.50 (9H, s), 2.04 (2H, s), 2.18 (6H, s), 2.23 (3H, s), 3.40 (2H, s), 3.57-3.65 (4H, m), 4.12 (2H, q, J=7.2 Hz), 4.43 (2H, d, J=7.3 Hz), 6.90 (2H, s), 7.26 (2H, s), 7.42-7.40 (5H, m), 7.56-7.58 (2H, m), 7.72-7.75 (2H, m), 7.97-7.99 (2H, m).

### (2) tert-Butyl 2-[4-[[hydrazinocarbonyhnethyl-(5-methyl-2-phenyloxazol-4-ylmethyl)amino]methyl]-2,6-dimethylphenoxy]-2-methylpropanoate

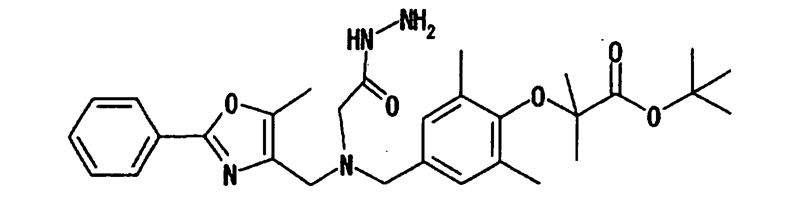

The compound (0.48 g) obtained in Reference Example 42-(1) was dissolved in tetrahydrofuran (20 ml), and with cooling with ice in water, diethylamine (0.98 ml) was added thereto, and the resulting solution was stirred at room temperature for 2 hours. The solvent was evaporated under reduced pressure, the resulting residue was dissolved in ethyl acetate, washed with an aqueous saturated sodium bicarbonate solution and then with saturated brine, and dried over anhydrous sodium sulfate. The solvent was evaporated under reduced pressure, and the resulting residue was purified by silica gel column chromatography (methanol/chloroform = 1/9) to obtain the entitled compound (0.3 g) as an yellow oily substance.
MS m/z: 537(M+H)⁺.

### [Reference Example 43]

### tert-Butyl 2-(2,6-difluoro-4-formylphenoxy)-2-methylpropanoate

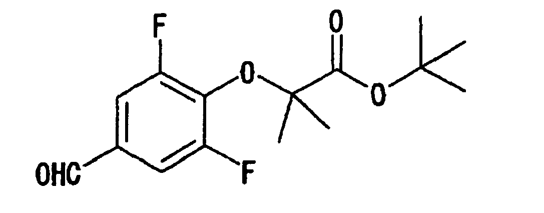

In the same manner as in Reference Example 12, the entitled compound (6.68 g) was obtained as a colorless oily substance by processing 3,5-difluoro-4-hydroxybenzaldehyde (3.8 g) and tert-butyl 2-bromo-2-methylpropanoate (20 g) in the presence of cesium carbonate (12.6 g) at 80°C for 3 days.
¹H-NMR
(400 MHz, CDCl₃) δ: 1.48 (9H, s), 1.59 (6H, s), 7.44 (2H, d, J=7.8 Hz), 9.85 (1H, t, J=1.6 Hz).
MS m/z: 323(M+H)⁺.

### [Reference Example 44]

### tert-Butyl 2-(3-formyl-4-methoxyphenoxy)-2-methylpropanoate

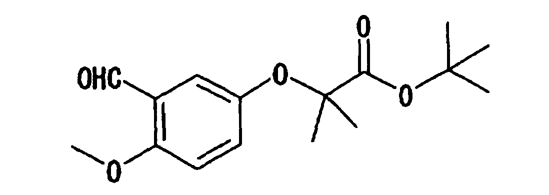

In the same manner as in Reference Example 12, the entitled compound (2.62 g) was obtained as a pale yellow oily substance from 5-hydroxy-2-methoxybenzaldehyde (2.1 g) and tert-butyl 2-bromo-2-methylpropanoate (9 g).
¹H-NMR (400 MHz, CDCl₃) δ: 1.48 (9H, s), 1.53 (6H, s), 3.89 (3H, s), 6.89 (1H, d, J=9.1 Hz), 7.16 (1H, dd, J=9.1,3.2 Hz), 7.34 (1H, d, J=3.2 Hz), 10.41 (1H, s).
MS m/z: 295(M+H)⁺.

### [Reference Example 45]

### (1) Ethyl 2-[4-[(benzyloxycarbonyl-tert-butoxycarbonylinethylamino)methyl]-2,6-dimethylphenoxy]-2-methylpropanoate

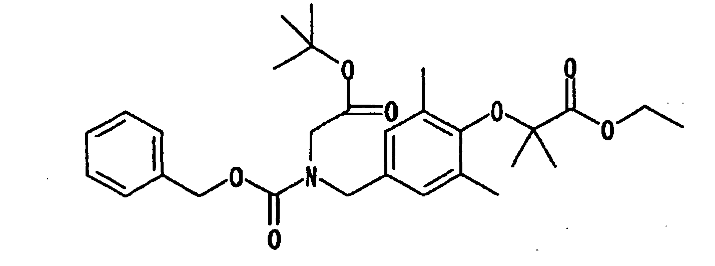

Dichloromethane (20 ml) solution of the compound (1.0 g) obtained in Reference Example 14-(1) and an aqueous saturated sodium bicarbonate solution (20 ml) were mixed, and with cooling with ice in water, benzyloxycarbonyl chloride (0.49 ml) was added thereto, and the resulting solution was stirred at room temperature for 18 hours. The reaction solution was diluted with ethyl acetate, washed with water and then saturated brine, and dried over anhydrous sodium sulfate. The solvent was evaporated under reduced pressure, and the resulting residue was purified by silica gel column chromatography (ethyl acetate/hexane = 1/2) to obtain the entitled compound (1.35 g) as a colorless oily substance.
MS m/z: 536(M+Na)⁺.

### (2) Ethyl 2-[4-[(benzyloxycarbonyl-carbamoylmethylamino)methyl]-2,6-dimethylphenoxy]-2-methylpropanoate

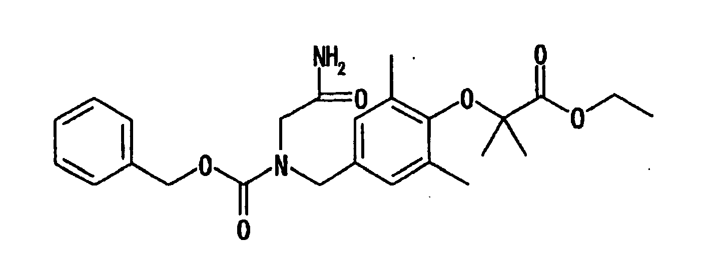

The compound (1.35 g) obtained in Reference Example 45-(1) was dissolved in dichloromethane (20 ml), and with cooling with ice in water, trifluoroacetic acid (5 ml) was added thereto, and the resulting solution was stirred at room temperature for 18 hours. The solvent was evaporated under reduced pressure, and the resulting residue was purified by silica gel column chromatography (methanol/chloroform = 1/19) to obtain ethyl 2-[4-[(benzyloxycarbonyl-carboxymethylamino)methyl]-2,6-dimethylphenoxy]-2-methylpropanoate (1.2 g) as a colorless oily substance. This was dissolved in acetonitrile (20 ml), and ammonium chloride (0.28 g), 1-(3-dimethylaminopropyl)-3-ethylcarbodiimide hydrochloride (0.76 g), 1-hydroxybenzotriazole (0.61 g) and N-methylmorpholine (0.58 ml) were added thereto, and the resulting solution was stirred at room temperature for 4 hours. The solvent was evaporated under reduced pressure, and the resulting residue was dissolved in ethyl acetate, and washed with water, an aqueous 10% citric acid solution, an aqueous saturated sodium bicarbonate solution and saturated brine in that order. This was dried over anhydrous sodium sulfate, and the solvent was evaporated under reduced pressure. The resulting residue was purified by silica gel column chromatography (methanol/chloroform = 1/9) to obtain the entitled compound (1.1 g) as a colorless oil.
MS m/z: 457(M+H)⁺.

### (3) Ethyl

### 2-[4-[[benzyloxycarbonyl-(3-methyl[1,2,4]oxadiazol-5-ylmethyl)amino]methyl]-2,6-dimethylphenoxy]-2-methylpropanoate

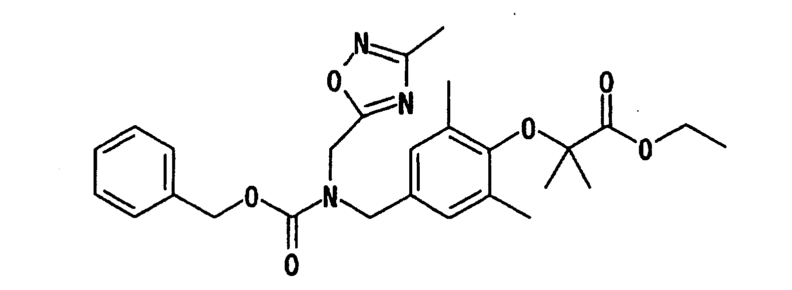

In the same manner as in Reference Example 19-(2), the entitled compound (0.73 g) was obtained as a pale yellow oily substance from the compound (1.10 g) obtained in Reference Example 45-(2).
¹H-NMR (400 MHz, CDCl₃) δ: 1.35 (3H, t, J=7.2 Hz), 1.45 (6H, s), 2.12,2.16 (6H, each s), 2.35,2.38 (3H, each s), 4.28 (2H, q, J=7.2 Hz), 4.51 (2H, s), 4.55,4.60 (2H, each s), 5.20,5.24 (2H, each s), 6.75,6.86 (2H, each s), 7.36-7.29 (5H, m).
MS m/z: 496(M+H)⁺.

### (4) Ethyl 2-[2,6-dimethyl-4-[[(3-methyl-[1,2,4]oxadiazol-5-ylmethyl)amino]methyl]phenoxy]-2-methylpropanoate

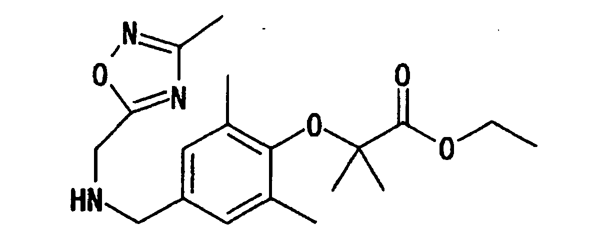

The compound (0.73 g) obtained in Reference Example 45-(3) was dissolved in dichloromethane (5 ml), and with cooling with ice in water, hydrogen bromide (30% acetic acid solution, 5 ml) was added thereto, and the resulting solution was stirred at room temperature for 6 hours. This was concentrated to dryness in vacuo to obtain a hydrobromide of the entitled compound (0.345 g) as a brown oily substance, which was used in the next reaction directly as it was.
¹H-NMR (400 MHz, CD₃OD) δ: 1.23 (3H, t, J=7.1 Hz), 1.36 (6H, s), 2.13 (6H, s), 2.32 (3H, s), 4.15 (2H, q, J=7.1 Hz), 4.22 (2H, s), 4.77 (2H, s), 7.10(2H, s).

### [Reference Example 46]

### Ethyl 2-[2,6-dimethyl-4-[[(5-methyl-2-phenyloxazol-4-ylmethyl)-[2-oxo-2-[N'-(pyridine-3-carbonyl)hydrazino]ethyl]amino]methyl]phenoxy]-2-methylpropanoate

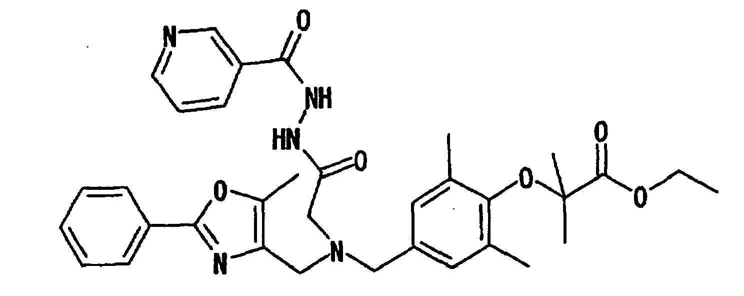

In the same manner as in Reference Example 16-(1), the entitled compound (0.170 g) was obtained as an yellow oily substance from the compound (0.200 g) obtained in Reference Example 14-(3) and nicotinic acid hydrazide (0.078 g).
¹H-NMR (400 MHz, CDCl₃) δ: 1.04 (3H, t, J=7.2 Hz), 1.15 (6H, s), 1.89 (6H, s), 1.96 (3H, s), 3.16 (2H, s), 3.35 (2H, s), 3.39 (2H, s), 3.97 (2H, q, J=7.0 Hz), 6.71 (2H, s), 7.07-7.11 (3H, m), 7.70-7.73 (2H, m), 7.76-7.79 (1H, m), 8.23-8.22 (1H, m), 8.45-8.47 (1H, m), 8.70 (1H, s).
MS m/z: 614(M+H)⁺.

### [Example 1]

### (1) tert-Butyl 2-[4-[[[2-(3-bromophenyl)-5-methyloxazol-4-ylmethyl]thiazol-2-ylmethylamino]methyl]phenoxy-2-methylpropionate

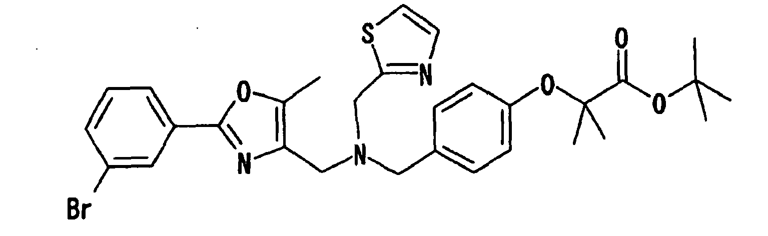

The compound (75 mg) of Reference Example 3 and 2-formylthiazole (19 µl) were dissolved in dichloromethane (1 ml), and sodium triacetoxyborohydride (62 mg) were added thereto and the resulting mixture was stirred for a day. The reaction solution was diluted with dichloromethane, washed with an aqueous sodium bicarbonate solution and saturated brine in that order, then dried over anhydrous sodium sulfate, and the solvent was evaporated under reduced pressure. The residue was purified by preparative thin-layer chromatography (hexane-ethyl acetate) to obtain the entitled compound (64 mg) as a pale yellow oily substance.

¹H-NMR (400 MHz, CDCl₃) δ: 1.43 (9H, s), 1.57 (6H, s), 2.27 (3H, s), 3.64 (2H, s), 3.69 (2H, s), 4.06 (2H, s), 6.83 (2H, d, J=8.6 Hz), 7.28-7.31 (4H, m), 7.53 (1H, d, J=9.1 Hz), 7.69 (1H, d, J=3.4 Hz), 7.92 (1H, d, J=7.8 Hz), 8.14 (1H, s).
MS m/z: 614(M+H)⁺.

### (2) 2-[4-[[[2-(3-Bromophenyl)-5-methyloxazol-4-ylmethyl]thiazol-2-ylmethylamino]methyl]phenoxy-2-methylpropionic acid hydrochloride

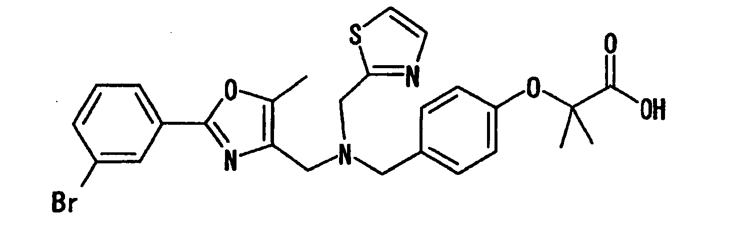

The compound (64 g) of Example 1-(1) was dissolved in dichloromethane (1.0 ml), and 4 N hydrochloric acid-dioxane solution (2 ml) was added thereto and the resulting solution was stirred at room temperature for a day. The solvent was evaporated under reduced pressure, and the residue was recrystallized from acetone-hexane to obtain the entitled compound (55 mg) as a pale yellow solid.

¹H-NMR (400 MHz, DMSO-d₆) δ: 1.50 (6H, s), 2.32 (3H, s), 4.10 (2H, s), 4.25 (2H, s), 4.53 (2H, s), 6.84 (2H, d, J=8.8 Hz), 7.48-7.53 (3H, m), 7.72-7.74 (1H, m), 7.84-7.96 (3H, m), 8.08 (1H, s).
MS m/z: 556(M+H)⁺.

### [Example 2]

### (1) Ethyl 2-[4-[[[2-(3-bromophenoxy)-5-methyloxazol-4-ylmethyl]-(1-methyl-1H-imidazol-2-ylmethyl)amino]methyl]phenoxy]-2-methylpropionate

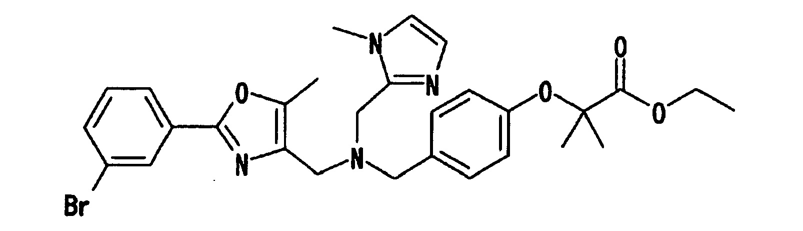

The compound (95 mg) of Reference Example 5 and 1-methyl-2-imidazolecarboxyaldehyde (32 mg) were dissolved in methylene chloride (2.0 ml), and sodium triacetoxyborohydride (83 mg) were added thereto and the resulting mixture was stirred for a day. The reaction solution was diluted with methylene chloride, washed with an aqueous sodium bicarbonate solution and saturated brine in that order, dried over anhydrous sodium sulfate, and the solvent was evaporated under reduced pressure. The residue was purified by preparative thin-layer chromatography (hexane-ethyl acetate) to obtain the entitled compound (76 mg) as a pale yellow oily substance.

¹H-NMR (400 MHz, CDCl₃) δ: 1.25 (3H, t, J=7.2 Hz), 1.57 (6H, s), 2.21 (3H, s), 3.50 (2H, s), 3.53 (3H, s), 3.59 (2H, s), 3.74 (2H, s), 4.23 (2H, q, J=7.1 Hz), 6.77 (1H, d, J=1.2 Hz), 6.79 (2H, d, J=7.0 Hz), 6.89 (1H, d, J=1.0 Hz), 7.19 (12H, d, J=8.6 Hz), 7.30 (1H, t, J=8.0 Hz), 7.53 (1H, d, J=9.1 Hz), 7.91 (1H, d, J=7.8 Hz), 8.14 (1H, s).
MS m/z: 583(M+H)⁺.

### (2) 2-[4-[[[2-(3-Bromophenoxy)-5-methyloxazol-4-ylmethyl]-(1-methyl-1H-imidazol-2-ylmethyl)amino]methyl]phenoxy]-2-methylpropionic acid

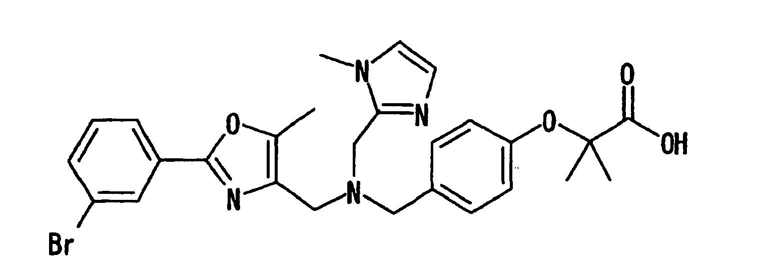

The compound (70 mg) of Example 2-(1) was dissolved in ethanol (10 ml), and an aqueous 1N sodium hydroxide solution (1 ml) and an aqueous 5 N sodium hydroxide solution (0.2 ml) were added thereto, and the resulting solution was heated under reflux for a day. The solvent was evaporated under reduced pressure, then the residue was neutralized with an aqueous 1N hydrochloric acid solution added thereto, and extracted with ethyl acetate. The resulting organic layer was washed with water and saturated brine in that order, dried over anhydrous sodium sulfate, and the solvent was evaporated under reduced pressure. The residue was purified by preparative thin-layer chromatography and recrystallized from acetone-hexane to obtain the entitled compound (29 mg) as a pale yellow solid.

¹H-NMR (400 MHz, DMSO-d₆) δ: 1.46 (6H, s), 2.25 (3H, s), 3.33-3.39 (5H, m), 3.43-3.46 (2H, m), 3.63-3.66 (2H, m), 6.76-6.78 (3H, m), 7.04 (1H, s), 7.19-7.22 (2H, m), 7.48 (1H, t, J=8.1 Hz), 7.69 (1H, d, J=7.8 Hz), 7.92 (1H, d, J=7.8 Hz), 8.03 (1H, s).
MS m/z: 555(M+H)⁺.

| Elementary Analysis, as C₂₇H₂₉BrN₄O4(H2O | | | |
|---|---|---|---|
| Calculated: | C,56.75; | H,5.47; | N,9.80. |
| Found: | C,57.01; | H,5.47; | N,9.30. |

### [Example 3]

### (1) Ethyl 2-[4-[[[2-(3-bromophenylr5-methyloxazol-4-ylmethyl]-(1-methyl-1H-benzimidazol-2-ylmethyl)amino]methylJphenoxy]-2-methylpropionate

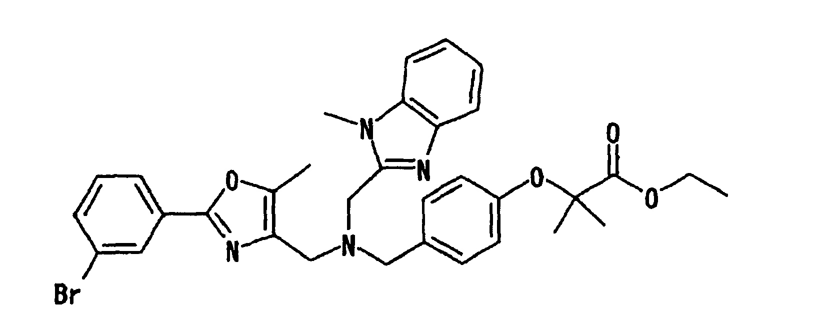

In the same manner as in Example 2-(1), the entitled compound (89 mg) was obtained as a colorless oily substance from the compound (95 mg) of Reference Example 5 and 1-methyl-2-formylbenzimidazole (47 mg).

1H-NMR (400 MHz, CDCl3) δ: 1.24 (3H, t, J=7.1 Hz), 1.56 (6H, s), 2.17 (3H, s), 3.56 (2H, s), 3.66, (2H, s), 3.73 (3H, s), 3.99 (2H, s), 4.23 (2H, q, J=7.1 Hz), 6.79 (2H, d, J=8.6 Hz), 7.20-7.32 (6H, m), 7.53 (1H, d, J=6.0 Hz), 7.70 (1H, d, J=5.8 Hz), 7.89 (1H, d, J=8.1 Hz), 8.11 (1H, s).
MS m/z: 633(M+H)+.

### (2) 2-[4-[[[2-(3-Bromophenyl)-5-methyloxazol-4-ylmethyl]-(1-methyl-1H-benzimidazol-2-ylmethyl)amino]methyl]phenoxy]-2-methylpropionic acid

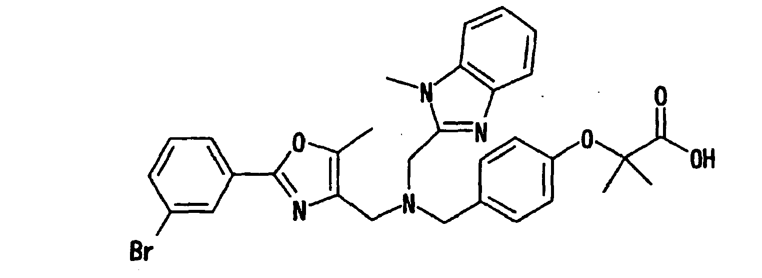

In the same manner as in Example 2-(2), the entitled compound (26 mg) was obtained as a pale yellow solid from the compound (85 mg) of Example 3-(1).
¹H-NMR (400 MHz, DMSO-d₆) δ: 1.46 (6H, s), 2.25 (3H, s), 3.33-3.39 (5H, m), 3.43-3.46 (2H, m), 3.63-3.66 (2H, m), 6.76-6.78 (3H, m), 7.04 (1H, s), 7:19-7.22 (2H, m), 7.48 (1H, t, J=8.1 Hz), 7.69 (1H, d, J=7.8 Hz), 7.92 (1H, d, J=7.8 Hz), 8.03 (1H, s).
MS m/z: 605(M+H)⁺.

| Elementary Analysis, as C₃₃H₃₁BrN₄O₅(0.75H₂O | | | |
|---|---|---|---|
| Calculated: | C,60.34; | H,5.31; | N,9.08. |
| Found: | C,60.74; | H,5.31; | N,8.63. |

### [Example 4]

### (1) Ethyl 2-[4-[[[2-(3-bromophenyl)-5-methyloxazol-4-ylmethyl]pyridin-4-ylmethylamino]methyl]phenoxy]-2-methylpropionate

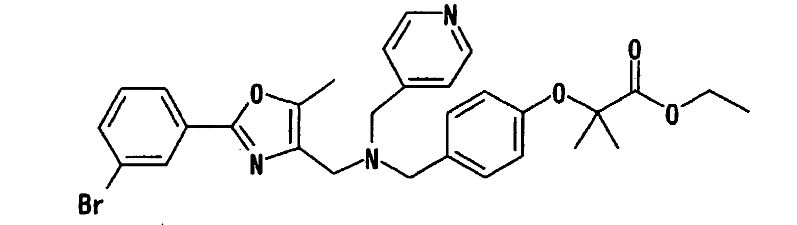

In the same manner as in Example 2-(1), the entitled compound (69 mg) was obtained as a colorless oily substance from the compound (75 mg) of Reference Example 5 and pyridine-4-aldehyde (22 µl).
¹H-NMR (400 MHz, CDCl₃) δ: 1.24 (3H, t, J=7.1 Hz), 1.56 (6H, s), 2.22 (3H, s), 3.52 (2H, s), 3.59, (2H, s), 3.59 (2H, s), 3.67 (2H, s), 4.23 (2H, q, J=7.1 Hz), 6.81 (2H, d, J=8.6 Hz), 7.24-7.35 (5H, m), 7.53 (1H, d, J=9.8 Hz), 7.91 (1H, d, J=7.8 Hz), 8.14 (1H, s), 8.53 (2H, d, J=5.9 Hz).
MS m/z: 580(M+H)⁺.

### (2) 2-[4-[[[2-(3-Bromophenyl)-S-methyloxazol-4-ylmethyl]pyridin-4-ylmethylamino]methyl]phenoxy]-2-methylpropionic acid

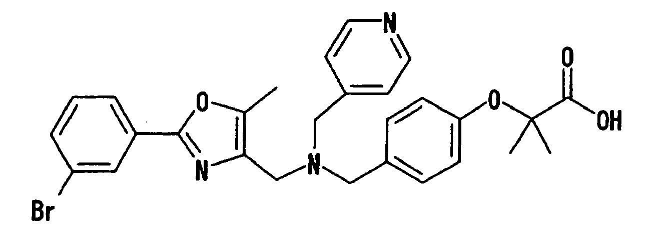

In the same manner as in Example 2-(2), the entitled compound (51 mg) was obtained as a colorless solid from the compound (67 mg) of Example 4-(1).
¹H-NMR (400 MHz, DMSO-d₆) δ: 1.47 (6H, s), 2.24 (3H, s), 3.48 (2H, s), 3.56 (2H, s), 3.64 (2H, s), 6.79 (2H, d, J=8.6 Hz), 7.29 (2H, d, J=8.1 Hz), 7.40 (2H, d, J=5.4 Hz), 7.49 (1H, t, J=7.8 Hz), 7.70 (1H, d, J=9.1 Hz), 7.92 (1H, d, J=7.8 Hz), 8.03 (1H, s), 8.49 (2H, d, J=4.9 Hz).
MS m/z: 550(M+H)⁺.

| Elementary Analysis, as C₂₈H₂₈BrN₃O₄•0.25H₂O•0.5EtOH | | | |
|---|---|---|---|
| Calculated: | C,60.26; | H,5.49; | N,7.27. |
| Found: | C,60.32; | H,5.55; | N,6.82. |

### [Example 5]

### (1) tert-Butyl 2-[4-[[[2-(3-bromophenyt)-5-methyloxazol-4-ylmethyl]thiazol4-ylmethylamino]methyl]phenoxy]-2-methylpropionate

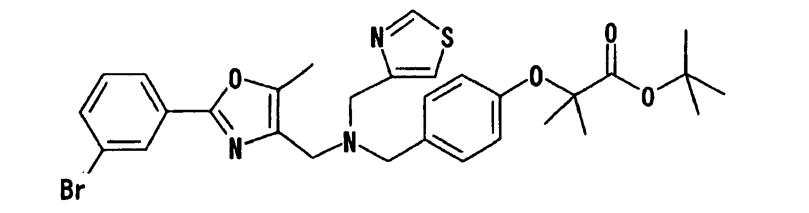

The compound (300 mg) of Reference Example 3 and 4-(chloromethyl)thiazole hydrochloride (129 mg) were dissolved in tetrahydrofuran (5 ml), and tetrabutylammonium iodide (108 mg) and triethylamine (3 ml) were added thereto and the resulting solution was heated under reflux for 5 days. The reaction solution was diluted with ethyl acetate, washed with an aqueous sodium bicarbonate solution, water, saturated brine in that order, dried over anhydrous sodium sulfate, and the solvent was evaporated under reduced pressure. The residue was purified by silica gel column chromatography (hexane-ethyl acetate) to obtain the entitled compound (140 mg) as an yellow oily substance.

¹H-NMR (400 MHz, CDCl₃) δ: 1.43 (9H, s), 1.45 (6H, s), 2.26 (3H, s), 3.59 (2H, s), 3.64 (2H, s), 3.95 (2H, s), 6.81 (2H, d, J=8.3 Hz), 7.22-7.3 5 (4H, m), 7.50-7.5 (1H, m), 7.91-7.95 (1H, m), 8.15 (1H, s), 8.78 (1H, s).
MS m/z: 614(M+H)⁺.

### (2) 2-[4-[[[2-(3-Bromophenyl)-5-methyloxazol-4-ylinethyl]thiazol-4-ylmethylamino]methyl]phenoxy]-2-methylpropionic acid hydrochloride

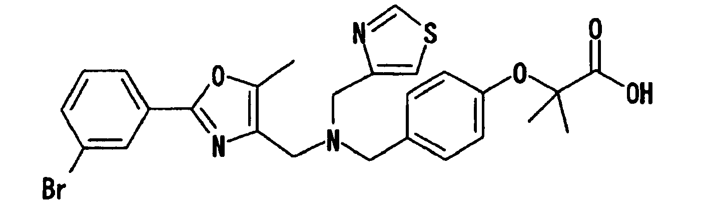

The compound (141 mg) of Example 5-(1) was dissolved in dichloromethane (3.0 ml), and 4 N hydrochloric acid-dioxane solution (5 ml) was added thereto and the resulting solution was stirred at room temperature for a day. The solvent was evaporated under reduced pressure, and the residue was purified by silica gel column chromatography (chloroform-methanol) and then dissolved in 4 N hydrochloric acid-dioxane solution. The resulting solution was concentrated under reduced pressure and the residue was recrystallized from acetone-ethyl acetate-hexane to obtain the entitled compound (83 mg) as a colorless solid.

¹H-NMR (400 MHz, DMSO-d₆) δ: 1.53 (6H, s), 2.34 (3H, s), 4.19 (2H, s), 4.39 (2H, s), 4.49 (2H, s), 6.86 (2H, d, J=8.3 Hz), 7.50-7.57 (3H, m), 7.76 (1H, d, J=4.7 Hz), 7.99 (1H, d, J=7.8 Hz), 8.02 (1H, s), 8.11 (1H, s), 9.26 (1H, s).
MS m/z: 558(M+H)⁺.

### [Example 6]

### (1) tert-Butyl 2-[4-[[[2-(3-bromophenyl)-5-methyloxazol-4-ylmethyl]-(2-methylthiazol-4-ylmethyl)amino]methyl]phenoxy]-2-methylpropionate

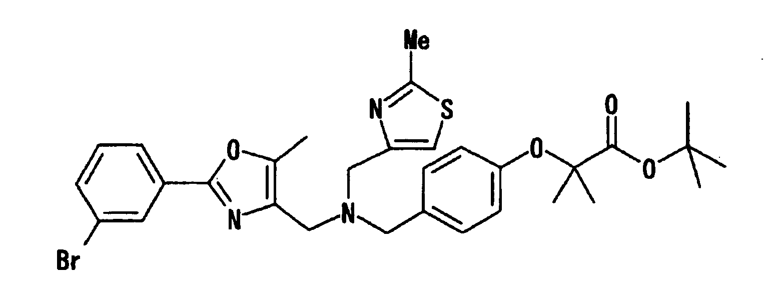

In the same manner as in Example 5-(1), the entitled compound (212 mg) was obtained as an yellow oily substance from the compound (300 mg) of Reference Example 3 and 4-chloromethyl-2-methylthiazole hydrochloride (140 mg).
¹H-NMR (400 MHz, CDCl₃) δ: 1.43 (9H, s), 1.53 (6H, s), 2.26 (3H, s), 2.70 (3H, s), 3.59 (2H, s), 3.64 (2H, s), 3.85 (2H, s), 6.81 (2H, d, J=8.3 Hz), 7.10 (1H, s), 7.24 (2H, d, J=6.9 Hz), 7.30 (1H, t, J=7.8 Hz), 7.50-7.54 (1H, m), 7.91-7.95 (1H, m), 8.15 (1H, s).
MS m/z: 628(M+H)⁺.

### (2) 2-[4-[[[2-(3-Bromophenyl)-5-methyloxazol-4-ylmethyl]-(2-methylthiazol-4-ylmethyl)amino]methyl]phenoxy]-2-methylpropionic acid hydrochloride

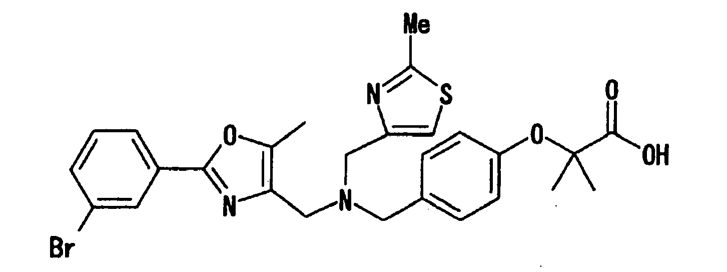

In the same manner as in Example 5-(2), the entitled compound (148 mg) was obtained as a colorless solid from the compound (210 mg) of Example 6-(1).
¹H-NMR (400 MHz, DMSO-d₆) δ: 1.53 (6H, s), 2.35 (3H, s), 2.69 (3H, s), 4.21 (2H, s), 4.39 (4H, bs), 6.87 (2H, d, J=8.8 Hz), 7.50-7.56 (3H, m), 7.77 (2H, d, J=7.4 Hz), 7.99 (1H, d, J=7.8 Hz), 8.11 (1H, s).
MS m/z: 570(M+H)⁺.

| Elementary Analysis, as C₂₇H₂₈BrN₃O₄S•2H₂O•HCl | | | |
|---|---|---|---|
| Calculated: | C,50.44; | H,5.17; | N,6.54. |
| Found: | C,50.44; | H,5.28; | N,6.12. |

### [Example 7]

### (1) tert-Butyl 2-[4-[[[2-(3-bromophenyl)-5-methyloxazol-4-ylmethyl]-(3,5-dimethylisoxazol-4-ylmethyl)amino]methyl]phenoxy]-2-methylpropionate

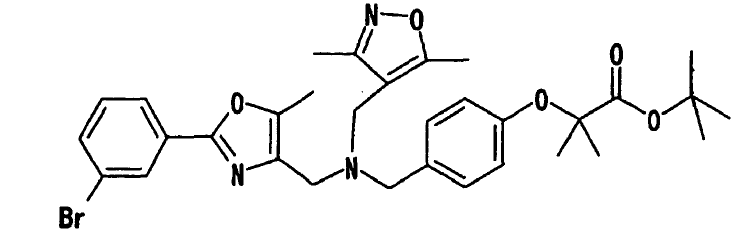

In the same manner as in Example 5-(1), the entitled compound (324 mg) was obtained as a colorless oily substance from the compound (300 mg) of Reference Example 3 and 4-chloromethyl-3,5-dimethylisoxazole (111 mg).
¹H-NMR (400 MHz, CDCl₃) δ: 1.44 (9H, s), 1.54 (6H, s), 2.17 (3H, s), 2.19 (3H, s), 2.36 (3H, s), 3.41 (2H, s), 3.42 (2H, s), 3.46 (2H, s), 6.81 (2H, d, J=8.5 Hz), 7.15 (2H, d, J=8.5 Hz), 6.31 (1H, t, J=8.1 Hz), 7.53 (1H, d, J=9.8 Hz), 7.91 (1H, d, J=7.8 Hz), 8.13 (1H, s).
MS m/z:626(M+H)⁺.

### (2) 2-[4-[[[2-(3-bromophenyl)-5-methyloxazol-4-ylmethyl]-(3,5-dimethylisoxazol-4-ylmethyl)amino]methyl]phenoxy]-2-methylpropionic acid hydrochloride

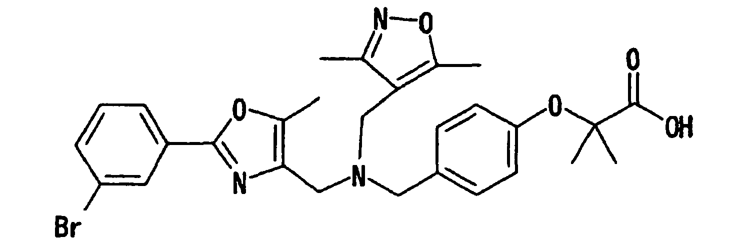

In the same manner as in Example 1-(2), the entitled compound (237 mg) was obtained as a colorless solid from the compound (141 mg) of Example 7-(1).
¹H-NMR (400 MHz, DMSO-d₆) δ: 1.52 (6H, s), 2.10 (3H, s), 2.32-2.43 (4H, m), 4.08-4.26 (4H, m), 4.31-4.48 (2H, m), 6.90 (2H, bs), 7.54 (3H, bs), 7.76 (1H, bs), 7.98 (1H, d, J=5.4 Hz), 8.12 (1H, s).
MS m/z: 570(M+H)⁺.

| Elementary Analysis, as C₂₈H₃₀BrN₃O₅•HCl | | | |
|---|---|---|---|
| Calculated: | C,55.59; | H,5.17; | N,6.95. |
| Found: | C,55.53; | H,5.36; | N,6.52. |

### [Example 8]

### (1) tert-Butyl 2-[4-[[[2-(3-bromophenyl)-5-methyloxazol4-ylmethyl]-methylcarbamoylmethylamino]methylphenoxy]-2-methylpropionate

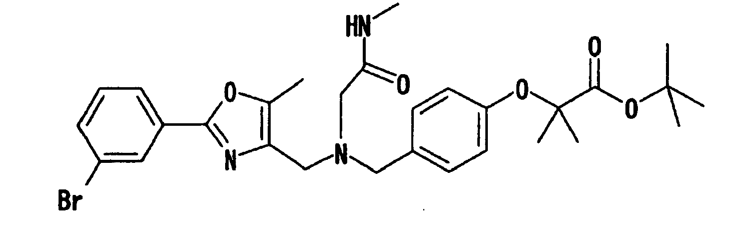

The compound (100 mg) of Reference Example 6 was dissolved in dimethylformamide (2 ml), and methylamine hydrochloride (20 mg), N-methylmorpholine (30 µl), 1-(3-dimethylaminopropyl)-3-ethylcarbodiimide hydrochloride (50 mg) and 1-hydroxybenzotriazole (35 mg) were added thereto and the resulting solution was stirred for a day. The reaction solution was diluted with ethyl acetate, washed with water and saturated brine in that order, and dried over anhydrous sodium sulfate. The solvent was evaporated under reduced pressure. The residue was purified by preparative thin-layer chromatography (chloroform-methanol) to obtain the entitled compound (97 mg) as a colorless oily substance.

¹H-NMR (400 MHz, CDCl₃) δ: 1.43 (9H, s), 1.58 (6H, s), 2.29 (3H, s), 2.79 (3H, d, J=4.9 Hz), 3.18 (2H, s), 3.51 (2H, s), 3.63 (2H, s), 6.81 (2H, d, J=8.3 Hz), 7.15 (2H, d, J=8.3 Hz), 7.33 (1H, t, J=7.8 Hz), 7.56 (1H, d, J=7.8 Hz), 7.63-7.69 (1H, m), 7.92 (1H, d, J=7.8 Hz), 8.15(1H, s).
MS m/z: 588(M+H)⁺.

### (2) 2-[4-[[[2-(3-Bromophenyl)-5-methyloxazol-4-ylmethyl]-methylcarbamoylmethylamino]methylphenoxy]-2-methylpropionic acid hydrochloride

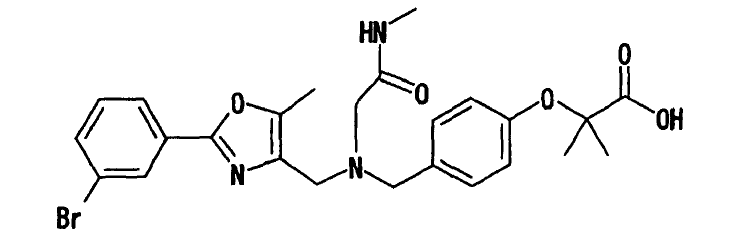

In the same manner as in Example 1-(2), the entitled compound (87 mg) was obtained as a colorless solid by processing the compound (95 mg) of Example 8-(1) followed by recrystallizing the resulting product from acetone-hexane.
¹H-NMR (400 MHz, CDCl₃) δ: 1.53 (6H, s), 2.43 (3H, s), 2.59 (3H, d, J=3.7 Hz), 3.82 (2H, s), 4.28-4.39 (4H, m), 6.87 (2H, d, J=8.6 Hz), 7.51-7.55 (3H, m), 7.76 (1H, d, J=8.1 Hz), 7.97 (1H, d, J=7.8 Hz), 8.11 (1H, s).
MS m/z: 532(M+H)⁺.

| Elementary Analysis, as C₂₅H₂₈BrN₃O₅•2H₂O-HCl | | | |
|---|---|---|---|
| Calculated: | C,49.80; | H,5.52; | N,6.97. |
| Found: | C,50.31; | H,5.23; | N,6.55. |

### [Example 9]

### (1) tert-Butyl 2-[4-[[[2-(3-bromophenyl)-5-methyloxazol-4-ylmethyl]dimethylcarbamoylmethylamino]methyl]phenoxy]-2-methylpropionate

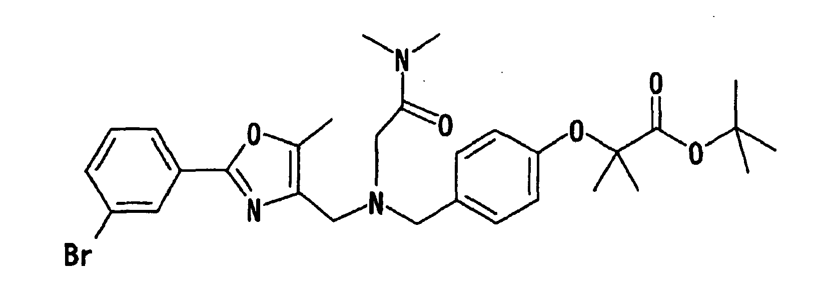

In the same manner as in Example 8-(1), the entitled compound (75 mg) was obtained as a colorless oily substance from the compound (100 mg) of Reference Example 6.
¹H-NMR (400 MHz, CDCl₃) δ: 1.44 (9H, s), 1.58 (6H, s), 2.29 (3H, s), 2.89 (6H, d, J=5.9 Hz), 3.36 (2H, s), 3.66 (2H, s), 3.71 (2H, s), 6.81 (2H, d, J=6.9 Hz), 7.23 (2H, d, J=8.3 Hz), 7.29 (1H, t, J=7.8 Hz), 7.53 (1H, d, J=7.4 Hz), 7.89-7.94 (1H, m), 8.15 (1H, s).
MS m/z: 602(M+H)⁺.

### (2) 2-[4-[[[2-(3-Bromophenyl)-5-methyloxazol-4-ylmethyl]dimethylcarbamoylmethylamino]methyl]phenoxy]-2-methylpropionic acid hydrochloride

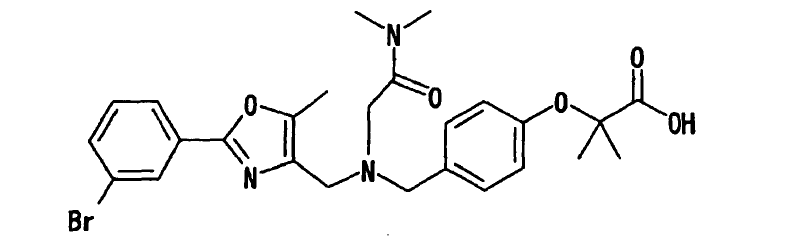

In the same manner as in Example 1-(2), the entitled compound (60 mg) was obtained as a colorless solid from the compound (70 mg) of Example 9-(1).
¹H-NMR (400 MHz, CDCl₃) δ: 1.54 (6H, s), 2.46 (3H, s), 2.82 (3H, s), 2.88 (3H, s), 4.19-4.39 (6H, m), 6.88 (2H, d, J=8.6 Hz), 7.52-7.60 (3H, m), 7.76 (1H, d, J=9.1 Hz), 7.98 (1H, d, J=7.8 Hz), 8.10 (1H, s).
MS m/z: 546(M+H)⁺.

### [Example 10]

### (1) tert-Butyl 2-[2-allyl-4-[[methylcarbamoylmethyl-(5-methyl-2-phenyloxazol-4-ylmethyl)amino]methyl]phenoxy]-2-methylpropanoate

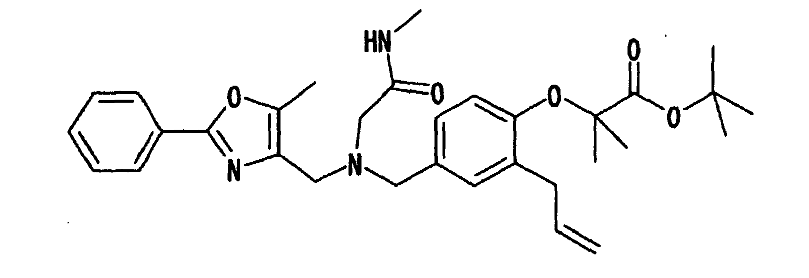

In the same manner as in Example 8-(1), the entitled compound (120.5 mg) was obtained as a solid from the compound obtained in Reference Example 9 and N-methylamine hydrochloride (100 mg).
¹H-NMR (400 MHz, CDCl₃) δ: 1.39 (9H, s), 1.56 (6H, s), 2.30 (3H, s), 2.77 (3H, d, J=6.6 Hz), 3.20 (2H, s), 3.35 (2H, d, J=6.6 Hz), 3.50 (2H, s), 3.61 (2H, s), 4.97-5.01 (2H, m), 5.85-6.00 (1H, m), 6.65 (1H, d, J=8.5 Hz), 6.98 (1H, dd, J=2.2,8.5 Hz), 7.07 (1H, d, J=8.5 Hz), 7.40-7.50 (3H, m), 7.72-7.80 (1H, m), 7.97-8.03 (2H, m).

### (2) 2-[2-Allyl-4-[[methylcarbamoylmethyl-(5-methyl-2-phenyloxazol-4-ylmethyl)amino]methyl]phenoxy]-2-methylpropanoic acid trifluoroacetate

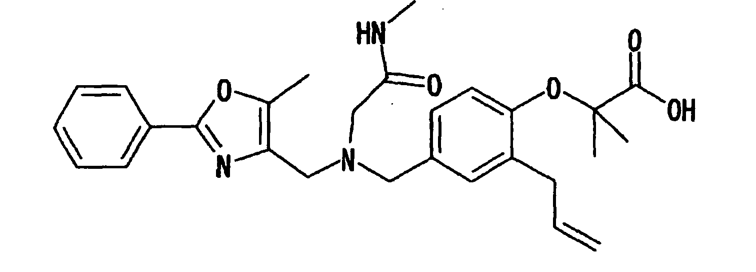

Trifluoroacetic acid (1 ml) was added to dichloromethane (3 ml) solution of the compound (125.0 mg) of Example 10-(1) and the resulting solution was stirred for 4 hours. The solvent was evaporated under reduced pressure to obtain the entitled compound (122.0 mg) as an oily substance.
¹H-NMR (400 MHz, CDCl₃) δ: 1.64 (6H, s), 2.36 (3H, s), 2.64 (3H, s), 3.36 (2H, d, J=6.6 Hz), 3.87 (2H, s), 4.25 (2H, s), 4.36 (2H, s), 5.00-5.10 (2H, m), 5.87-5.98 (1H, m), 6.60-7.05 (3H, m), 7.34 (1H, s), 7.43-7.48 (3H, m), 7.92-7.98 (2H, m), 8.17-8.27 (1H, m).

### [Example 11]

### (1) tert-Butyl 2-[2-allyl-4-[[methylearbamoylmethyl-[5-methyl-2-(3-bromophenyl)oxazol-4-ylmethyl]amino]methyl]phenoxy]-2-methylpropanoate

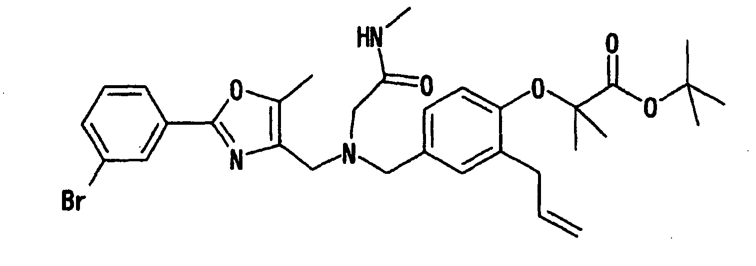

In the same manner as in Example 8-(1), the entitled compound (140.1 mg) was obtained as an oily substance from the compound obtained in Reference Example 11 and N-methylamine hydrochloride (100 mg).
¹H-NMR (400 MHz, CDCl₃) δ: 1.39 (9H, s), 1.56 (6H, s), 2.30 (3H, s), 2.77 (3H, d, J=6.6 Hz), 3.20 (2H, s), 3.35 (2H, d, J=6.6 Hz), 3.50 (2H, s), 3.61 (2H, s), 4.95-5.05 (2H, m), 5.85-6.00 (1H, m), 6.65 (1H, d, J=8.5 Hz), 6.98 (1H, dd, J=2.2,8.5 Hz), 7.07 (1H, d, J=8.5 Hz), 7.30-7.35 (1H, m), 7.50-7.75 (2H, m), 7.92-7.98 (1H, m), 8.17-8.27 (2H, m).

### (2) 2-[2-Allyl-4-[[methylcarbamoylmethyl-[5-methyl-2-(3-bromophenyl)oxazol-4-ylmethyl]amino]methyl]phenoxy]-2-methylpropanoic acid trifluoroacetate

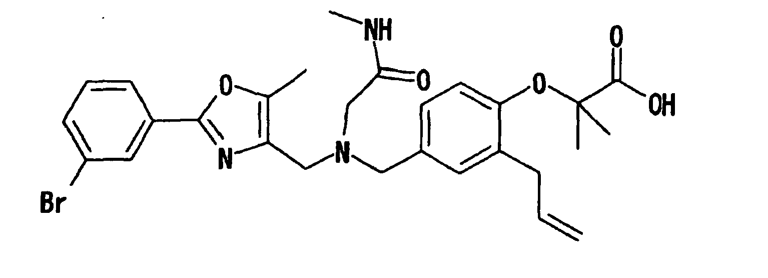

In the same manner as in Example 10-(2), trifluoroacetic acid (1 ml) was added to dichloromethane (3 ml) solution of the compound (141.0 mg) of Example 11, and the resulting solution was stirred for 4 hours. The solvent was evaporated under reduced pressure to obtain the entitled compound (122.0 mg) as an oily substance.
¹H-NMR (400 MHz, CDCl₃) δ: 1.64 (6H, s), 2.37 (3H, s), 2.71 (3H, s), 3.37 (2H, d, J=6.4 Hz), 3.88 (2H, s), 4.29 (2H, s), 4.39 (2H, s), 5.00-5.10 (2H, m), 5.88-5.98 (1H, m), 6.62-7.05 (2H, m), 7.34 (1H, s), 7.43-7.68 (3H, m), 7.92-7.98 (1H, m), 8.17-8.27 (2H, m).

### [Example 12]

### (1) tert-Butyl 2-4-[[[2-(3-bromophenyl)-5-methyloxazol-4-ylmethyl]oxazol-2-ylmethylamino]methyl]phenoxyJ-2-methylpropionate

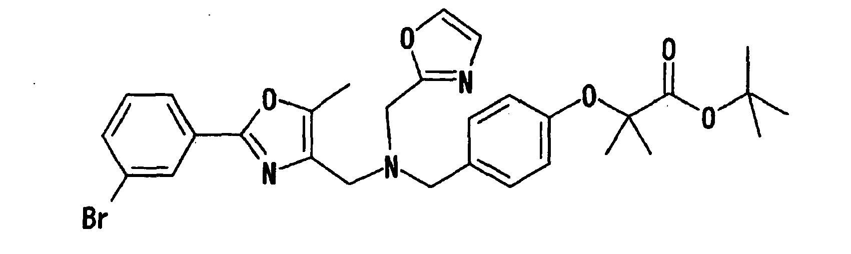

In the same manner as in Example 1-(1), the entitled compound (193 mg) was obtained as a colorless oily substance from the compound obtained (1.3 g) of Reference Example 3 and oxazole-2-carbaldehyde (244 mg).
¹H-NMR (400 MHz, CDCl₃) δ: 1.45 (9H, s), 1.56 (6H, s), 2.30 (3H, s), 3.65 (2H, s), 3.68 (2H, s), 3.88 (2H, s), 6.82 (1H, d, J=8.6 Hz), 6.85 (2H, d, J=8.6 Hz), 7.24 (2H, d, J=8.3 Hz), 7.30 (1H, t, J=7.9 Hz), 7.53 (1H, d, J=6.8 Hz), 7.64 (1H, s), 7.93 (1H, d, J=7.8 Hz), 8.16 (1H, s).
MS m/z: 598(M+H)⁺.

2-4-[[[2-(3-Bromophenyl)-5-methyloxazol-4-ylmethyl]oxazol-2-ylmethylamino]methyl]phenoxy]-2-methylpropionic acid hydrochloride

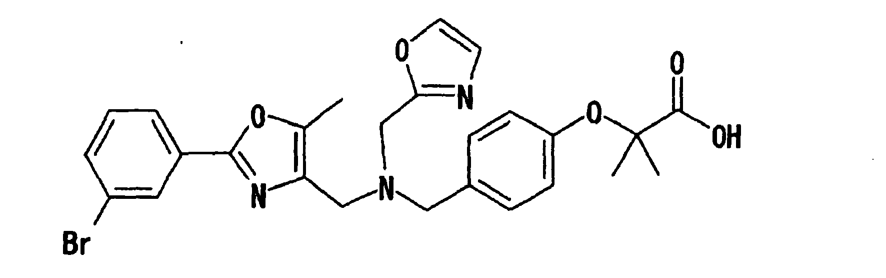

In the same manner as in Example 1-(2), the entitled compound (50 mg) was obtained as a colorless solid from the compound (190 mg) of Example 12-(1).
¹H-NMR (400 MHz, CDCl₃) δ: 1.52 (6H, s), 2.36 (3H, s), 4.07-4.25 (6H, s), 6.84 (2H, d, J=8.8 Hz), 7.32 (1H, bs), 7.44 (2H, d, J=9.0 Hz), 7.94 (1H, d, J=8.1 Hz), 8.07 (1H, bs), 8.20(1H, s).
MS m/z: 542(M+H)⁺.

| Elementary Analysis, as C₂₆H₂₆BrN₃O₅•0.75H₂O•HCl | | | |
|---|---|---|---|
| Calculated: | C,52.90; | H,4.87; | N,7.12. |
| Found: | C,52.91; | H,4.94; | N,6.77. |

### [Example 13]

### (1) Ethyl 2-[2,6-dimethyl-4-[[(5-methyl-2-phenyloxazol-4-ylmethyl)oxazol-2-ylmethylamino]methyl]phenoxy]-2-methylpropanoate

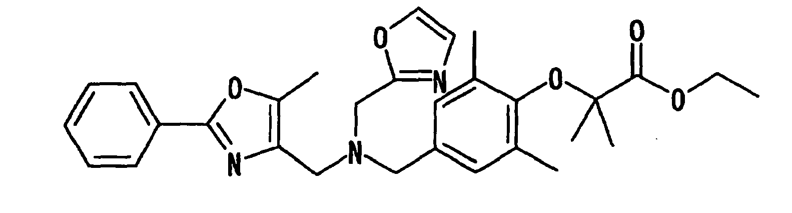

The compound (162 mg) of Reference Example 12 and the compound (150 mg) of the Reference Example 13-(3) were dissolved in dichloromethane (3 ml), and sodium triacetoxyborohydride (180 mg) was added thereto and the resulting mixture was stirred overnight at room temperature. The reaction mixture was extracted with ethyl acetate, washed with an aqueous sodium bicarbonate solution and saturated brine in that order, and dried over anhydrous sodium sulfate. The solvent was evaporated under reduced pressure. The residue was purified by silica gel column chromatography (hexane/ethyl acetate = 1/1) to obtain the entitled compound (307 mg) as a pale yellow oily substance.

¹H-NMR (400 MHz, CDCl₃) δ: 1.35 (3H, t, J=7.1 Hz), 1.45 (6H, s), 2.17 (6H, s), 2.27 (3H, s), 3.63 (2H, s), 3.65 (2H, s), 3.93 (2H, s), 4.28 (2H, q, J=6.9 Hz), 6.98 (2H, s), 7.08 (1H, s), 7.41-7.43 (3H, m), 7.64 (1H, s), 8.00-8.02 (2H, m).
MS m/z: 518(M+H)⁺.

### (2) 2-[2,6-Dimethyl-4-[[(5-methyl-2-phenyloxazol-4-ylmethyl)oxazol-2-ylmethylamino]methyl]phenoxy]-2-methylpropanoic acid

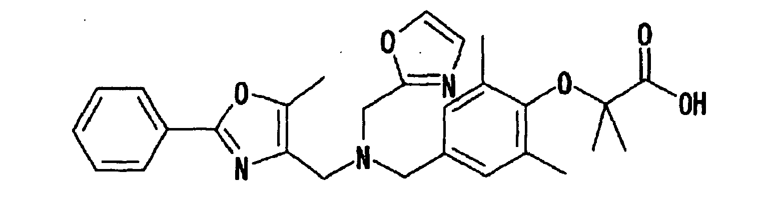

The compound (300 mg) of Example 13-1 was dissolved in methanol (2 ml), and an aqueous 2N sodium hydroxide solution (1 ml) was added thereto and the resulting solution was stirred at room temperature for 3 days. The reaction solution was concentrated under reduced pressure, and the resulting residue was neutralized with an aqueous IN hydrochloric acid solution added thereto. This was diluted with ethyl acetate, washed with saturated brine, and dried over anhydrous sodium sulfate. The solvent was evaporated under reduced pressure. The residue was purified by silica gel column chromatography (chloroform/methanol = 9/1). Thus obtained, the entitled compound was dissolved in dichloromethane, 4 N hydrochloric acid-dioxane solution (2 ml) was added thereto and the resulting solution was concentrated under reduced pressure, and the residue was recrystallized from a mixture of ethyl acetate and hexane to obtain a hydrochloride of the entitled compound (201 mg) as a colorless solid.

¹H-NMR (400 MHz, DMSO-d₆) δ: 1.33 (6H, s), 2.14 (6H, s), 2.32 (3H, s), 4.11-4.35 (6H, m), 7.14 (2H, s), 7.30 (1H, s), 7.51-7.55 (3H, m), 7.93-7.95 (2H, m), 8.19 (1H, s).
MS m/z: 490(M+H)⁺.

| Elementary Analysis, as C₂₈H₃₁N₃O₅•HCl | | | |
|---|---|---|---|
| Calculated: | C,63.93; | H,6.13; | N,7.99. |
| Found: | C,63.63; | H,6.42; | N,7.58. |

### [Example 14]

### (1) Ethyl 2-[2,6-dimethyl-4-[[methylcarbamoylmethyl-(5-methyl-2-phenyloxazol-4-ylmethyl)amino]methyl]phenoxy]-2-methylpropanoate

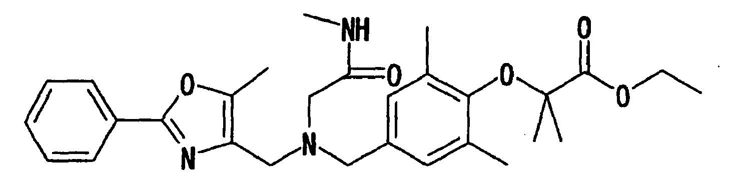

The compound (3.0 g) of Reference 14-(3) was dissolved in N,N-dimethylformamide (10 ml), and methylamine (2 M tetrahydrofuran solution, 4.5 ml), 1-(3-dimethylaminopropyl)-3-ethylcarbodiimide hydrochloride (2.27 g) and 1-hydroxybenzotriazole (1.82 g) were added thereto and the resulting solution was stirred at room temperature for 13 hours. The reaction solution was concentrated under reduced pressure, the residue was dissolved in ethyl acetate, and washed with water, an aqueous 10% citric acid solution, water; an aqueous saturated sodium bicarbonate solution and saturated brine in that order. This was dried over anhydrous sodium sulfate, and the solvent was evaporated under reduced pressure. The residue was purified by silica gel column chromatography (eluted with ethyl acetate) to obtain the entitled compound (2.59 g) as an yellow oily substance.

¹H-NMR (400 MHz, CDCl₃) δ: 1.35 (3H, t, J=7.1 Hz), 1.45 (6H, s), 2.17 (6H, s), 2.25 (3H, s), 2.80 (3H, d, J=4.9 Hz), 3.22 (2H, s), 3.49 (2H, s), 3.60 (2H, s), 4.28 (2H, q, J=7.2 Hz), 6.89 (2H, s), 7.43-7.47 (3H, m), 7.80 (1H, s), 7.99-8.01 (2H, m).
MS m/z: 508(M+H)⁺.

### (2) 2-[2,6-Dimethyl-4-[[methylcarbamoylmethyl-(5-methyl-2-phenyloxazol-4-ylmethyl)amino]methyl]phenoxy]-2-methylpropanoic acid

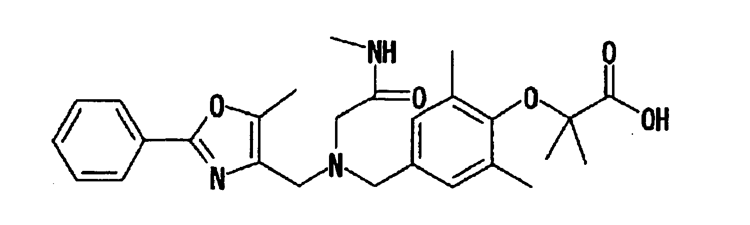

The compound (2.59 g) of Example 14-(1) was dissolved in methanol (2.0 ml), and an aqueous 1N sodium hydroxide solution (5 ml) and an aqueous 5N sodium hydroxide solution (2 ml) were added thereto and the resulting solution was stirred at room temperature for 13 hours. The reaction solution was concentrated under reduced pressure, and the residue was neutralized with an aqueous 1N hydrochloric acid solution added thereto. This was diluted with ethyl acetate, washed with water and saturated brine in that order, and dried over anhydrous sodium sulfate. The solvent was evaporated under reduced pressure. The residue was purified by silica gel column chromatography (chloroform/methanol = 7/1) and then recrystallized from a mixture of ethyl acetate and hexane to obtain the entitled compound (1.85 g) as a colorless solid.

¹H-NMR (400 MHz, DMSO-d₆) δ: 1.31 (6H, s), 2.12 (6H, s), 2.22 (3H, s), 2.60 (3H, d, J=4.6 Hz), 3.09 (2H, s), 3.50 (2H, s), 3.56 (2H, s), 6.99 (2H, s), 7.48-7.53 (3H, m), 7.74-7.78 (1H, m), 7.91-7.93 (2H, m).
MS m/z: 480(M+H)⁺.

| Elementary Analysis, as C₂₇H₃₃N₃O₅ | | | |
|---|---|---|---|
| Calculated: | C,67.62; | H,6.94; | N,8.76. |
| Found: | C,67.57; | H,7.09; | N,8.56. |

### [Example 15]

### (1) Ethyl 2-[4-[[dimethylcarbamoylmethyl-(5-methyl-2-phenyloxazol-4-ylmethyl)amino]methyl]-2,6-dimethylphenoxy]-2-methylpropanoate

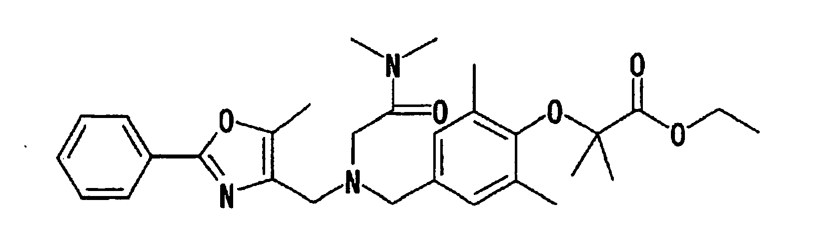

In the same manner as in Example 14-(1), the entitled compound (1.80 g) was obtained as a pale yellow oily substance from the compound (2.37 g) of Reference Example 14-(3) and dimethylamine (40% aqueous solution, 0.76 ml).
¹H-NMR (400 MHz, CDCl₃) δ: 1.35 (3H, t, J=7.1 Hz), 1.45 (6H, s), 2.17 (6H, s), 2.26 (3H, s), 2.89 (6H, s), 3.39 (2H, s), 3.66 (4H, s), 4.29 (2H, q, J=7.1 Hz), 6.97 (2H, s), 7.40-7.46 (3H, m), 7.98-8.01 (2H, m).
MS m/z: 522(M+H)⁺.

### (2) 2-[4-[[Dimethylcarbamoylmethyl-(5-methyl-2-phenyloxazol-4-ylmethyl)amino]methyl]-2,6-dimethylphenoxy]-2-methylpropanoic acid

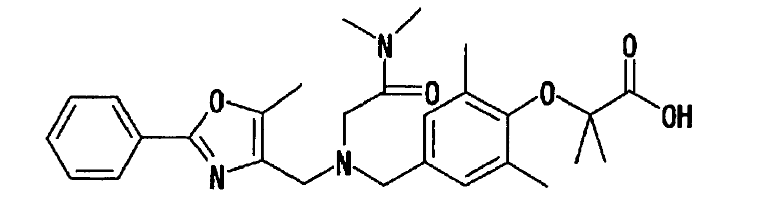

In the same manner as in Example 13-(2), a hydrochloride of the entitled compound (1.3 g) was obtained as a colorless solid by processing the compound (1.8 g) of Example 15-(1) followed by recrystallizing the resulting product from a mixed solvent of dichloromethane-ethyl acetate-hexane.
¹H-NMR (400 MHz, DMSO-d₆) δ: 1.36 (6H, s), 2.18 (6H, s), 2.42 (3H, s), 2.84 (3H, s), 2.89 (3H, s), 4.11-4.40 (6H, m), 7.31 (2H, s), 7.54-7.59 (3H, m), 7.97-7.99 (2H, m).
MS m/z: 494(M+H)⁺.

| Elementary Analysis, as C₂₈H₃₅N₃O₅·H₂O·HCl-0.2CH₂Cl₂ | | | | |
|---|---|---|---|---|
| Calculated: | C,59.94; | H,6.85; | Cl,8.78; | N,7.44. |
| Found: | C,60.15; | H,6.74; | Cl,9.12; | N,7.36. |

### [Example 16]

### (1) Ethyl 2-[2,6-dimethyl-4-[[(5-methyl-2-phenyloxazol-4-ylmethyl)thiazol-2-ylmethylamino]methyl]phenoxy]-2-methylpropanoate

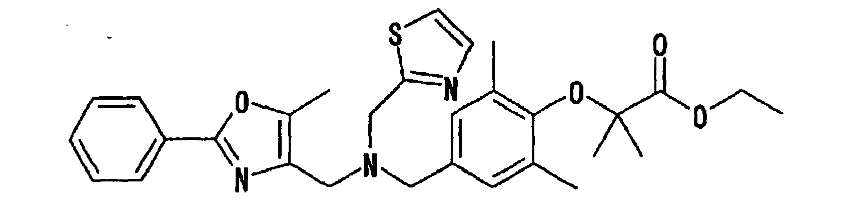

In the same manner as in Example 13-(1), the entitled compound (499 mg) was obtained as a colorless oily substance from the compound (264 mg) of Reference Example 12 and the compound (285 mg) of Reference Example 15.
¹H-NMR (400 MHz, CDCl₃) δ:1.20 (3H, t, J=7.1 Hz), 1.45 (6H, s), 2.22 (6H, s), 2.28 (3H, s), 3.63 (2H, s), 3.65 (2H, s), 4.10 (2H, s), 4.24 (2H, q, J=7.1 Hz), 7.05 (2H, s), 7.28 (1H, d, J=3.4 Hz), 7.35-7.45 (3H, m), 7.70 (1H, d, J=3.4 Hz), 7.96-8.00 (2H, m).

### (2) 2-[2,6-Dimethyl-4-[[(5-methyl-2-phenyloxazol-4-ylmethyl)thiazol-2-ylmethylamino]methyl]phenoxy]-2-methylpropanoic acid

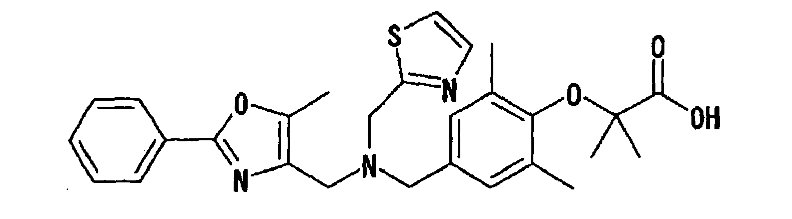

The compound (499 mg) of Example 16-(1) was dissolved in tetrahydrofuran (4 ml), and methanol (2 ml) and an aqueous 1N sodium hydroxide solution (2 ml) were added thereto and the resulting solution was stirred at room temperature for 1 hour. This was neutralized with an aqueous 1N hydrochloric acid solution, extracted with ethyl acetate, and dried over anhydrous sodium sulfate. The solvent was evaporated under reduced pressure to obtain the entitled compound (452 mg) as a pale yellow solid.
¹H-NMR (400 MHz, CDCl₃) δ: 1.50 (6H, s), 2.22 (6H, s), 2.28 (3H, s), 3.63 (2H, s), 3.65-3.68 (3H, br s), 4.10 (2H, s), 7.05 (2H, s), 7.28 (1H, d, J=3.4 Hz), 7.40-7.45 (3H, m), 7.78 (1H, d, J=3.4 Hz), 7.98-8.02 (2H, m).

### [Example 17]

### (1) Ethyl 2-[2,6-dimethyl-4-[[(5-methyl-2-phenyloxazol-4-ylmethyl)-(S-oxo-4,5-dihydro-[1,3,4]oxadiazol-2-ylmethyl)amino]methyl]phenoxy]-2-methylpropanoate

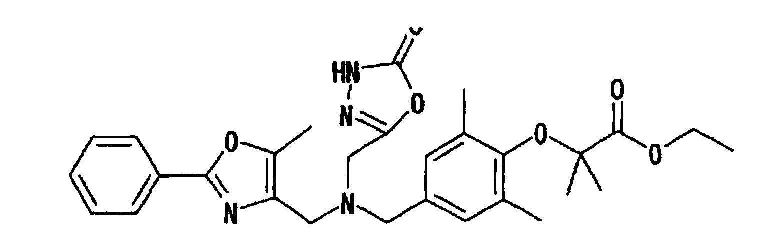

The compound (200 mg) of Reference Example 16-(2) was dissolved in dioxane (5 ml), and triphosgene (234 mg) was added thereto and the resulting solution was stirred overnight at room temperature. Further, triphosgene (234 mg) was added thereto and the solution was stirred at 60°C for 4 hours, and then the reaction solution was concentrated under reduced pressure. The residue was dissolved in ethyl acetate, and washed with an aqueous saturated sodium bicarbonate solution and saturated brine. This was dried over anhydrous sodium sulfate, and the solvent was evaporated under reduced pressure. The residue was purified by silica gel column chromatography (ethyl acetate/hexane = 1/2) to obtain the entitled compound (176 mg) as a colorless oily substance.

¹H-NMR (400 MHz, CDCl₃) δ: 1.35 (3H, t, J=7.1 Hz), 1.57 (6H, s), 2.18 (6H, s), 2.28 (3H, s), 3.65 (2H, s), 3.67 (2H, s), 3.73 (2H, s), 4.28 (2H, q, J=7.1 Hz), 6.97 (2H, s), 7.40-7.46 (3H, m), 7.99-8.01 (2H, m), 8.54(1H, s).
MS m/z: 535(M+H)⁺.

(2) 2-[2,6-Dimethyl-4-[[(5-methyl-2-phenyloxazol-4-ylinethyl)-(5-oxo-4,5-dihydro-[1,3,4]oxadiazol-2-yhnethyl)amino]methyl]phenoxy]-2-methylpropanoic acid

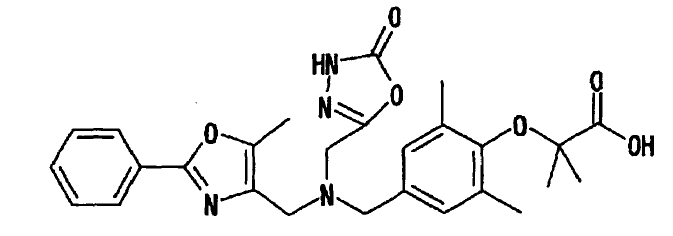

In the same manner as in Example 14-(2), the entitled compound (100 mg) was obtained as a colorless solid from the compound (170 mg) of Example 17-(1).
¹H-NMR(400 MHz, DMSO-d₆) δ: 1.32 (6H, s), 2.12 (6H, s), 2.26 (3H, s), 3.57 (2H, s), 3.59 (2H, s), 3.62 (2H, s), 6.95 (2H, s), 7.46-7.53 (3H, m), 7.90-7.92 (2H, m).
MS m/z: 505(M-H)⁻.

### [Example 18]

### (1) Ethyl 2-[2,6-dimethyl-4-[[(5-methyl-[1,3,4]oxadiazol-2-ylmethyl)-(5-methyl-2-phenyloxazol-4-ylmethyl)amino]methyl]phenoxy]-2-methylpropanoate

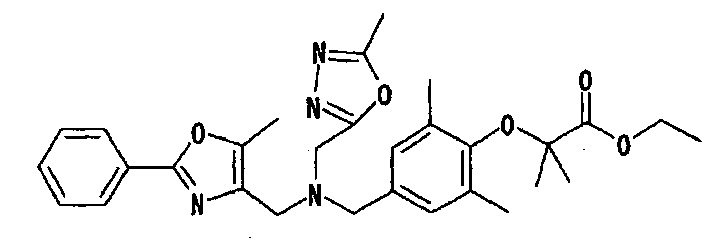

Triphenylphosphine (675 mg) was dissolved in dichloromethane (15 ml), and with stirring with cooling with ice in water, hexachloroethane (510 mg) and triethylamine (716 mg) were added thereto. Then, dichloromethane (5 ml) solution of the compound (0.5 g) of Reference Example 17 was added to it, and the resulting solution was stirred at room temperature for 13 hours. An aqueous saturated sodium bicarbonate solution was added to it, and the solution was stirred for 1 hour and then extracted three times with dichloromethane. This was washed with saturated brine, then dried over anhydrous sodium sulfate, and concentrated under reduced pressure. The residue was purified by silica gel column chromatography (ethyl acetate/hexane = 2/1) to obtain the entitled compound (577 mg) as a pale yellow oily substance.

¹H-NMR (400 MHz, CDCl₃) δ: 1.35 (3H, t, J=7.2 Hz), 1.45 (6H, s), 2.18 (6H, s), 2.27 (3H, s), 2.51 (3H, s), 3.67 (4H, br s), 3.99 (2H, s), 4.28 (2H, q, J=7.1 Hz), 6.98 (2H, s), 7.40-7.45 (3H, m), 8.00-8.02 (2H, m).
MS m/z: 533(M+H)⁺.

### (2) 2-[2,6-Dimethyl-4-[[(5-methyl-[1,3,4]oxadiazol-2-ylmethyl)-(5-methyl-2-phenyloxazol-4-ylmethyl)amino]methyl]phenoxy]-2-methylpropanoic acid

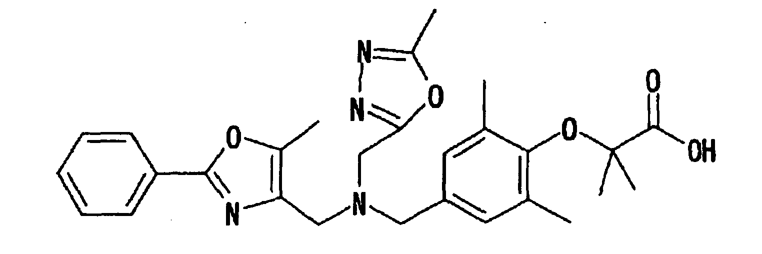

In the same manner as in Example 13-(2), a hydrochloride of the entitled compound (315 mg) was obtained as a colorless solid from the compound (0.57 g) of Example 18-(1).
¹H-NMR (400 MHz, DMSO-d₆) δ: 1.33 (6H, s), 2.13 (6H, s), 2.29 (3H, s), 2.46 (3H, s), 3.66-4.26 (6H, m), 7.05 (2H, s), 7.50-7.52 (3H, m), 7.91-7.93 (2H, m).
MS m/z: 505(M+H)⁺.

### [Example 19]

### (1) Ethyl 2-[4-[[carbamoylmethyl-(5-methyl-2-phenyloxazol-4-ylmethyl)amino]methyl]-2,6-dimethylphenoxy]-2-methylpropanoate

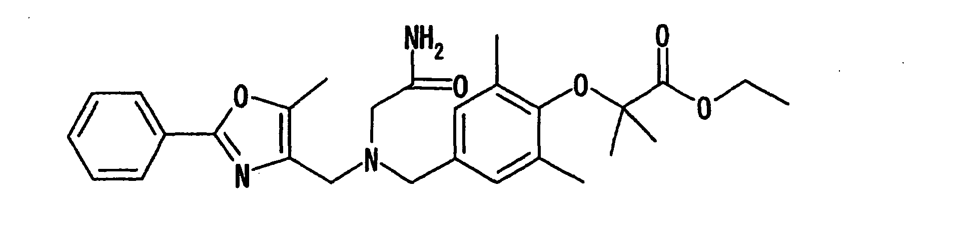

In the same manner as in Reference Example 16-(1), the entitled compound (528 mg) containing some impurities was obtained as a pale yellow oil from the compound (400 mg) of Reference Example 14-(3) and ammonium chloride (55 mg).
¹H-NMR (400 MHz, CDCl₃) δ: 1.35 (3H, t, J=7.1 Hz), 1.45 (6H, s), 2.17 (6H, s), 2.25 (3H, s), 3.24 (2H, s), 3.54 (2H, s), 3.60 (2H, s), 4.28 (2H, q, J=7.1 Hz), 6.91 (2H, s), 7.44-7.45 (3H, m), 7.98-8.02 (2H, m).
MS m/z: 494(M+H)⁺.

### (2) Ethyl 2-[2,6-dimethyl-4-[[(3-methyl-[1,2,4]oxadiazol-5-ylmethyl)-(5-methyl-2-phenyloxazol-4-ylmethyl)amino]methyl]-phenoxy]-2-methylpropanoate

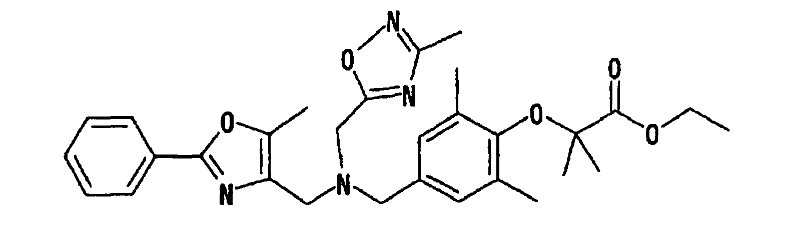

N,N-dimethylacetamide dimethyl acetal (5 ml) was added to the compound (528 mg) of Example 19-(1), and the resulting mixture was heated at 120°C for 2 hours. The solvent was evaporated under reduced pressure, and an aqueous 50% hydroxylamine solution (70 ml) and an aqueous 70% acetic acid solution (6 ml) were added to the residue. The solution was stirred overnight at room temperature. The solvent was evaporated under reduced pressure, and the residue was dissolved in ethyl acetate. This was washed with an aqueous saturated sodium bicarbonate solution and then with saturated brine, and dried over anhydrous sodium sulfate. The solvent was evaporated, and the residue was purified by silica gel column chromatography (ethyl acetate/hexane = 1/3) to obtain the entitled compound (379 mg) as a colorless oily substance.

¹H-NMR (400 MHz, CDCl₃) δ: 1.35 (3H, t, J=7.1 Hz), 1.45 (6H, s), 2.18 (6H, s), 2.27 (3H, s), 2.41 (3H, s), 3.67 (2H, s), 3.70 (2H, s), 4.04 (2H, s), 4.28 (2H, q, J=7.1 Hz), 7.00 (2H, s), 7.40-7.46 (3H, m), 7.99-8.01 (2H, m).
MS m/z: 533(M+H)⁺.

### (3) 2-[2,6-Dimethyl-4-[[(3-methyl-[1,2,4]oxadiazol-5-ylmethyl)-(5-methyl-2-phenyloxazol-4-ylmethyl)amino]methyl]-phenoxy]-2-methylpropanoic acid

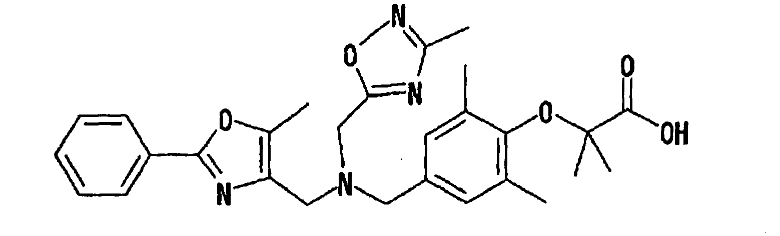

In the same manner as in Example 13-(2), a hydrochloride of the entitled compound (273 mg) was obtained from the compound (375 mg) of Example 19-(2).
¹H-NMR (400 MHz, DMSO-d₆) δ: 1.32 (6H, s), 2.13 (6H, s), 2.27 (3H, s), 2.31 (3H, s), 3.81 (4H, br s), 4.15 (2H, br s), 7.01 (2H, s), 7.49-7.53 (3H, m), 7.90-7.92 (2H, m).
MS m/z: 505(M+H)⁺.

| Elementary Analysis, as C₂₈H₃₃ClN₄O₅•1.4H₂O(HCl | | | |
|---|---|---|---|
| Calculated: | C,61.65; | H,6.19; | N,10.27. |
| Found: | C,62.02; | H,6.53; | N,9.87. |

### [Example 20]

### (1) Ethyl 2-[4-[[(5-methyl-2-phenyloxazol-4-ylmethyl)-oxazol-2-ylmethylamino]methyl]phenoxy]hexanoate

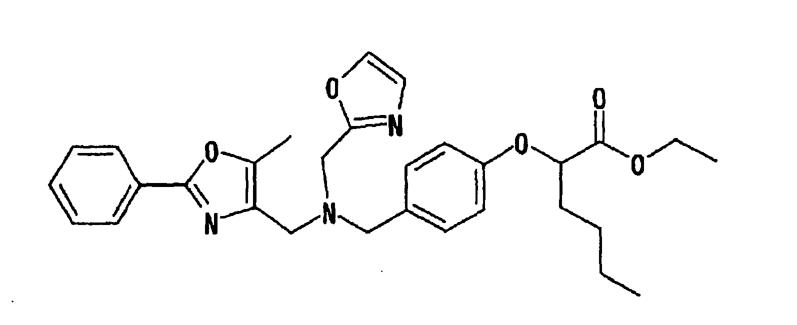

In the same manner as in Example 13-(1), the entitled compound (96 mg) was obtained as a colorless oily substance from the compound (50 mg) of Reference Example 13-(3) and the compound (54 mg) of Reference Example 18.
¹H-NMR (400 MHz, CDCl₃) δ: 0.92 (3H, t, J=7.2 Hz), 1.24 (3H, t, J=7.1 Hz), 1.35-1.42 (4H, m), 1.90-1.97 (2H, m), 2.31 (3H, s), 3.66 (2H, s), 3.69 (2H, s), 3.87 (2H, s), 4.20 (2H, q, J=7.1 Hz), 4.54-4.58 (1H, m), 6.83 (2H, d, J=8.5 Hz), 7.08 (1H, d, J=0.7 Hz), 7.31 (2H, d, J=8.8 Hz), 7.41-7.43 (3H, m), 7.63 (1H, d, J=0.7 Hz), 7.99-8.01 (2H, m).
MS m/z: 518(M+H)⁺.

### (2) 2-[4-[[(5-Methyl-2-phenyloxazol-4-ylmethyl)-oxazol-2-ylmethylamino]methyl]phenoxy]hexanoic acid

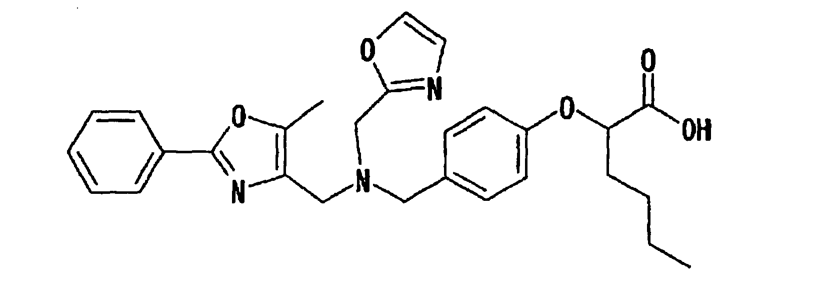

In the same manner as in Example 13-(2), a hydrochloride of the entitled compound (51 mg) was obtained as a colorless solid from the compound (95 mg) of Example 20-(1).
¹H-NMR (400 MHz, CDCl₃) δ: 0.87 (3H, t, J=7.1 Hz), 1.14-1.45 (4H, m), 1.74-1.93 (3H, m), 2.34 (3H, s), 4.03-4.23 (6H, m), 4.67-4.70 (1H, m), 6.90 (2H, d, J=8.3 Hz), 7.30 (1H, s), 7.43 (2H, brs), 7.51-7.54 (3H, m), 7.93-7.95 (2H, m), 8.18 (1H, s).
MS m/z: 488(M-H)⁻.

### [Example 21]

### (1) Ethyl 2-[2,6-dimethyl-4-[[[2-(5-methyl-2-phenyloxazol-4-yl)ethyl]thiazol-2-ylmethylamino]methyl]phenoxy]-2-methylpropionate

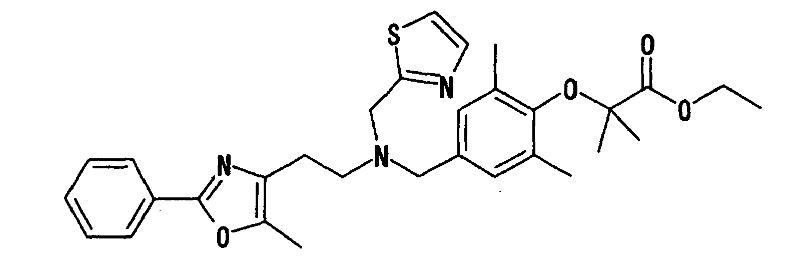

The compound (200 mg) of Reference Example 19 and 2-formylthiazole (0.06 ml) were dissolved in dichloromethane (5 ml), and sodium triacetoxyborohydride (188 mg) was added thereto and the resulting solution was stirred at room temperature for 23 hours. This was diluted with ethyl acetate, washed with water and saturated brine, and dried over anhydrous sodium sulfate. The solvent was evaporated under reduced pressure, and the residue was purified by silica gel column chromatography (ethyl acetate/hexane = 1/1) to obtain the entitled compound (223 mg) as a pale yellow oily substance.

¹H-NMR (400 MHz, CDCl₃) δ: 1.34 (3H, t, J=7.1 Hz), 1.43 (6H, s), 2.15 (6H, s), 2.24 (3H, s), 2.70 (2H, t, J=7.4 Hz), 2.86 (2H, t, J=7.2 Hz), 3.64 (2H, s), 3.98 (2H, s), 4.27 (2H, q, J=7.1 Hz), 6.97 (2H, s), 7.22 (1H, d, J=3.4 Hz), 7.38-7.44 (3H, m), 7.66 (1H, d, J=3.2 Hz), 7.93-7.95 (2H, m).
MS m/z: 548(M+H)⁺.

### (2) 2-[2,6-Dimethyl-4-[[[2-(5-methyl-2-phenyloxazol-4-yl)ethyl]thiazol-2-ylmethylamino]methyl]phenoxy]-2-methylpropionic acid

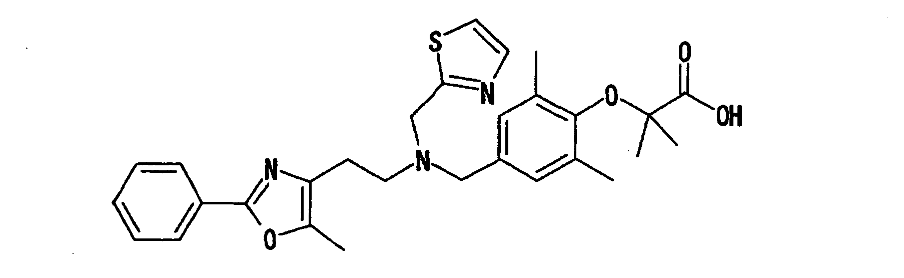

In the same manner as in Example 13-(2), a hydrochloride of the entitled compound (167 mg) was obtained as a colorless solid from the compound (220 mg) of Example 21-(1).
¹H-NMR (400 MHz, DMSO-d₆) δ: 1.35 (6H, s), 2.15 (6H, s), 2.34 (3H, s), 3.06 (2H, t, J=7.6 Hz), 3.34 (2H, t, J=7.8 Hz), 4.41 (2H, s), 4.82 (2H, s), 7.28 (2H, s), 7.50-7.54 (3H, m), 7.88-7.90 (2H, m), 7.94 (1H, d, J=3.4 Hz), 8.02 (1H, d, J=3.2 Hz).
MS m/z: 520(M+H)⁺.

| Elementary Analysis, as C₂₉H₃₄ClN₃O₄S•2HCl | | | |
|---|---|---|---|
| Calculated: | C,58.78; | H,5.95; | N,7.09. |
| Found: | C,58.75; | H,5.99; | N,7.01. |

### [Example 22]

### (1) Ethyl 2-[4-[[benzoxazol-2-ylmethyl-(methyl-2-phenyloxazol-4-ylmethyl)amino]methyl]-2,6-dimethylphenoxy]-2-methylpropionate

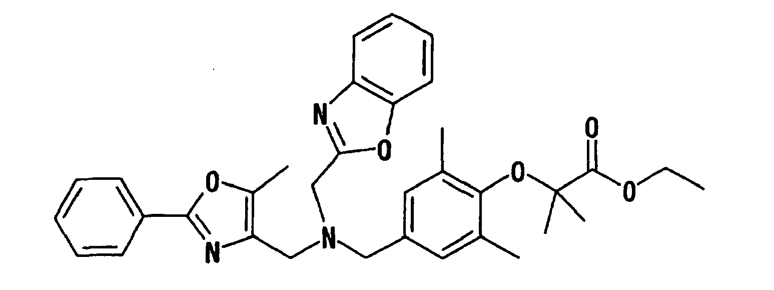

The compound (200 mg) of Reference Example 20 and 2-chloromethylbenzoxazole (77 mg) were dissolved in N,N-dimethylformamide (5 ml), and cesium carbonate (300 mg) was added thereto and the resulting mixture was stirred at 70°C for 20 hours. This was diluted with ethyl acetate, washed with water and saturated brine, and dried over anhydrous sodium sulfate. The solvent was evaporated under reduced pressure, and the residue was purified by silica gel column chromatography (ethyl acetate/hexane = 1/1) to obtain the entitled compound (100 mg) as a pale yellow oily substance.

¹H-NMR (400 MHz, CDCl₃) δ: 1.35 (3H, t, J=7.2 Hz), 1.43 (6H, s),2.17(6H, s),2.26(3H, s), 3.74 (2H, s), 3.76 (2H, s), 4.11 (2H, s), 4.28 (2H, q, J=7.2 Hz), 7.02 (2H, s), 7.31-7.33 (2H, m), 7.40-7.43 (3H, m), 7.52-7.54 (1H, m), 7.70-7.73 (1H, m), 7.98-8.00 (2H, m).
MS m/z: 568(M+H)⁺.

### (2) 2-[4-[[Benzoxazol-2-ylmethyl-(methyl-2-phenyloxazol-4-ylmethyl)amino]methyl]-2,6-dimethylphenoxy]-2-methylpropionic acid

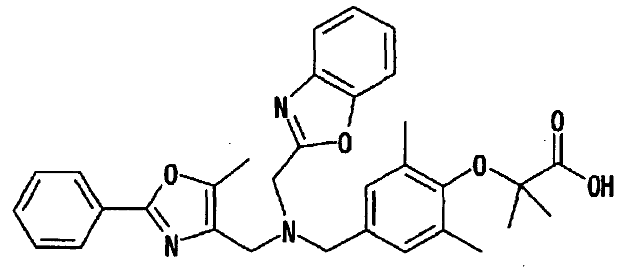

In the same manner as in Example 13-(2), a hydrochloride of the entitled compound (49 mg) was obtained as a colorless solid from the compound (100 mg) of Example 22-(1).
¹H-NMR (400 MHz, DMSO-d₆) δ: 1.31 (6H, s), 2.13 (6H, s), 2.33 (3H, s), 4.02-4.46 (6H, m), 7.15 (2H, s), 7.38-7.45 (2H, m), 7.51-7.53 (3H, m), 7.73-7.79 (2H, m), 7.87-8.00 (2H, m).
MS m/z: 520(M+H)⁺.

| Elementary Analysis, as C₃₂H₃₄ClN₃O₅•3/4H₂O·HCl | | | |
|---|---|---|---|
| Calculated: | C,65.19; | H,6.07; | N,7.13. |
| Found: | C,65.31; | H,6.11; | N,7.00. |

### [Example 23]

### (1) Ethyl 2-[4-[[bis-(5-methyl-2-phenyloxazol-4-ylmethyl)amino]methyl]-2,6-dimethylphenoxy]-2-methylpropionate

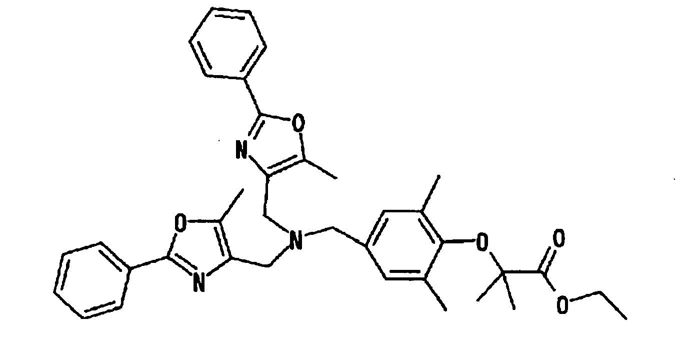

The compound (50 mg) of Reference Example 21-(2) and 5-methyl-2-phenyloxazol-4-carbaldehyde (75 mg) were dissolved in dichloromethane (2 ml), and sodium triacetoxyborohydride (120 mg) was added thereto and the resulting solution was stirred at room temperature for 17 hours. This was diluted with ethyl acetate, then washed with water and saturated brine, and dried over anhydrous sodium sulfate. The solvent was evaporated under reduced pressure, and the residue was purified by silica gel column chromatography (ethyl acetate/hexane = 1/3) to obtain the entitled compound (105 mg) as a pale yellow oily substance.

¹H-NMR (400 MHz, CDCl₃) δ: 1.35 (3H, t, J=7.2 Hz), 1.44 (6H, s), 2.17 (6H, s), 2.29 (6H, s), 3.61 (2H, s), 3.68 (4H, s), 4.28 (2H, q, J=7.1 Hz), 7.00 (2H, s), 7.40-7.45 (6H, m), 8.00-8.02 (4H, m).
MS m/z: 608(M+H)⁺.

### (2) 2-[4-[[Bis(5-methyl-2-phenyloxazol-4-ylmethyl)amino]methyl]-2,6-dimethylphenoxy]-2-methylpropionic acid

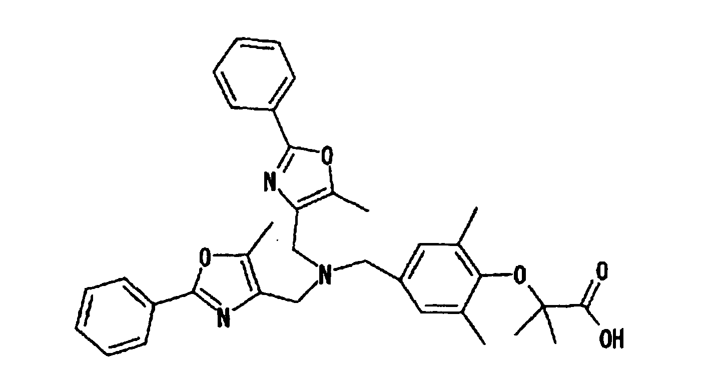

In the same manner as in Example 13-(2), a hydrochloride of the entitled compound (57 mg) was obtained as a colorless solid from the compound (100 mg) of Example 23-(1).
¹H-NMR (400 MHz, DMSO-d₆) δ: 1.34 (6H, s), 2.15 (6H, s), 2.38 (6H, s), 4.32-4.51 (6H, m), 7.29 (2H, s), 7.53-7.57 (6H, m), 7.96-7.98 (4H, m).

| Elementary Analysis, as C₃₅H₃₈ClN₃O₅•5/4H₂O(HCl | | | |
|---|---|---|---|
| Calculated: | C,65.82; | H,6.39; | N,6.58. |
| Found: | C,65.94; | H,6.44; | N,6.32. |

### [Example 24]

### (1) Ethyl 2-[2,6-dimethyl-4-[[(4-methyloxazol-2-ylmethyl)(5-methyl-2-phenyloxazol-4-ylmethyl)amino]methyl]phenoxy]-2-methylpropanoate

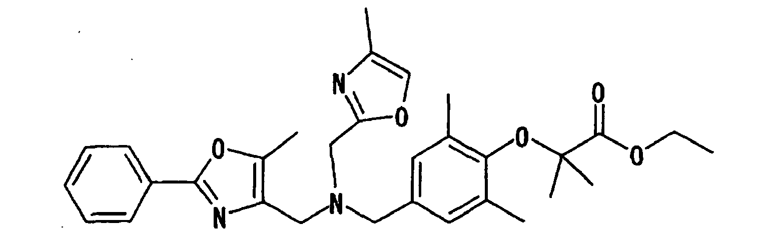

Triphenylphosphine (0.341 g), hexachloroethane (0.260 g) and triethylamine (0.361 ml) were added to dichloromethane (10 ml), and after stirred for 15 minutes with cooling with ice in water, the compound (0.242 g) obtained in Reference Example 22-(2) was added to it, then the solution was warmed up to room temperature and stirred overnight. Water was added to the reaction solution, extracted with ethyl acetate, and washed with water and then with saturated brine. This was dried over anhydrous sodium sulfate, and the solvent was evaporated under reduced pressure. The resulting residue was purified by silica gel column chromatography (ethyl acetate/hexane = 1/1) to obtain the entitled compound (0.085 g) as a pale yellow oily substance.
¹H-NMR (400 MHz, CDCl₃) δ: 1.35 (3H, t, J=7.2 Hz), 1.45 (6H, s), 2.16 (3H, s), 2.17 (6H, s), 2.26 (3H, s), 3.62 (2H, s), 3.63 (2H, s), 3.88 (2H, s), 4.28 (2H, q, J=7.2 Hz), 6.99 (2H, s), 7.34 (1H, s), 7.40-7.44 (3H, m), 8.02-7.99 (2H, m).
MS m/z: 532(M+H)⁺.

### (2) 2-[2,6-Dimethyl-4-[[(4-methyloxazol-2-ylmethyl)(5-methyl-2-phenyloxazol-4-ylmethyl)amino]methyl]phenoxy]-2-methylpropanoic acid

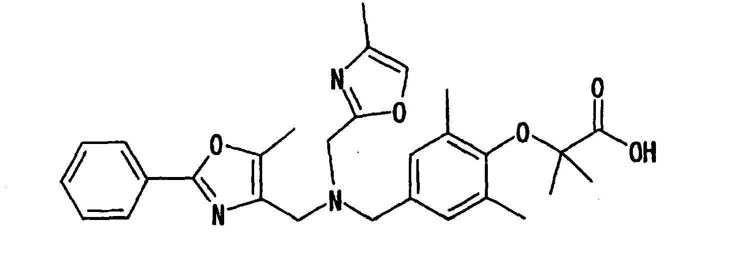

The compound (0.080 g) obtained in Example 24-(1) was dissolved in methanol (5 ml), and an aqueous 1N sodium hydroxide solution (2 ml) was added thereto and the resulting solution was heated under reflux for 3 hours. The reaction solution was cooled, then neutralized with an aqueous 1N hydrochloric acid solution added thereto, and concentrated under reduced pressure. The residue was extracted with ethyl acetate, and washed with water and then with saturated brine. This was dried over anhydrous sodium sulfate, and the solvent was evaporated under reduced pressure. The resulting residue was purified by silica gel column chromatography (methanol/chloroform = 1/9) to obtain the entitled compound. This was dissolved in 4 N hydrochloric acid-dioxane (1 ml), and dried up under reduced pressure to obtain a hydrochloride of the entitled compound (0.060 g) as a colorless solid.
¹H-NMR (400 MHz, DMSO-d₆) δ: 1.35 (6H, s), 2.09 (3H, s), 2.16 (6H, s), 2.33 (3H, s), 3.54-3.60 (4H, m), 4.07-4.32 (3H, m), 7.14 (2H, s), 7.52-7.55 (3H, m), 7.86 (1H, s), 7.96-7.92 (2H, m).
MS m/z: 504(M+H)⁺.

| Elementary Analysis, as C₂₉H₃₃N₃O₅•HCl•2H₂O | | | |
|---|---|---|---|
| Calculated: | C,60.46; | H,6.65; | N,7.29. |
| Found: | C,60.94; | H,6.82; | N,6.76. |

### [Example 25]

### (1) Ethyl 2-[2,6-dimethyl-4-[[oxazol-2-ylmethyl-(5-phenyl[1,3,4]oxadiazol-2-ylmethyl)amino]methyl]phenoxy]-2-methylpropanoate

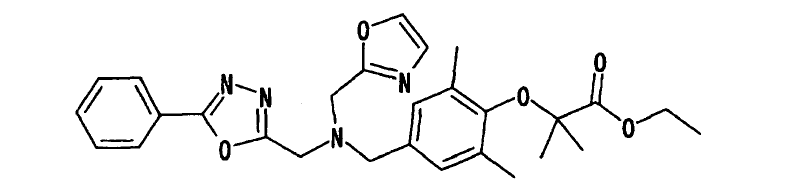

In the same manner as in Example 24-(1), the entitled compound (0.090 g) was obtained as an yellow oily substance by processing the compound (0.115 g) obtained in Reference Example 24-(2).
¹H-NMR (400 MHz, CDCl₃) δ: 1.35 (3H, t, J=6.6 Hz), 1.45 (6H, s), 2.18 (6H, s), 3.75 (2H, s), 4.02 (2H, s), 4.11 (2H, d, J=4.4 Hz), 4.28 (2H, q, J=7.2 Hz), 7.00 (2H, s), 7.08 (1H, d, J=1.0 Hz), 7.49-7.55 (3H, m), 7.64 (1H, d, J=1.0 Hz), 8.07-8.05 (2H, m).
MS m/z: 505(M+H)⁺.

### (2) 2-[2,6-Dimethyl-4-[[oxawl-2-ylmethyl-(5-phenyl[1,3,4]oxadiazol-2-ylmethyl)amino]methyl]phenoxy]-2-methylpropanoic acid

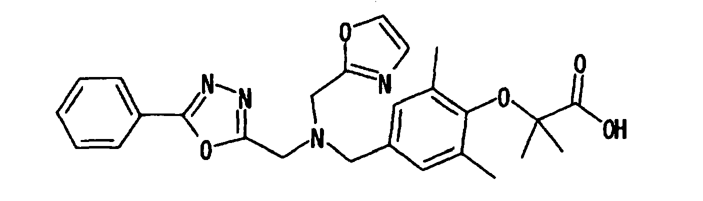

In the same manner as in Example 24-(2), a hydrochloride of the entitled compound (0.050 g) was obtained as a colorless solid from the compound (0.090 g) obtained in Example 25-(1).
¹H-NMR (400 MHz, DMSO-d₆) δ: 1.30 (6H, s), 2.11 (6H, s), 3.71 (2H, s), 3.98 (2H, s), 4.08 (2H, s), 6.96 (2H, s), 7.17 (1H, d, J=0.7 Hz), 7.65-7.59 (3H, m), 7.96-7.98 (2H, m), 8.08 (1H, d, J=1.0 Hz).
MS m/z: 477(M+H)⁺.

| Elementary Analysis, as C₂₆H₂₈N₄O₅·1/4HCl·3/4H₂O | | | |
|---|---|---|---|
| Calculated: | C,62.56; | H,6.01; | N,11.22. |
| Found: | C,62.98; | H,5.87; | N,10.79. |

### [Example 26]

### Ethyl 2-[2,6-dimethyl-4-[[oxadiazol-2-ylmethyl-(5-phenyloxazol-2-ylmethyl)amino]methyl]phenoxy]-2-methylpropanoate

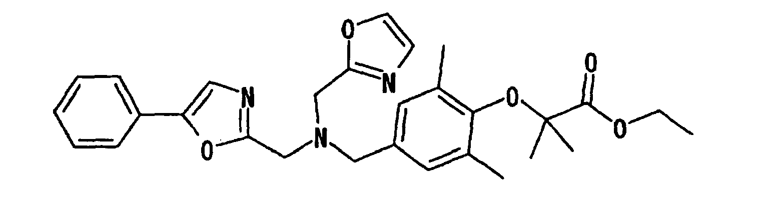

In the same manner as in Example 24-(1), the entitled compound (0.06 g) was obtained as an yellow oily substance by processing the compound (0.12 g) obtained in Reference Example 25.
¹H-NMR (400 MHz, CDCl₃) δ: 1.35 (3H, t, J=7.1 Hz), 1.45 (6H, s), 2.18 (6H, s), 3.72 (2H, s), 3.98 (2H, s), 4.00 (2H, s), 4.28 (2H, q, J=7.1 Hz), 7.02 (2H, s), 7.09 (1H, s), 7.28 (1H, s), 7.34 (1H, d, J=7.3 Hz), 7.44-7.40 (2H, m), 7.63-7.65 (3H, m).
MS m/z: 504(M+H)⁺.

### (2) 2-[2,6-Dimethyl-4-[[oxadiazol-2-ylmethyl-(5-phenyloxazol-2-ylmethyl)amino]methyl]phenoxy]-2-methylpropanoic acid

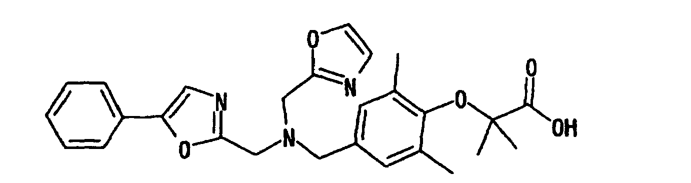

In the same manner as in Example 24-(2), a hydrochloride of the entitled compound (0.04 g) was obtained as a pale yellow solid from the compound (0.06 g) obtained in Example 26-(1).
¹H-NMR (400 MHz, DMSO-d₆) δ: 1.32 (6H, s), 2.12 (6H, s), 3.67 (2H, s), 3.92 (2H, s), 3.95 (2H, s), 6.96 (2H, s), 7.18 (1H, s), 7.35-7.39 (1H, m), 7.48 (2H, t, J=7.6 Hz), 7.60 (1H, s), 7.70-7.68 (2H, m), 8.08 (1H, d, J=0.7 Hz).
MS m/z: 476(M+H)⁺.

| Elementary Analysis, as C₂₇H₂₉N₃O₅•3/4HCl | | | |
|---|---|---|---|
| Calculated: | C,64.49; | H,5.96; | N,8.36. |
| Found: | C,64.33; | H,6.13; | N,7.94. |

### [Example 27]

### (1) Ethyl 2-[2,6-dimethyl-4-[[oxadiazol-2-ylmethyl-(5-thiophen-2-yl-[1,3,4]oxadiazol-2-ylmethyl)amino]methyl]phenoxy]-2-methylpropanoate

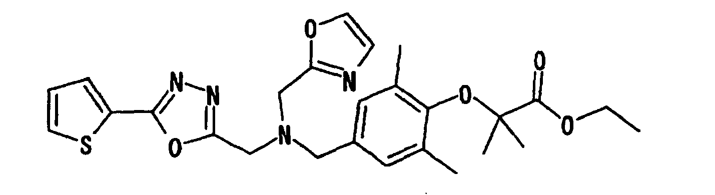

In the same manner as in Example 24-(1), the entitled compound (0.09 g) was obtained as a pale yellow oily substance by processing the compound (0.12 g) obtained in Reference Example 26.
¹H-NMR (400 MHz, CDCl₃) δ: 1.35 (3H, t, J=7.1 Hz), 1.45 (6H, s), 2.18 (6H, s), 3.74 (2H, s), 4.00 (2H, s), 4.08 (2H, s), 4.28 (2H, q, J=7.2 Hz), 7.00 (2H, s), 7.09 (1H, s), 7.18 (1H, dd, J=4.9,3.9 Hz), 7.56 (1H, dd, J=5.1,1.2 Hz), 7.64 (1H, s), 7.76 (1H, dd, J=3.8,1.1 Hz).
MS m/z: 511 (M+H)⁺. (2) 2-[2,6-Ditnethyl-4-[[oxadiazol-2-ylmethyl-(5-thiophen-2-yl-[1,3,4]oxadiazol-2-ylmethyl)amino]methyl]phenoxy]-2-methylpropanoic acid

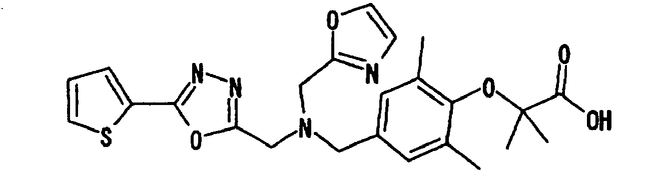

In the same manner as in Example 24-(2), a hydrochloride of the entitled compound (0.06 g) was obtained as a colorless solid from the compound (0.09 g) obtained in Example 27-(1).
¹H-NMR (400 MHz, DMSO-d₆) δ: 1.31 (6H, s), 2.12 (6H, s), 3.69 (2H, s), 3.96 (2H, s), 4.05 (2H, s), 6.95 (2H, s), 7.17 (1H, d, J=0.7 Hz), 7.30 (1H, dd, J=4.9,3.7 Hz), 7.78 (1H, dd, J=3.7,1.2 Hz), 7.95 (1H, dd, J=5.1,1.2 Hz), 8.07 (1H, d, J=0.7 Hz).
MS m/z: 483(M+H)⁺.

| Elementary Analysis, as C₂₄H₂₆N₄O₅S•1/2HCl•1/5C₄H₈O₂ | | | | |
|---|---|---|---|---|
| Calculated: | C,57.46; | H,5.46; | N,10.81; | S,6.19. |
| Found: | C,57.45; | H,5.26; | N,10.44; | S,6.11. |

### [Example 28]

### (1) Ethyl 2-[2,6-dimethyl-4-[[oxazol-2-ylmethyl-(3-thiophen-2-yl-[1,2,4]oxadiazol-5-ylmethyl)amino]methyl]phenoxy]-2-methylpropanoate

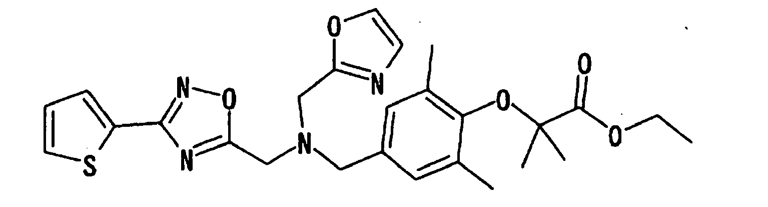

The compound obtained in Reference Example 27-(2) was dissolved in tetrahydrofuran (5 ml), and tetrabutylammonium fluoride (1 M tetrahydrofuran solution, 0.034 ml) was added thereto and the resulting solution was stirred at room temperature for 3 days. The solvent was evaporated under reduced pressure, and the resulting residue was dissolved in ethyl acetate. The solution was washed with water and saturated brine, and dried over anhydrous sodium sulfate. This was concentrated to dryness under reduced pressure, and the residue was purified by silica gel column chromatography (ethyl acetate/hexane = 3/1) to obtain the entitled compound (0.14 g) as a pale yellow oily substance.

¹H-NMR (400 MHz, CDCl₃) δ: 1.35 (3H, t, J=7.1 Hz), 1.45 (6H, s), 2.19 (6H, s), 3.76 (2H, s), 4.04 (2H, s), 4.11 (2H, s), 4.28 (2H, q, J=7.2 Hz), 7.00 (2H, s), 7.09 (1H, d, J=0.7 Hz), 7.17-7.15 (1H, m), 7.51 (1H, dd, J=5.0,1.1 Hz), 7.64 (1H, d, J=0.7 Hz), 7.82 (1H, dd, J=3.8,1.1 Hz).
MS m/z: 511(M+H)⁺. (2) 2-[2,6-Dimethyl-4-[[oxazol-2-ylmethyl-(3-thiophen-2-yl-[1,2,4]oxadiazol-5-ylmethyl)amino]methyl]phenoxy]-2-methylpropanoic acid

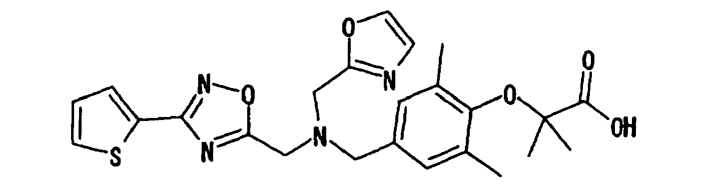

In the same manner as in Example 24-(2), the entitled compound (0.091 g) was obtained as a pale yellow solid from the compound (0.14 g) obtained in Example 28-(1).
¹H-NMR (400 MHz, DMSO-d₆) δ: 1.32 (6H, s), 2.13 (6H, s), 3.72 (2H, s), 3.97 (2H, s), 4.14 (2H, s), 6.96 (2H, s), 7.17 (1H, s), 7.27 (1H, dd, J=4.9,3.7 Hz), 7.79 (1H, dd, J=3.7,1.2 Hz), 7.88 (1H, dd, J=4.9,1.2 Hz), 8.07 (1H, d, J=0.7 Hz).
MS m/z: 483(M+H)⁺.

| Elementary Analysis, as C₂₄H₂₆N₄O₅S | | | |
|---|---|---|---|
| Calculated: | C,59.74; | H,5.43; | N,11.61. |
| Found: | C,60.31; | H,5.79; | N,11.08. |

### (1) Ethyl 2-[2-fluoro-6-methoxy-4-[[(5-methyl-2-phenyloxazol-4-ylmethyl)oxazol-2-ylmethylamino]methyl]phenoxy]-2-methylpropanoate

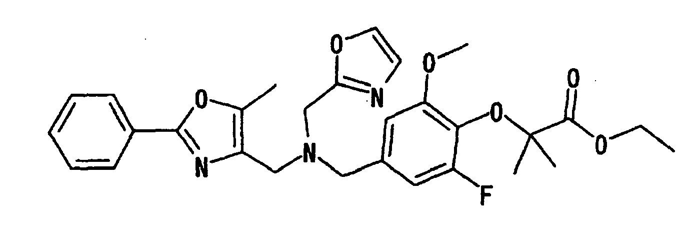

In the same manner as in Example 13-(1), the entitled compound (0.14 g) was obtained as a colorless oily substance from the compound (0.11 g) obtained in Reference Example 28 and the compound (0.10 g) obtained in Reference Example 13-(3).
¹H-NMR (400 MHz, CDCl₃) δ: 1.32 (3H, t, J=7.2 Hz), 1.49 (6H, s), 2.31 (3H, s), 3.68 (2H, s), 3.70 (2H, s), 3.78 (3H, s), 3.91 (2H, s), 4.25 (2H, q, J=7.1 Hz), 6.76 (1H, d, J=9.8 Hz), 7.09 (1H, s), 7.23 (1H, s), 7.40-7.44 (3H, m), 7.64 (1H, s), 7.98-8.02 (2H, m).
MS m/z: 538(M+H)⁺. (2) 2-[2-Fluoro-6-methoxy-4-[[(5-methyl-2-phenyloxazol-4-ylmethyl)oxazol-2-ylmethylamino]methyl]phenoxy]-2-methylpropanoic acid

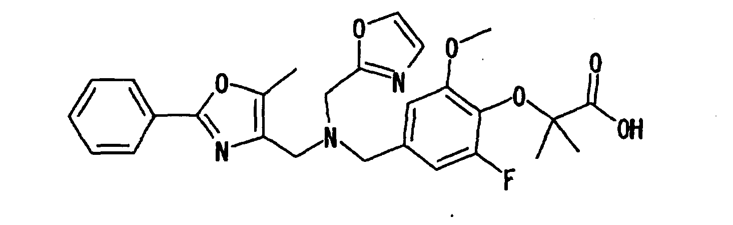

In the same manner as in Example 24-(2), a hydrochloride of the entitled compound (0.04 g) was obtained as a colorless solid from the compound (0.14 g) obtained in Example 29-(1).
¹H-NMR (400 MHz, CDCl₃) δ: 1.55 (6H, s), 2.48 (3H, s), 4.01 (3H, s), 4.06-4.30 (6H, m), 6.96 (1H, d, J=10.0 Hz), 7.22 (1H, s), 7.26 (1H, s), 7.43-7.48 (3H, m), 7.75 (1H, s), 7.98-7.94 (2H, m).
MS m/z: 510(M+H)⁺.

| Elementary Analysis, as C₂₇H₂₈FN₃O₆•HCl•1/4C₂H₈O₂•1/2H₂O | | | |
|---|---|---|---|
| Calculated: | C,58.28; | H,5.59; | N,7.28. |
| Found: | C,58.75; | H,5.66; | N,6.85. |

### [Example 30]

### (1) Ethyl 2-[2-methoxy-6-methyl-4-[[(5-methyl-2-phenyloxazol-4-ylmethyl)oxazol-2-ylmethylamino]methyl]phenoxy]-2-methylpropanoate

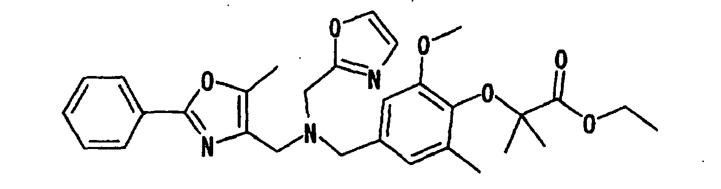

In the same manner as in Example 13-(1), the entitled compound (0.16 g) was obtained as a colorless oily substance from the compound (0.093 g) obtained in Reference Example 29 and the compound (0.10 g) obtained in Reference Example 13-(3).
¹H-NMR (400 MHz, CDCl₃) δ: 1.34 (3H, t, J=7.4 Hz), 1.43 (6H, s), 2.20 (3H, s), 2.29 (3H, s), 3.66 (2H, s), 3.67 (2H, s), 3.70 (3H, s), 3.91 (2H, s), 4.28 (2H, q, J=7.4 Hz), 6.74 (1H, s), 6.82 (1H, s), 7.08 (1H, s), 7.39-7.45 (3H, m), 7.63 (1H, s), 8.02-7.98 (2H, m).
MS m/z: 534(M+H)⁺. (2) 2-[2-Methoxy-6-methyl-4-[[(5-methyl-2-phenyloxazol-4-yhnethyl)oxazol-2-ylmethylamino]methyl]phenoxy]-2-methylpropanoic acid

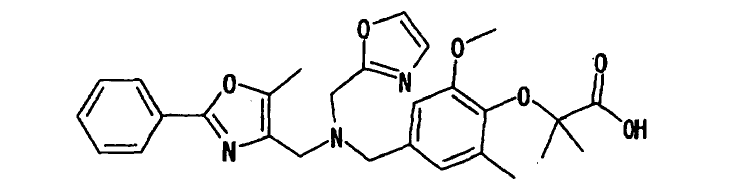

In the same manner as in Example 24-(2), a hydrochloride of the entitled compound (0.112 g) was obtained as a colorless solid from the compound (0.154 g) obtained in Example 30-(1).
¹H-NMR (400 MHz, CDCl₃) δ: 1.51 (6H, s), 2.26 (3H, s), 2.48 (3H, s), 3.93 (3H, s), 4.08-4.66 (6H, m), 6.93 (1H, s), 7.23 (1H, s), 7.26 (1H, s), 7.43-7.47 (3H, m), 7.76 (1H, s), 7.98-7.93 (2H, m).
MS m/z: 506(M+H)⁺.

| Elementary Analysis, as C₂₈H₃₁N₃O₆•HCl•1/4C₄H₈O₂•1/2H₂O | | | |
|---|---|---|---|
| Calculated: | C,60.78; | H,6.16; | N,7.33. |
| Found: | C,60.67; | H,6.26; | N,6.83. |

### [Example 31]

### (1) Ethyl 2-[2,6-dimethyl-4-[[(5-methyl-[1,3,4]oxadiazol-2-ylmethyl)-(5-methyl-2-p-tolyloxazol-4-ylmethyl)amino]methyl]phenoxy]-2-methylpropanoate

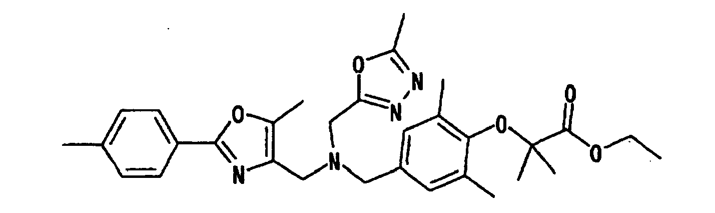

The compound (0.147 g) obtained in Reference Example 30-(5) and 4-chloromethyl-5-methyl-2-p-tolyloxazole (0.09 g) were dissolved in N,N-dimethylformamide (3 ml), and cesium carbonate (0.198 g) and potassium iodide (0.02 g) were added thereto and the resulting mixture was stirred at 60°C for 3 days. The reaction mixture was diluted with ethyl acetate, and washed with water and saturated brine in that order. This was dried over anhydrous sodium sulfate, then the solvent was evaporated under reduced pressure, and the resulting residue was purified by silica gel column chromatography (ethyl acetate/hexane = 3/1) to obtain the entitled compound (0.15 g) as an yellow oily substance.
¹H-NMR (400 MHz, CDCl₃) δ: 1.35 (3H, t, J=7.1 Hz), 1.45 (6H, s), 2.18 (6H, s), 2.26 (3H, s), 2.39 (4H, s), 2.51 (3H, s), 3.66 (4H, br s), 3.99 (2H, s), 4.28 (2H, q, J=7.2 Hz), 6.98 (2H, s), 7.24 (2H, d, J=8.1 Hz), 7.89 (2H, d, J=8.1 Hz).
MS m/z: 547(M+H)⁺. (2) 2-[2,6-Dimethyl-4-[[(5-methyl-[1,3,4]oxadiazol-2-ylmethyl)-(5-methyl-2-p-tolyloxazol-4-ylmethyl)amino]methyl]phenoxy]-2-methylpropanoic acid

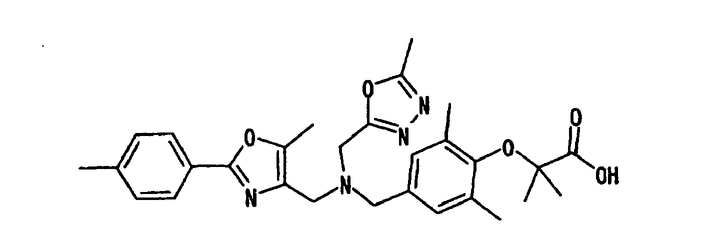

The compound (0.15 g) obtained in Example 31-(1) was dissolved in methanol (10 ml), and an aqueous 1N sodium hydroxide solution (2 ml) was added thereto, and the resulting solution was stirred at room temperature for 19 hours and then heated under reflux for 5 hours. After cooled, an aqueous 1N hydrochloric acid solution (2 ml) was added thereto, and concentrated under reduced pressure. The residue was extracted with ethyl acetate, and washed with water and saturated brine in that order. This was dried over anhydrous sodium sulfate, and the solvent was evaporated under reduced pressure. The resulting residue was purified by silica gel column chromatography (methanol/chloroform = 1/9) to obtain the entitled compound (0.082 g) as a colorless solid.
¹H-NMR (400 MHz, DMSO-d₆) δ: 1.33 (6H, s), 2.14 (6H, s), 2.25 (3H, s), 2.36 (3H, s), 2.45 (3H, s), 3.57 (2H, s), 3.61 (2H, s), 3.91 (2H, s), 6.96 (2H, s), 7.32 (2H, d, J=8.1 Hz), 7.81 (2H, d, J=8.1 Hz).
MS m/z: 519(M+H)⁺.

| Elementary Analysis, as C₂₉H₃₄N₄O₅•3/5H₂O | | | |
|---|---|---|---|
| Calculated: | C,65.79; | H,6.70; | N,10.58. |
| Found: | C,66.10; | H,6.75; | N,10.10. |

### [Example 32]

### (1) Ethyl 2-[4-[[[2-(4-chlorophenyl)-5-methyloxazol-4-ylmethyl]-(5-methyl-[1,3,4]oxadiazol-2-ylmethyl)amino]methyl]-2,6-dimethylphenoxy]-2-methylpropanoate

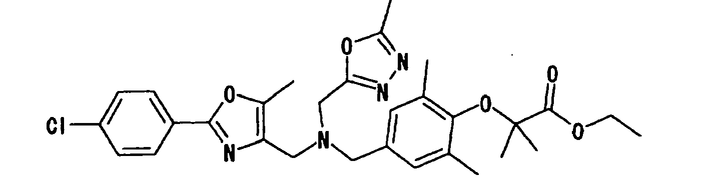

In the same manner as in Example 31-(1), the entitled compound (0.18 g) was obtained as a pale yellow oily substance from the compound (0.147 g) obtained in Reference Example 30-(5) and 4-chloromethyl-2-(4-chlorophenyl)-5-methyloxazole (0.098 g).
¹H-NMR (400 MHz, CDCl₃) δ: 1.35 (3H, t, J=7.1 Hz), 1.45 (5H, s), 2.18 (5H, s), 2.27 (2H, s), 2.51 (3H, d, J=0.7 Hz), 3.66 (1H, s), 3.66 (2H, s), 3.98 (2H, s), 4.28 (2H, q, J=7.1 Hz), 6.97 (2H, s), 7.41 (2H, d, J=8.1 Hz), 7.94 (2H, d, J=8.3 Hz).
MS m/z: 567(M+H)⁺.

### (2) 2-[4-[[[2-(4-Chlorophenyl)-5-methyloxazol-4-ylmethyl]-(5-methyl-[1,3,4]oxadiazol-2-ylmethyl)amino]methyl]-2,6-dimethylphenoxy]-2-methylpropanoic acid

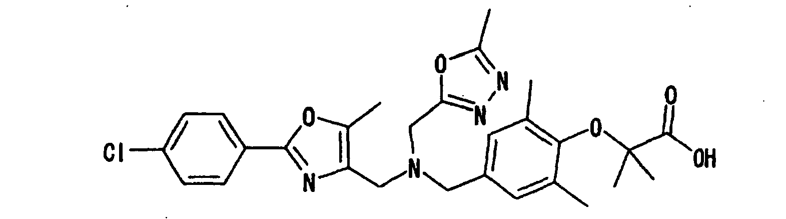

In the same manner as in Example 31-(2), the entitled compound (0.094 g) was obtained as a colorless solid from the compound (0.18 g) obtained in Example 32-(1).
¹H-NMR (400 MHz, DMSO-d₆) δ: 1.33 (6H, s), 2.13 (6H, s), 2.26 (3H, s), 2.46 (3H, s), 3.59 (2H, s), 3.61 (2H, s), 3.91 (2H, s), 6.96 (2H, s), 7.58 (2H, d, J=8.6 Hz), 7.92 (2H, d, J=8.6 Hz).
MS m/z: 540(M+H)⁺.

| Elementary Analysis, as C₂₈H₃₁ClN₄O₅•3/4H₂O | | | |
|---|---|---|---|
| Calculated: | C,60.87; | H,5.93; | N,10.14. |
| Found: | C,61.36; | H,5.92; | N,9.64. |

### [Example 33]

### (1) Ethyl 2-[2,6-dimethyl-4-[[(3-phenyl[1,2,4]oxadiazol-5-ylmethyl)thiazol-2-ylmethylamino]methyl]phenoxy]-2-methylpropanoate

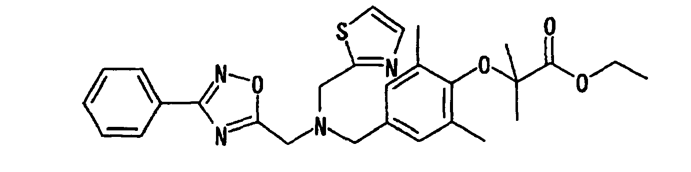

In the same manner as in Example 31-(1), the entitled compound (0.080 g) was obtained as a pale yellow oily substance from 5-chloromethyl-3-phenyl[1,2,4]oxadiazole (0.070 g) and the compound (0.13 g) obtained in Reference Example 31.
¹H-NMR (400 MHz, CDCl₃) δ: 1.35 (3H, t, J=7.1 Hz), 1.45 (6H, s), 2.20 (5H, s), 3.78 (2H, s), 4.10 (2H, s), 4.22 (2H, s), 4.28 (2H, q, J=7.1 Hz), 7.05 (2H, s), 7.33 (1H, d, J=3.4 Hz), 7.49-7.52 (4H, m), 7.72 (1H, d, J=3.2 Hz), 8.12-8.10 (2H, m).
MS m/z: 521(M+H)⁺. (2) 2-[2,6-Dimethyl-4-[[(3-phenyl[1,2,4]oxadiazol-5-ylinethyl)thiazol-2-ylmethylamino]methyl]phenoxy]-2-methylpropanoic acid

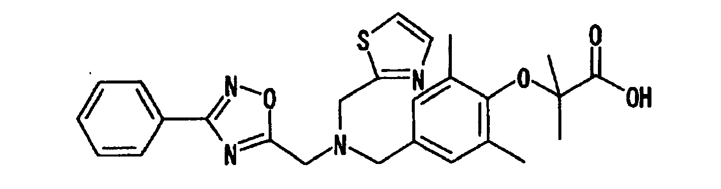

In the same manner as in Example 31-(2), the entitled compound (0.038 g) was obtained as a pale yellow oily substance from the compound (0.080 g) obtained in Example 33-(1).
¹H-NMR (400 MHz, DMSO-d₆) δ: 1.31 (6H, s), 2.14 (6H, s), 3.74 (2H, s), 4.15 (2H, s), 4.17 (2H, s), 7.03 (2H, s), 7.61-7.57 (3H, m), 7.69 (1H, d, J=3.2 Hz), 7.74 (1H, d, J=3.4 Hz), 8.01-8.03 (2H, m).
MS m/z: 493(M+H)⁺.

### [Example 34]

### (1) Ethyl 2-[2,6-dimethyl-4-[[(5-phenyloxazol-2-ylmethyl)thiazol-2-ylmethylamino]methyl]phenoxy]-2-methylpropanoate

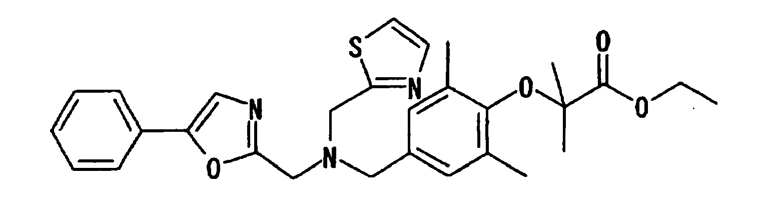

In the same manner as in Example 31-(1), the entitled compound (0.053 g) was obtained as a pale yellow oily substance from 2-chloromethyl-5-phenyloxazole (0.045 g) and the compound (0.084 g) obtained in Reference Example 31.
¹H-NMR (400 MHz, CDCl₃) δ: 1.35 (4H, t, J=7.1 Hz), 1.45 (7H, s), 2.19 (6H, s), 3.73 (2H, s), 3.96 (2H, s), 4.16 (2H, s), 4.28 (2H, q, J=7.2 Hz), 7.07 (2H, s), 7.27-7.36 (4H, m), 7.43 (2H, t, J=7.6 Hz), 7.66-7.64 (2H, m), 7.71 (1H, d, J=3.4 Hz).
MS m/z: 520(M+H)⁺.

### (2) 2-[2,6-Dimethyl-4-[[(5-phenyloxazol-2-ylmethyl)thiazol-2-ylmethylamino]methyl]phenoxy]-2-methylpropanoic acid

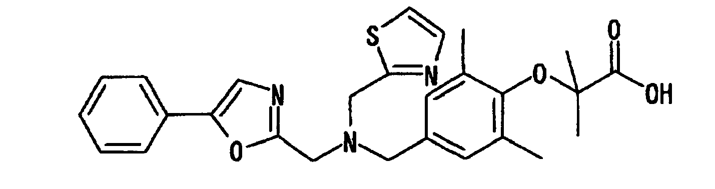

In the same manner as in Example 31-(2), the entitled compound (0.034 g) was obtained as a pale yellow solid from the compound (0.050 g) obtained in Example 34-(1).
¹H-NMR (400 MHz, DMSO-d₆) 8: 1.32 (6H, s), 2.14 (6H, s), 3.66 (2H, s), 3.91 (2H, s), 4.10 (2H, s), 7.04 (2H, s), 7.40-7.36 (1H, m), 7.49 (2H, t, J=7.7 Hz), 7.62 (1H,s), 7.67-7.73 (4H, m).
MS m/z: 492(M+H)⁺.

### [Example 35]

### (1) Ethyl 2-[2,6-dimethyl-4-[[[5-methyl-2-(5-methylthiophen-2-yl)oxazol-4-ylmethyl]thiazol-2-ylmethylamino]methyl]phenoxy]-2-methylpropanoate

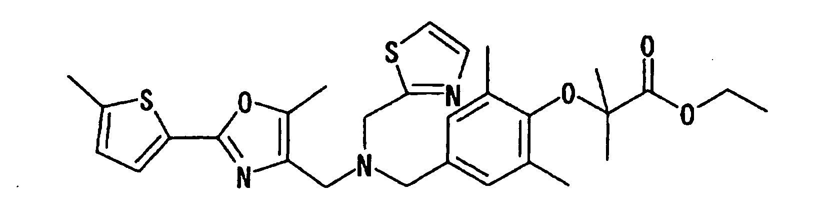

In the same manner as in Example 31-(1), the entitled compound (0.155 g) was obtained as a pale yellow oily substance from the compound (0.143 g) obtained in Reference Example 31 and the compound (0.090 g) obtained in Reference Example 32.
¹H-NMR (400 MHz, CDCl₃) δ: 1.35 (3H, t, J=7.1 Hz), 1.45 (7H, s), 2.19 (7H, s), 2.21 (3H, s), 2.51 (3H, s), 3.60 (2H, s), 3.63 (2H, s), 4.06 (2H, s), 4.28 (2H, q, J=7.0 Hz), 6.73 (1H, d, J=3.2 Hz), 7.03 (2H, s), 7.27 (1H, d, J=2.9 Hz), 7.38 (1H, d, J=3.4 Hz), 7.68 (1H, d, J=3.2 Hz).
MS m/z: 554(M+H)⁺.

### (2) 2-[2,6-Dimethyl-4-[[[5-methyl-2-(5-methylthiophen-2-yl)oxazol-4-ylmethyl]thiazol-2-ylmethylamino]methyl]phenoxy]-2-methylpropanoic acid

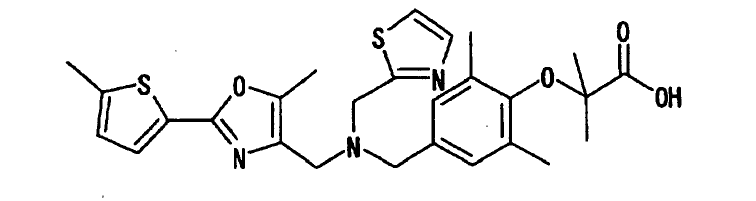

In the same manner as in Example 31-(2), the entitled compound (0.114 g) was obtained as a colorless solid from the compound (0.15 g) obtained in Example 35-(1).
¹H-NMR (400 MHz, DMSO-d₆) δ: 1.33 (6H, s), 2.15 (6H, s), 2.19 (3H, s), 3.29 (2H, s), 3.53 (2H, s), 3.57 (3H, s), 3.99 (2H, s), 6.88 (1H, dd, J=3.6, 1.1 Hz), 7.03 (2H, s), 7.40 (1H, d, J=3.4 Hz), 7.64 (1H, d, J=3.2 Hz), 7.71 (1H, d, J=3.4 Hz).
MS m/z: 526(M+H)⁺.

| Elementary Analysis, as C₂₇H₃₁N₃O₄S₂•1/2H₂O | | | | |
|---|---|---|---|---|
| Calculated: | C,60.65; | H,6.03; | N,7.86; | S,11.99. |
| Found: | C,60.70; | H,5.90; | N,7.66; | S,11.68. |

### [Example 36]

### (1) Ethyl 2-[2,6-dimethyl-4-[[[5-methyl-2-(5-methylthiophen-2-yl)oxazol-4-ylmethyl]oxazol-2-ylmethylamino]methyl]phenoxy]-2-methylpropanoate

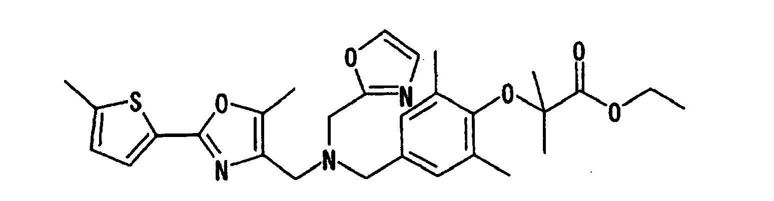

In the same manner as in Example 31-(1), the entitled compound (0.296 g) was obtained as an yellow oily substance from the compound (0.23 g) obtained in Reference Example 23 and the compound (0.15 g) obtained in Reference Example 32.
¹H-NMR (400 MHz, CDCl₃) δ: 1.35 (3H, t, J=7.1 Hz), 1.45 (6H, s), 2.17 (6H, s), 2.23 (3H, s), 2.51 (3H, s), 3.61 (4H, s), 3.90 (2H, s), 4.28 (2H, q, J=7.1 Hz), 6.73 (1H, d, J=2.7 Hz), 6.98 (2H, s), 7.07 (1H, s), 7.39 (1H, d, J=3.7 Hz), 7.63 (1H, s).
MS m/z: 538(M+H)⁺.

### (2) 2-[2,6-Dimethyl-4-[[[5-methyl-2-(5-methylthiophen-2-yl)oxazol-4-ylmethyl]oxazol-2-ylmethylamino]methyl]phenoxy]-2-methylpropanoic acid

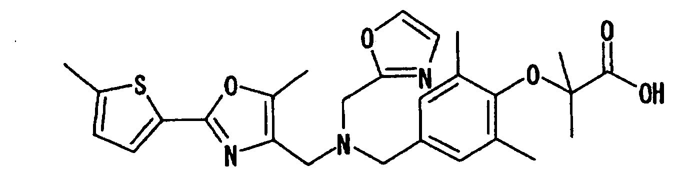

In the same manner as in Example 31-(2), the entitled compound (0.150 g) was obtained as a pale yellow solid from the compound (0.296 g) obtained in Example 36-(1).
¹H-NMR (400 MHz, DMSO-d₆) δ: 1.33 (6H, s), 2.13 (6H, s), 2.21 (3H, s), 3.51 (2H, s), 3.56 (2H, s), 3.57 (3H, s), 3.79 (2H, s), 6.88 (1H, dd, J=3.7, 1.0 Hz), 6.95 (2H, s), 7.18 (1H, d, J=0.7 Hz), 7.39 (1H, d, J=3.7 Hz), 8.07 (1H, d, J=0.7 Hz).
MS m/z: 510(M+H)⁺.

| Elementary Analysis, as C₂₇H₃₁N₃O₅S•1/2H₂O | | | | |
|---|---|---|---|---|
| Calculated: | C,62.53; | H,6.22; | N,8.10; | S,6.18. |
| Found: | C,62.73; | H,6.15; | N,7.77; | S,6.00. |

### [Example 37]

### (1) Ethyl 2-[2,6-dimethyl-4-[[[5-methyl-2-(5-methylthiophen-2-yl)oxazol-4-ylmethyl]-(5-methyloxazol-2-ylmethyl)amino]methyl]phenoxy]-2-inethylpropanoate

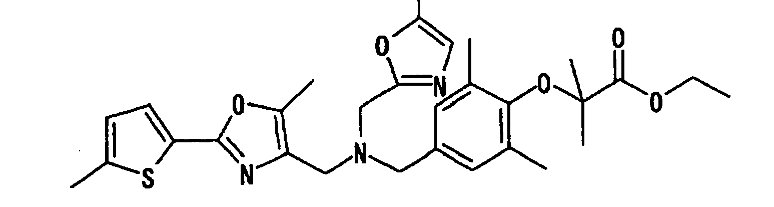

In the same manner as in Example 31-(1), the entitled compound (0.16 g) was obtained as a pale yellow oily substance from the compound (0.090 g) obtained in Reference Example 32 and the compound (0.142 g) obtained in Reference Example 33-(4).
¹H-NMR (400 MHz, CDCl₃) δ: 1.35 (3H, t, J=7.1 Hz), 1.45 (6H, s), 2.18 (6H, s), 2.24 (3H, s), 2.30 (3H, s), 2.51 (3H, s), 3.61 (2H, s), 3.61 (2H, s), 3.82 (2H, s), 4.28 (2H, q, J=7.2 Hz), 6.65 (1H, d, J=1.2 Hz), 6.73 (1H, dd, J=3.7, 1.0 Hz), 6.98 (2H, s), 7.39 (1H, d, J=3.7 Hz).
MS m/z: 552(M+H)⁺.

### (2) 2-[2,6-Dimethyl-4-[[[5-methyl-2-(5-methylthiophen-2-yl)oxazol-4-ylmethyl]-(5-methyloxazol-2-ylmethyl)amino]methyl]phenoxy]-2-methylpropanoic acid

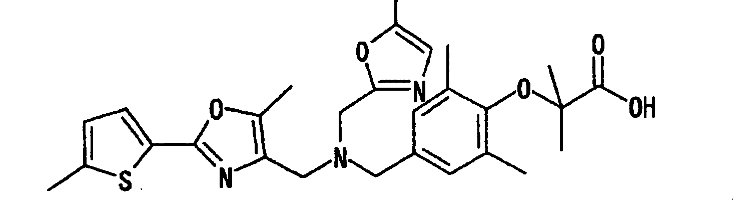

In the same manner as in Example 31-(2), the entitled compound was obtained from the compound (0.16 g) obtained in Example 37-(1), then 4 N hydrochloric acid-dioxane was added thereto and dried to solidness under reduced pressure to obtain a hydrochloride of the entitled compound (0.11 g) as a colorless solid.
¹H-NMR (400 MHz, DMSO-d₆) δ: 1.35 (6H, s), 2.16 (6H, s), 2.27-2.28 (6H, m), 2.49-2.51 (3H, m), 4.21-4.01 (6H, m), 6.89-6.92 (2H, m), 7.12 (2H, s), 7.45 (1H, d, J=3.7 Hz).
MS m/z: 524(M+H)⁺.

| Elementary Analysis: as C₂₈H₃₃N₃O₅S•HCl•1/2H₂O | | | | |
|---|---|---|---|---|
| Calculated: | C,59.09; | H,6.20; | N,7.38; | S,5.63. |
| Found: | C,59.20; | H,6.04; | N,7.13; | S,5.65. |

### [Example 38]

### (1) Ethyl 2-[2,6-dimethyl-4-[[(5-methyloxazol-2-ylinethyl)-(3-phenyl[1,2,4]oxadiazol-5-ylmethyl)amino]methyl]phenoxy]-2-methylpropanoate

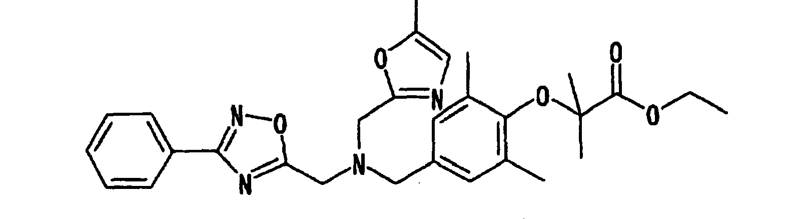

In the same manner as in Example 31-(1), the entitled compound (0.12 g) was obtained as a pale yellow oily substance from 5-chloromethyl-3-phenyl[1,2,4-oxadiazole (0.070 g) and the compound (0.13 g) obtained in Reference Example 33-(4).
¹H-NMR (400 MHz, CDCl₃) δ: 1.35 (3H, t, J=7.1 Hz), 1.45 (6H, s), 2.19 (6H, s), 2.29 (3H, d, J=1.2 Hz), 3.77 (2H, s), 3.98 (2H, s), 4.12 (2H, s), 4.28 (2H, q, J=7.2 Hz), 6.66 (1H, d, J=1.2 Hz), 7.01 (2H, s), 7.52-7.48 (3H, m), 8.09-8.12 (2H, m).
MS m/z: 519(M+H)⁺.

### (2) 2-[2,6-Dimethyl-4-[[(5-methyloxazol-2-ylmethyl)-(3-phenyl[1,2,4]oxadiazol-5-ylmethyl)amino]methyl]phenoxy]-2-methylpropanoic acid

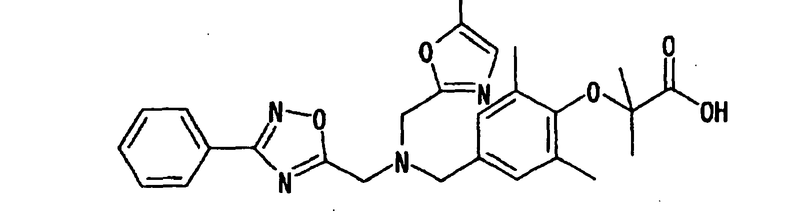

In the same manner as in Example 31-(2), the entitled compound was obtained from the compound (0.12 g) obtained in Example 38-(1), then 4 N hydrochloric acid-dioxane was added thereto and dried up under reduced pressure to obtain a hydrochloride of the entitled compound (0.082 g) as a pale yellow oily substance.
¹H-NMR (400 MHz, DMSO-d₆) δ: 1.32 (6H, s), 2.14 (6H, s), 2.22 (3H, s), 3.76 (2H, s), 3.91 (2H, s), 4.17 (2H, s), 6.73 (1H, d, J=1.2 Hz), 6.98 (2H, s), 7.56-7.61 (3H, m), 7.99-8.01 (2H, m).
MS m/z: 491(M+H)⁺.

### [Example 39]

### (1) Ethyl 2-[2,6-dimethyl-4-[[(5-methyloxazol-2-ylmethyl)-(5-phenyloxazol-2-ylmethyl)amino]methyl]phenoxy]-2-methylpropanoate

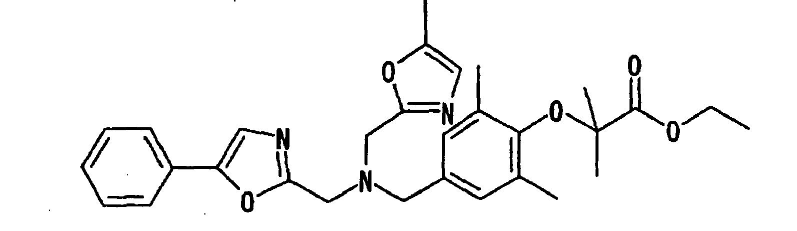

In the same manner as in Example 31-(1), the entitled compound (0.11 g) was obtained as a pale yellow oily substance from 2-chloromethyl-5-phenyloxazole (0.045 g) and the compound (0.084 g) obtained in Example 33-(4).
MS m/z: 518(M+H)⁺.

### (2) 2-[2,6-Dimethyl-4-[[(5-methyloxazol-2-ylmethyl)-(5-phenyloxazol-2-ylmethyl)amino]methyl]phenoxy]-2-methylpropanoic acid

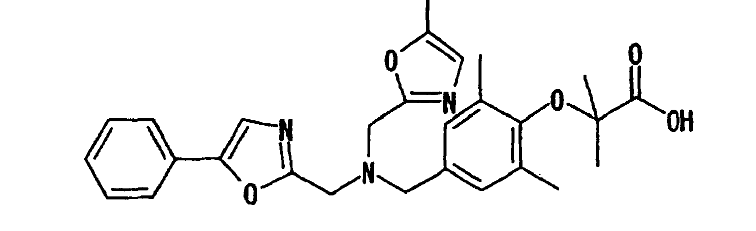

In the same manner as in Example 31-(2), the entitled compound was obtained from the compound (0.11 g) obtained in Example 39-(1), then 4 N hydrochloric acid-dioxane was added thereto and dried to solidness under reduced pressure to obtain a hydrochloride of the entitled compound (0.033 g) as a pale yellow solid.
¹H-NMR (400 MHz, DMSO-d₆) δ: 1.32 (6H, s), 2.13 (6H, s), 2.25 (3H, s), 3.76 (2H, s), 3.95 (2H, s), 4.00 (2H, s), 6.78 (1H, d, J=1.2 Hz), 6.99 (2H, s), 7.37-7.40 (1H, m), 7.46-7.50 (2H, m), 7.62 (1H, s), 7.68-7.72 (2H, m).
MS m/z: 490(M+H)⁺.

### [Example 40]

### (1) Ethyl 2-[2,6-dimethyl-4-[[methylcarbamoylmethyl-(5-methyl-2-p-tolyloxazol-4-ylmethyl)amino]methyl]phenoxy]-2-methylpropanoate

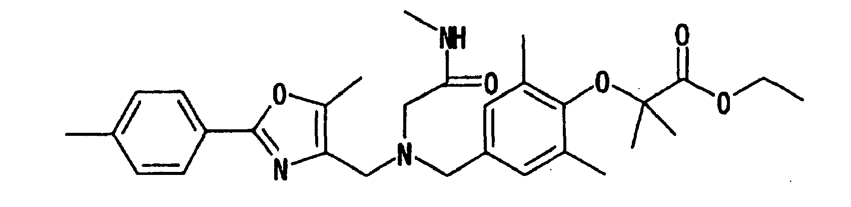

In the same manner as in Example 14-(1), the entitled compound (0.084 g) was obtained as a colorless oily substance from the compound (0.232 g) obtained in Reference Example 34-(2), methylamine hydrochloride (0.037 g) and triethylamine (0.172 ml).
¹H-NMR (400 MHz, CDCl₃) δ: 1.35 (3H, t, J=7.2 Hz), 1.45 (6H, s), 2.17 (6H, s), 2.25 (3H, s), 2.41 (3H, s), 2.79 (3H, d, J=4.9 Hz), 3.26 (2H, s), 3.55 (2H, s), 3.66 (2H, s), 4.28 (2H, q, J=7.2 Hz), 6.92 (2H, s), 7.26 (2H, d, J=7.8 Hz), 7.80-7.94 (3H, m).
MS m/z: 522(M+H)⁺.

### (2) 2-[2,6-Dimethyl-4-[[methylcarbamoylmethyl-(5-methyl-2-p-tolyloxazol-4-ylmethyl)amino]methyl]phenoxy]-2-methylpropanoic acid

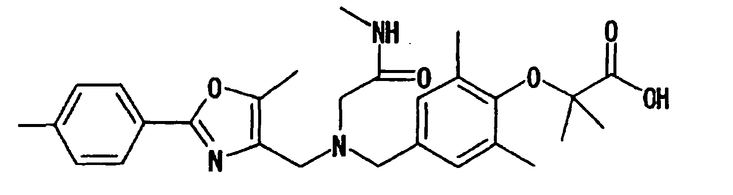

In the same manner as in Example 16-(2), the compound (0.084 g) obtained in Example 40-(1) was treated and then purified by silica gel column chromatography (11% methanolchloroform) to obtain the entitled compound (0.072 g) as a colorless solid.
¹H-NMR (400 MHz, CDCl₃) δ: 1.49 (6H, s), 2.20 (6H, s), 2.27 (3H, s), 2.40 (3H, s), 2.78 (3H, d, J=4.9 Hz), 3.24 (2H, br s), 3.53 (2H, br s), 3.63 (2H, br s), 6.92 (2H, s), 7.22-7.29 (2H, m), 7.85-8.01 (3H, m).
MS m/z: 494(M+H)⁺.

| Elementary Analysis, as C₂₈H₃N₃O₅•1/2H₂O | | | |
|---|---|---|---|
| Calculated: | C,66.91; | H,7.22; | N,8.36. |
| Found: | C,67.10; | H,7.28; | N,8.09. |

### [Example 41]

### (1) Ethyl 2-[4-[[[2-(4-chlorophenyl)-5-methyloxazol-4-ylmethyl]methylcarbamoylmethylamino]methyl]-2,6-dimethylphenoxy]-2-methylpropanoate

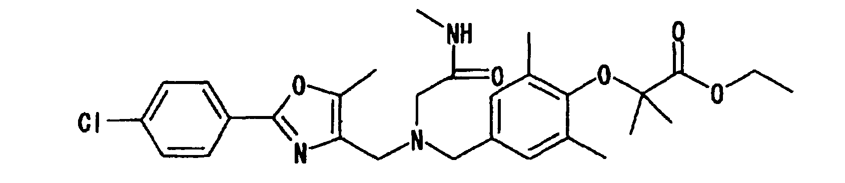

In the same manner as in Example 40-(1), the entitled compound (0.220 g) was obtained as a pale yellow oily substance from the compound (0.261 g) obtained in Reference Example 35-(2).
¹H-NMR (400 MHz, CDCl₃) δ: 1.35 (3H, t, J=7.2 Hz), 1.45 (6H, s), 2.17 (6H, s), 2.24 (3H, s), 2.79 (3H, d, J=5.1 Hz), 3.22 (2H, s), 3.50 (2H, s), 3.59 (2H, s), 4.28 (2H, q, J=7.2 Hz), 6.88 (2H, s), 7.40-7.46 (2H, m), 7.64-7.75 (1H, m), 7.90-7.96 (2H, m).
MS m/z: 542(M+H)⁺.

### (2) 2-[4-[[[2-(4-Chlorophenyl)-5-methyloxazol-4-ylmethyl]methylcarbamoylmethylamino]methyl]-2,6-dimethylphenoxy]-2-methylpropanoic acid

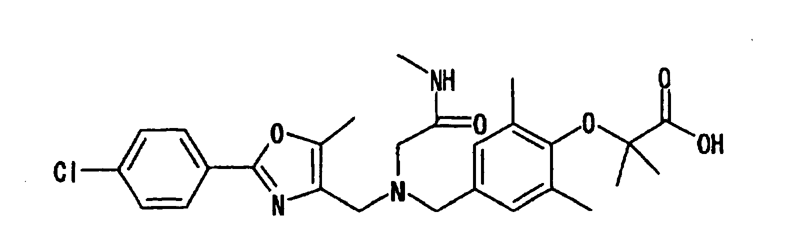

In the same manner as in Example 40-(2), the entitled compound (0.180 g) was obtained as a colorless solid from the compound (0.220 g) obtained in Example 41-(1).
¹H-NMR (400 MHz, CDCl₃) δ: 1.49 (6H, s), 2.20 (6H, s), 2.28 (3H, s), 2.79 (3H, d, J=4.9 Hz), 3.24 (2H, br s), 3.53 (2H, br s), 3.63 (2H, br s), 6.92 (2H, br s), 7.40-7.47 (2H, m), 7.76-7.89 (1H, m), 7.90-7.97 (2H, m).
MS m/z: 514(M+H)⁺.

| Elementary Analysis, as C₂₇H₃₂ClN₃O₅ | | | | |
|---|---|---|---|---|
| Calculated: | C,63.09; | H,6.27; | Cl,6.90; | N,8.17. |
| Found: | C,63.02; | H,6.30; | Cl,6.97; | N,8.10. |

### [Example 42]

### (1) Ethyl 2-[4-[[[2-(3-chlorophenyl)-5-methyloxazol-4-ylmethyl]methylcarbamoylmethylamino]methyl]-2,6-dimethylphenoxy]-2-methylpropanoate

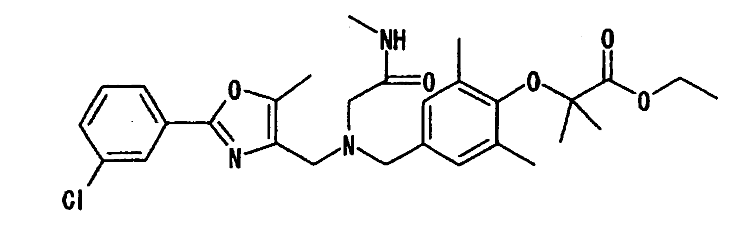

In the same manner as in Example 40-(1), the entitled compound (0.194 g) was obtained as a pale yellow oily substance from the compound (0.214 g) obtained in Reference Example 36-(2).
¹H-NMR (400 MHz, CDCl₃) δ: 1.35 (3H, t, J=7.2 Hz), 1.45 (6H, s), 2.17 (6H, s), 2.25 (3H, s), 2.81 (3H, d, J=4.9 Hz), 3.22 (2H, s), 3.50 (2H, s), 3.59 (2H, s), 4.28 (2H, q, J=7.2 Hz), 6.89 (2H, br s), 7.36-7.43 (2H, m), 7.65-7.75 (1H, m), 7.84-7.91 (1H, m), 7.97-8.00 (1H, m).
MS m/z: 542(M+H)⁺.

### (2) 2-[4-[[[2-(3-Chlorophenyl)-5-methyloxazol-4-ylmethyl]methylcarbamoylmethylamino]methyl]-2,6-dimethylphenoxy]-2-methylpropanoic acid

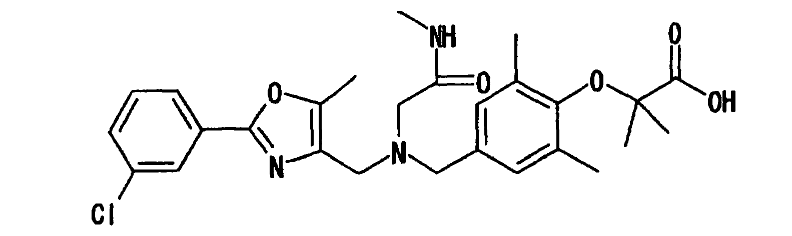

In the same manner as in Example 40-(2), the entitled compound (0.156 g) was obtained as a colorless solid from the compound (0.194 g) obtained in Example 42-(1).
¹H-NMR (400 MHz, CDCl₃) δ: 1.49 (6H, s), 2.20 (6H, s), 2.29 (3H, s), 2.80 (3H, d, J=4.9 Hz), 3.24 (2H, s), 3.53 (2H, s), 3.62 (2H, s), 6.92 (2H, s), 7.36-7.44 (2H, m), 7.76-7.92 (2H, m), 7.97-8.01 (1H, m).
MS m/z: 514(M+H)⁺.

| Elementary Analysis, as C₂₇H₃₂ClN₃O₅•1/4H₂O | | | | |
|---|---|---|---|---|
| Calculated: | C,62.54; | H,6.32; | C1,6.84; | N,8.10. |
| Found: | C,62.45; | H,6.36; | C1,6.71; | N,7.80. |

### [Example 43]

### (1) tert-Butyl 2-[2-methoxy-6-methyl-4-[[methylcarbamoylmethyl-(5-methyl-2-phenyloxazol-4-ylmethyl)amino]methyl]phenoxy]-2-methylpropanoate

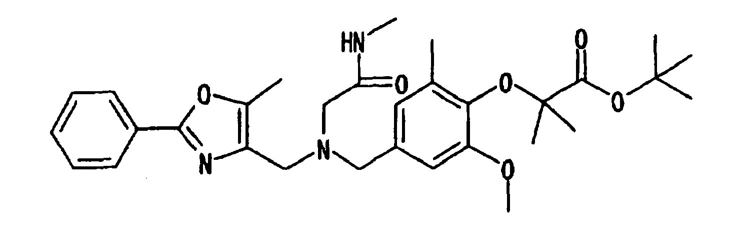

In the same manner as in Example 14-(1), the entitled compound (0.109 g) was obtained as a colorless oily substance from the compound (0.118 g) obtained in Reference Example 37-(3).
¹H-NMR (400 MHz, CDCl₃) δ: 1.40 (6H, s), 1.50 (9H, s), 2.20 (3H, s), 2.28 (3H, s), 2.79 (3H, d, J=4.9 Hz), 3.22 (2H, s), 3.52 (2H, s), 3.62 (2H, s), 3.68 (3H, s), 6.65 (1H, d, J=1.7 Hz), 6.68 (1H, d, J=1.7 Hz), 7.41-7.48 (3H, m), 7.85 (1H, q, J=4.9 Hz), 7.97-8.02 (2H, m).
MS m/z: 552(M+H)⁺.

### (2) 2-[2-Methoxy-6-methyl-4-[[methylcarbamoylmethyl-(5-methyl-2-phenyloxazol-4-ylmethyl)amino]methyl]phenoxy]-2-methylpropanoic acid

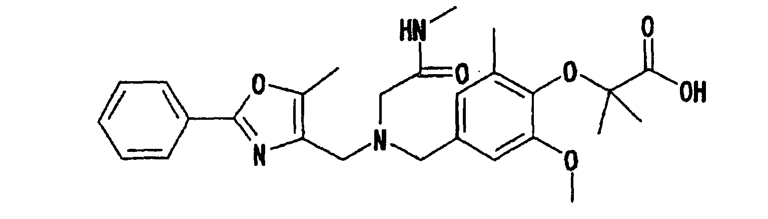

In the same manner as in Example 1-(2), the compound (0.109 g) obtained in Example 43-(1) was treated and then purified by silica gel column chromatography (methanol/chloroform = 8/92) to obtain the entitled compound (0.087 g) as a colorless solid.
¹H-NMR (400 MHz, CDCl₃) δ: 1.48 (6H, s), 2.24 (3H, s), 2.31 (3H, s), 2.81 (3H, d, J=4.9 Hz), 3.21 (2H, s), 3.55 (2H, s), 3.65 (2H, s), 3.77 (3H, s), 6.73 (1H, d, J=1.7 Hz), 6.76 (1H, d, J=1.7 Hz), 7.44-7.48 (3H, m), 7.90 (1H, q, J=4.6 Hz), 8.01-7.96 (2H, m).
MS m/z: 496(M+H)⁺.

| Elementary Analysis, as C₂₇H₃₃N₃O₆•1/2H₂O | | | |
|---|---|---|---|
| Calculated: | C,64.27; | H,6.79; | N,8.33. |
| Found: | C,64.34; | H,6.71; | N,8.16. |

### [Example 44]

### (1) tert-Butyl 2-[2-methoxy-6-methyl-4-[[(5-methyl-2-phenyloxazol-4-ylmethyl)thiazol-2-ylmethylamino]methyl]phenoxy]-2-methylpropanoate

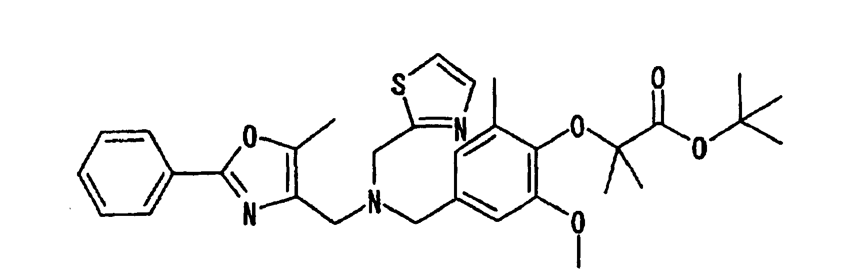

In the same manner as in Example 1-(1), the entitled compound (0.058 g) was obtained as a colorless oily substance from the compound (0.100 g) obtained in Reference Example 37-(2).
¹H-NMR (400 MHz, CDCl₃) δ: 1.40 (6H, s), 1.51 (9H, s), 2.22 (3H, s), 2.28 (3H, s), 3.66 (2H, s), 3.68 (2H, s), 3.76 (3H, s), 4.08 (2H, s), 6.77 (1H, d, J=1.7 Hz), 6.90 (1H, d, J=1.7 Hz), 7.28 (1H, d, J=3.2 Hz), 7.38-7.45 (3H, m), 7.69 (1H, d, J=3.4 Hz), 7.97-8.02 (2H, m).
MS m/z:578(M+H)⁺.

### (2) 2-[2-Methoxy-6-methyl-4-[[(5-methyl-2-phenyloxawl-4-ylmethyl)thiazol-2-ylmethylamino]methyl]phenoxy]-2-methylpropanoic acid

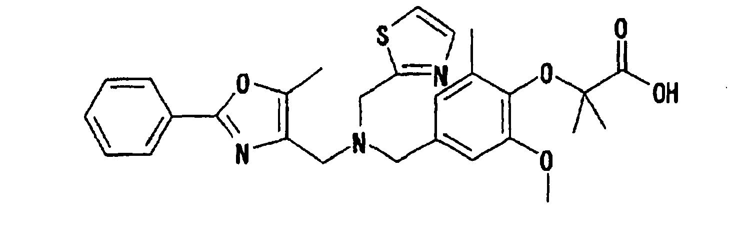

In the same manner as in Example 1-(2), the entitled compound (0.041 g) was obtained as a colorless solid from the compound (0.058 g) obtained in Example 44-(1).
¹H-NMR (400 MHz, CDCl₃) δ: 1.49 (6H, s), 2.27 (3H, s), 2.31 (3H, s), 3.69 (2H, s), 3.74 (2H, s), 3.87 (3H, s), 4.07 (2H, s), 6.85 (1H, s), 7.01 (1H, s), 7.29 (1H, d, J=3.2 Hz), 7.40-7.47 (3H, m), 7.70 (1H, d, J=3.2 Hz), 8.01-7.97 (2H, m).
MS m/z: 522(M+H)⁺.

| Elementary Analysis, as C₂₈H₃₁N₃O₅S•1/4H₂O | | | | |
|---|---|---|---|---|
| Calculated: | C,63.92; | H,6.03; | N,7.99; | S,6.09. |
| Found: | C,63.92; | H,5.97; | N,7.63; | S,5.93. |

### [Example 45]

### (1) Ethyl 2-[2-fluoro-4-[[(5-methyl-2-phenyloxazol-4-ylmethyl)oxazol-2-ylmethylamino]methyl]phenoxy]-2-methylpropanoate

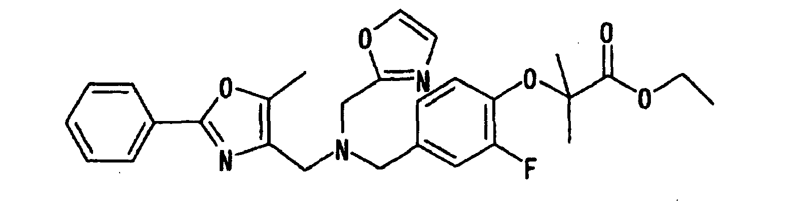

In the same manner as in Example 13-(1), the entitled compound (0.082 g) was obtained as a colorless oily substance from the compound (0.094 g) obtained in Reference Example 38 and the compound obtained in Reference Example 13-(3).
¹H-NMR (400 MHz, CDCl₃) δ: 1.28 (3H, t, J=7.1 Hz), 1.56 (6H, s), 2.31 (3H, s), 3.67 (2H, s), 3.70 (2H, s), 3.89 (2H, s), 4.24 (2H, q, J=7.1 Hz), 6.92 (1H, t, J=8.4 Hz), 7.03 (1H, d, J=8.3 Hz), 7.08 (1H, s), 7.19 (1H, dd, J=11.8, 1.7 Hz), 7.38-7.46 (3H, m), 7.64 (1H, s), 8.03-7.98 (2H, m).
MS m/z: 508(M+H)⁺.

### (2) 2-[2-Fluoro-4-[[(5-methyl-2-phenyloxazol-4-ylmethyl)oxazol-2-ylmethylamino]methyl]phenoxy]-2-methylpropanoic acid

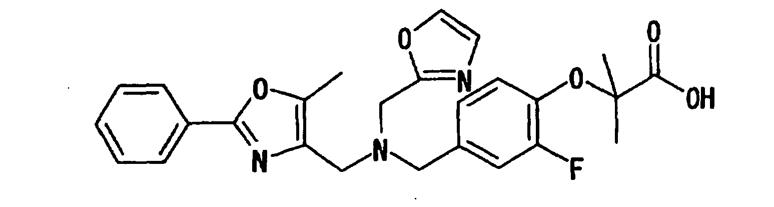

In the same manner as in Example 24-(2), a hydrochloride of the entitled compound (0.059 g) was obtained as a colorless solid from the compound (0.082 g) obtained in Example 45-(1).
MS m/z: 450(M+H)⁺.

| Elementary Analysis, as C₂₆H₂₆FN₃O₅·HCl | | | |
|---|---|---|---|
| Calculated: | C,60.52; | H,5.27; | N,8.14. |
| Found: | C,60.38; | H,5.23; | N,7.98. |

### [Example 46]

### (1) Ethyl 2-[2,6-dimethyl-4-[[[2-(5-methyl-2-phenyloxazol-4-yl)ethyl]oxazol-2-ylmethylamino]methyl]phenoxy]-2-methylpropanoate

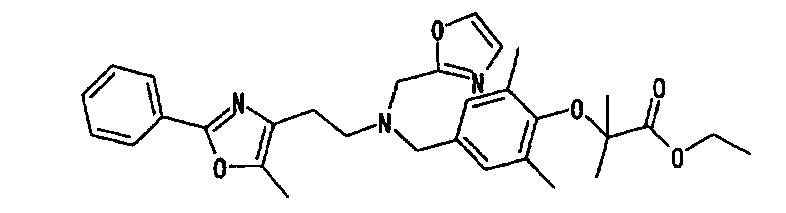

In the same manner as in Example 31-(1), the entitled compound (0.069 g) was obtained as a colorless oily substance from the compound (0.100 g) obtained in Reference Example 23 and 2-(5-methyl-2-phenyloxazol-4-yl)ethyl toluene-4-sulfonate (0.206 g).
¹H-NMR (400 MHz, CDCl₃) δ: 1.35 (3H, t, J=7.1 Hz), 1.44 (6H, s), 2.15 (6H, s), 2.28 (3H, s), 2.67-2.74 (2H, m), 2.81-2.87 (2H, m), 3.64 (2H, s), 3.86 (2H, s), 4.28 (2H, q, J=7.1 Hz), 6.93 (2H, s), 7.06 (1H, d, J=0.7 Hz), 7.37-7.44 (3H, m), 7.61 (1H, d, J=0.7 Hz), 7.99-7.93 (2H, m). MS m/z: 532(M+H)⁺.

### (2) 2-[2,6-Dimethyl-4-[[[2-(5-methyl-2-phenyloxazol-4-yl)ethyl]oxazol-2-ylmethylamino]methyl]phenoxy]-2-methylpropanoic acid

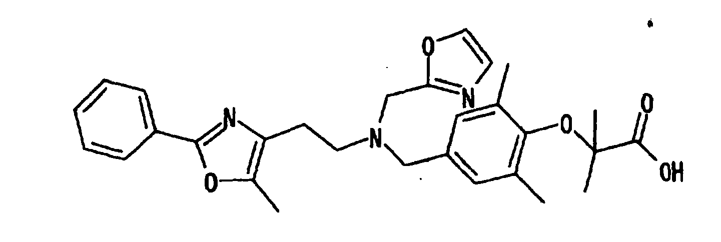

In the same manner as in Example 31-(2), the entitled compound (0.026 g) was obtained as a pale yellow oily substance from the compound (0.069 g) obtained in Example 46-(1).
¹H-NMR (400 MHz, CDCl₃) δ: 1.46 (6H, s), 2.16 (6H, s), 2.30 (3H, s), 2.66 (2H, t, J=7.2 Hz), 2.83 (2H, t, J=7.2 Hz), 3.63 (2H, s), 3.88 (2H, s), 6.93 (2H, s), 7.08 (1H, s), 7.44-7.38 (3H, m), 7.63 (1H, d, J=0.7 Hz), 7.95-7.90 (2H, m).
MS m/z: 504(M+H)⁺.

### [Example 47]

### (1) Ethyl 2-[2,6-dimethyl-4-[[oxadiazol-2-ylmethyl-(5-phenylisoxazol-3-ylmethyl)amino]methyl]phenoxy]-2-methylpropanoate

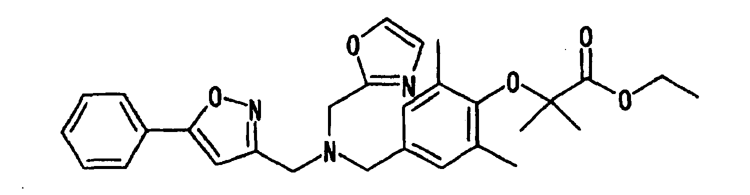

In the same manner as in Example 31-(1) but using potassium carbonate in place of cesium carbonate, the entitled compound (0.142 g) was obtained as a pale yellow oily substance from the compound (0.166 g) obtained in Reference Example 23 and the compound obtained in Reference Example 39.
¹H-NMR (400 MHz, CDCl₃) δ: 1.35 (3H, t, J=7.1 Hz), 1.45 (6H, s), 2.19 (6H, s), 3.64 (2H, s), 3.81 (2H, s), 3.85 (2H, s), 4.28 (2H, q, J=7.1 Hz), 6.59 (1H, s), 6.99 (2H, s), 7.09 (1H, s), 7.41-7.48 (3H, m), 7.65 (1H, s), 7.79 (2H, d, J=8.3 Hz).
MS m/z: 504(M+H)⁺.

### (2) 2-[2,6-Dimethyl-4-[[oxadiazol-2-ylmethyl-(5-phenylisoxazol-3-ylmethyl)amino]methyl]phenoxy]-2-methylpropanoic acid

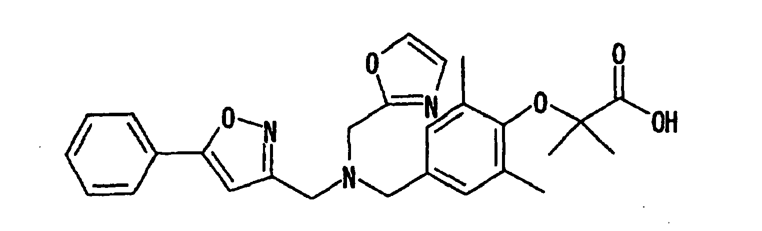

In the same manner as in Example 31-(2), the entitled compound (0.083 g) was obtained as a colorless solid from the compound (0.138 g) obtained in Example 47-(1).
¹H-NMR (400 MHz, DMSO-d₆) δ: 1.33 (6H, s), 2.15 (6H, s), 3.59 (2H, s), 3.80 (2H, s), 3.81 (2H, s), 6.98 (2H, s), 7.01 (1H, s), 7.19 (1H, s), 7.48-7.57 (3H, m), 7.88 (2H, d, J=7.3 Hz), 8.08 (1H, s).
MS m/z: 476(M+H)⁺.

| Elementary Analysis, as C₂₇H₂₉N₃O₅•1/4C₄O₂•3/4H₂O | | | |
|---|---|---|---|
| Calculated: | C,66.98; | H,6.32; | N,8.37. |
| Found: | C,67.15; | H,6.31; | N,8.01. |

### [Example 48]

### (1) Ethyl 2-[2-ethyl-4-[[(s-methyl-2-phenyloxazol-4-ylmethyl)-oxazol-2-ylmethylamino]methyl]phenoxy]-2-methylpropanoate

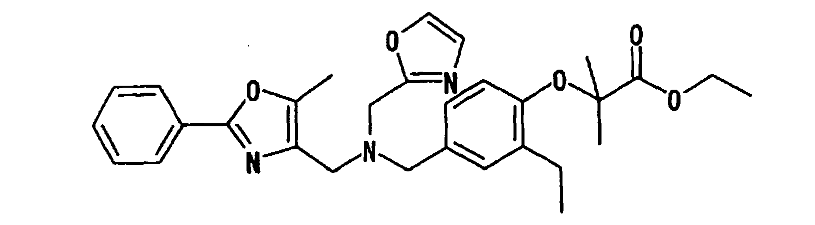

In the same manner as in Example 13-(1), the entitled compound (0.107 g) was obtained as a colorless oily substance from the compound (0.100 g) obtained in Reference Example 13-(3) and ethyl 2-(2-ethyl-4-formylphenoxy)-2-methylpropanoate (0.099 g).
¹H-NMR (400 MHz, CDCl₃) δ: 1.19 (3H, t, J=7.6 Hz), 1.23 (3H, t, J=7.1 Hz), 1.58 (6H, s), 2.30 (3H, s), 2.63 (2H, q, J=7.4 Hz), 3.66 (2H, s), 3.67 (2H, s), 3.89 (2H, s), 4.22 (2H, q, J=7.1 Hz), 6.59 (1H, d, J=8.1 Hz), 7.09-7.04 (2H, m), 7.19 (1H, d, J=1.7 Hz), 7.45-7.36 (3H, m), 7.63 (1H, s), 8.02-7.97 (2H, m).
MS m/z: 518(M+H)⁺.

### (2) 2-[2-Ethyl-4-[[(5-methyl-2-phenyloxazol-4-ylmethyl)-oxazol-2-ylmethylamino]methyl]phenoxy]-2-methylpropanoic acid

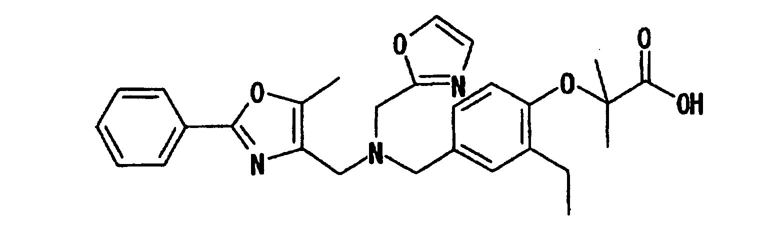

In the same manner as in Example 31-(2), the entitled compound was obtained from the compound (0.107 g) obtained in Example 48-(1), and then 4 N hydrochloric acid-dioxane was added to it and concentrated to dryness to obtain a hydrochloride of the entitled compound (0.075 g) as a colorless solid.
MS m/z: 490(M+H)⁺.

| Elementary Analysis, as C₂₈H₃₁N₃O₅•HCl | | | | |
|---|---|---|---|---|
| Calculated: | C,63.93; | H,6.13; | Cl,6.74; | N,7.99. |
| Found: | C,63.74; | H,6.19; | Cl,6.46; | N,7.64. |

### [Example 49]

### (1) Ethyl 2-[2-ethyl-6-methyl-4-[[(5-methyl-2-phenyloxazol-4-ylmethyl)oxazol-2-ylmethylamino]methyl]phenoxy]-2-methylpropanoate

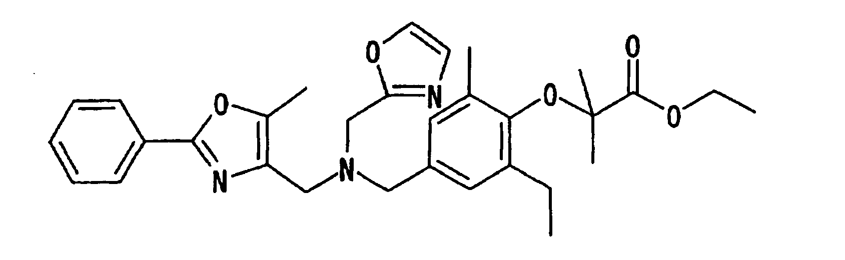

In the same manner as in Example 13-(1), the entitled compound (0.156 g) was obtained as a colorless oily substance from the compound (0.120 g) obtained in Reference Example 13-(3) and the compound (0.124 g) obtained in Reference Example 40.
¹H-NMR (400 MHz, CDCl₃) δ: 1.18 (3H, t, J=7.6 Hz), 1.35 (3H, t, J=7.4 Hz), 1.44 (6H, s), 2.18 (3H, s), 2.28 (3H, s), 2.56 (2H, q, J=7.5 Hz), 3.66 (2H, s), 3.66 (2H, s), 3.93 (2H, s), 4.28 (2H, q, J=7.1 Hz), 7.00 (1H, s), 7.04 (1H, s), 7.08 (1H, s), 7.39-7.45 (3H, m), 7.64 (1H, s), 8.03-7.98 (2H, m).
MS m/z: 532(M+H)⁺.

### (2) 2-[2-Ethyl-6-methyl-4-[[(5-methyl-2-phenyloxazol-4-ylmethyl)oxazol-2-ylmethylamino]methyl]phenoxy]-2-methylpropanoic acid

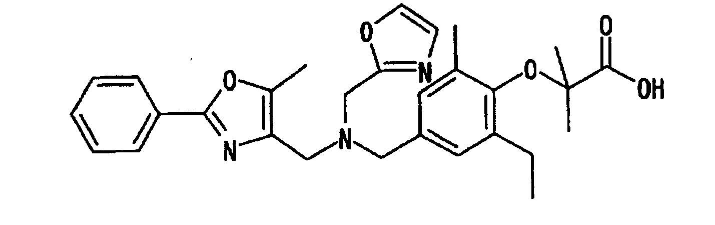

In the same manner as in Example 31-(2), the entitled compound was obtained from the compound (0.154 g) obtained in Example 49-(1), then 4 N hydrochloric acid-dioxane was added to it and concentrated to dryness, and crystallized from ethyl acetate-hexane to obtain a hydrochloride of the entitled compound (0.094 g) as a colorless solid.
MS m/z: 504(M+H)⁺.

| Elementary Analysis, as C₂₉H₃₃N₃O₅•0.85HCl•0.5H₂O | | | | |
|---|---|---|---|---|
| Calculated: | C,64.08; | H,6.46; | Cl,5.54; | N,7.70. |
| Found: | C,64.33; | H,6.64; | Cl,5.56; | N,7.28. |

### [Example 50]

### (1) tert-Butyl 2-[2,6-dimethyl-4-[[(5-methyl-2-phenyloxazol-4-ylmethyl)-(5-trifluoromethyl-[1,3,4]oxadiazol-2-ylmethyl)amino]methyl]phenoxy]-2-methylpropanoate

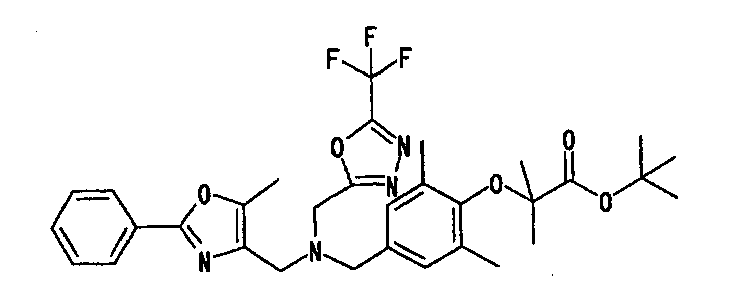

In the same manner as in Example 18, the entitled compound (0.036 g) was obtained as a pale yellow oily substance from the compound (0.135 g) obtained in Reference Example 41-(4).
¹H-NMR (400 MHz, CDCl₃) δ: 1.41 (6H, s), 1.51 (9H, s), 2.21 (6H, s), 2.28 (3H, s), 3.70 (2H, s), 3.72 (2H, s), 4.12 (2H, s), 6.96 (2H, s), 7.42-7.44 (3H, m), 8.00-7.98 (2H, m).
MS m/z: 615(M+H)⁺.

### (2) 2-[2,6-Dimethyl-4-[[(5-methyl-2-phenyloxazol-4-ylmethyl)-(5-trifluoromethyl-[1,3,4]oxadiazol-2-ylmethyl)amino]methyl]phenoxy]-2-methylpropanoic acid

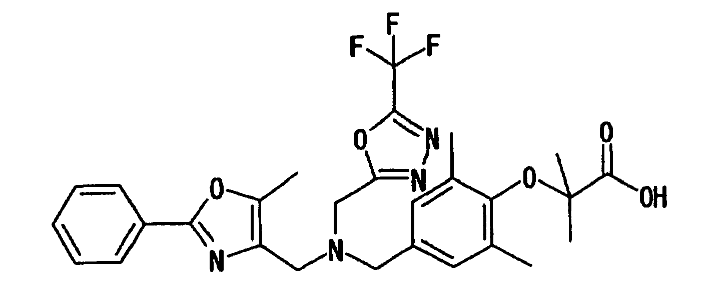

The compound (0.035 g) obtained in Example 50-(1) was dissolved in dichloromethane (5 ml), and with cooling with ice in water, trifluoroacetic acid (1 ml) was added thereto and the resulting solution was stirred at room temperature for 15 hours. The solvent was evaporated, and the resulting residue was purified by preparative thin-layer silica gel chromatography (methanol/chloroform = 1/19) to obtain the entitled compound. This was dissolved in 4 N hydrochloric acid-dioxane solution added thereto, and then concentrated to dryness to obtain a hydrochloride of the entitled compound (0.030 g) as a colorless solid.
¹H-NMR (400 MHz, DMSO-d₆) δ: 1.33 (6H, s), 2.13 (6H, s), 2.29 (3H, s), 3.72 (4H, s), 4.13 (2H, s), 6.98 (2H, s), 7.49-7.53 (3H, m), 7.92-7.89 (2H, m).
MS m/z: 559(M+H)⁺.

### [Example 51]

### (1) tert-Butyl 2-[2,6-dimethyl-4-[[(5-methyl-2-phenyloxazol-4-ylmethyl)-(5-oxo-4,5-dihydro-[1,3,4]oxadiazol-2-ylmethyl)amino]methyl]phenoxy]-2-methylpropanoate

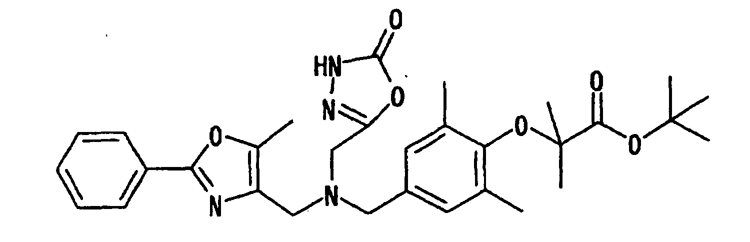

In the same manner as in Example 17-(1), the entitled compound (0.052 g) was obtained as a pale yellow oily substance from the compound (0.100 g) obtained in Reference Example 41-(2).
MS m/z: 563(M+H)⁺.

### (2) tert-Butyl 2-[2,6-dimethyl-4-[[(4-methyl-5-oxo-4,5-dihydro-[1,3,4]oxadiazol-2-ylmethyl)-(5-methyl-2-phenyloxazol-4-ylmethyl)amino]methyl]phenoxy]-2-methylpropanoate

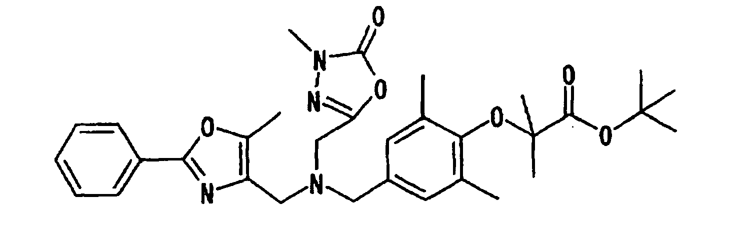

The compound (0.052 g) obtained in Example 51-(1), methanol (0.50 ml) and triphenylphosphine (0.038 g) were dissolved in tetrahydrofuran (5 ml), and at -78°C, diisopropyl azodicarboxylate (0.03 ml) was dropwise added thereto, then the resulting solution was warmed up to room temperature and stirred for 19 hours. The reaction solution was diluted with ethyl acetate, washed with water and then with saturated brine, and dried over anhydrous sodium sulfate. The solvent was evaporated under reduced pressure, and the resulting residue was purified by silica gel column chromatography (ethyl acetate/hexane = 2/1) to obtain the entitled compound (0.055 g) as a pale yellow oily substance.
¹H-NMR (400 MHz, CDCl₃) δ: 1.41 (6H, s), 1.51 (9H, s), 2.21 (6H, s), 2.28 (3H, s), 3.37 (3H, s), 3.64 (2H, s), 3.65 (2H, s), 3.72 (2H, s), 6.97 (2H, s), 7.42-7.45 (3H, m), 8.01-7.98 (2H, m).
MS m/z: 577(M+H)⁺.

### (3) 2-[2,6-Dimethyl-4-[[(4-methyl-5-oxo-4,5-dihydro-[1,3,4]oxadiazol-2-ylmethyl)-(5-methyl-2-phenyloxazol-4-ylmethyl)amino]methyl]phenoxy]-2-methylpropanoic acid

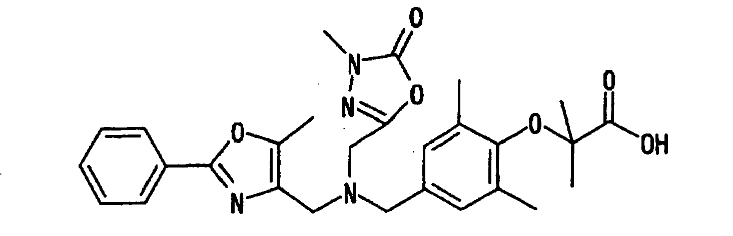

In the same manner as in Example 50-(2), a hydrochloride of the entitled compound (0.036 g) was obtained as a colorless solid from the compound (0.052 g) obtained in Example 51-(2).
¹H-NMR (400 MHz, DMSO-d₆) δ: 1.34 (6H, s), 2.15 (6H, s), 2.30 (3H, s), 3.27 (3H, s), 3.56-3.71 (4H, m), 3.87-3.95 (2H, m), 7.07 (2H, s), 7.52-7.54 (3H, m), 7.95-7.92 (2H, m).
MS m/z: 521(M+H)⁺.

| Elementary Analysis, as C₂₈H₃₂N₄O₆•HCl• 1/2H₂O | | | |
|---|---|---|---|
| Calculated: | C,59.41; | H,6.05; | N,9.90. |
| Found: | C,59.60; | H,6.15; | N,9.69. |

### [Example 52]

### (1) tert-Butyl 2-[2,6-difluoro-4-[[(5-methyl-2-phenyloxazol-4-ylmethyl)-oxazol-2-ylmethylamino]methyl]phenoxy]-2-methylpropanoate

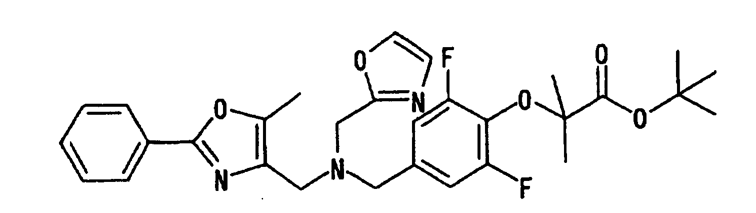

In the same manner as in Example 13-(1), the entitled compound (0.201 g) was obtained as a colorless oily substance from the compound (0.223 g) obtained in Reference Example 43 and the compound (0.100 g) obtained in Reference Example 13-(3).
¹H-NMR (400 MHz, CDCl₃) δ: 1.48 (9H, s), 1.49 (6H, s), 2.32 (3H, s), 3.68 (2H, s), 3.71 (2H, s), 3.90 (2H, s), 7.00 (2H, d, J=8.6 Hz), 7.09 (1H, s), 7.38-7.46 (3H, m), 7.65 (1H, d, J=0.5 Hz), 8.03-7.98 (2H, m).
MS m/z: 554(M+H)⁺.

### (2) 2-[2,6-Difluoro-4-[[(5-methyl-2-phenyloxazol-4-ylmethyl)-oxazol-2-ylmethylamino]methyl]phenoxy]-2-methylpropanoic acid

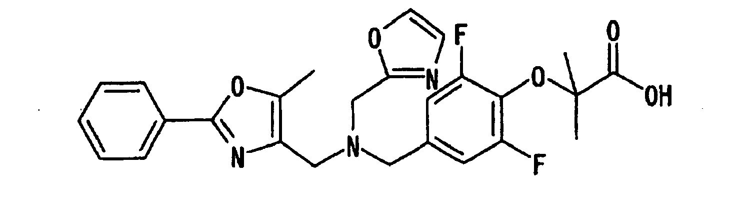

In the same manner as in Example 5-(2), a hydrochloride of the entitled compound (0.150 g) was obtained as a colorless solid from the compound (0.201 g) obtained in Example 52-(1).
MS m/z: 498(M+H)⁺.

| Elementary Analysis, as C₂₆H₂₅F₂N₃O₅•HCl | | | |
|---|---|---|---|
| Calculated: | C,58.49; | H,4.91; | N,7.87. |
| Found: | C,58.38; | H,4.87; | N,7.69. |

### [Example 53]

### (1) tert-Butyl 2-[4-methoxy-3-[[(5-methyl-2-phenyloxazol-4-ylmethyl)-oxazol-2-ylmethylamino]methyl]phenoxy]-2-methylpropanoate

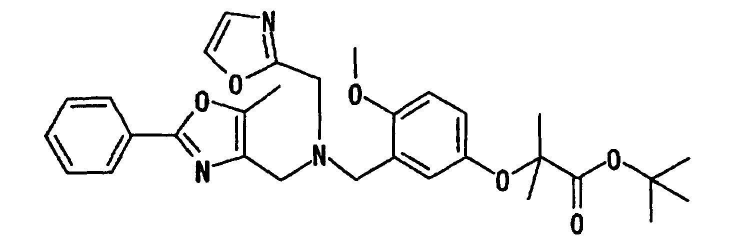

In the same manner as in Example 13-(1), the entitled compound (0.183 g) was obtained as a colorless oily substance from the compound (0.219 g) obtained in Reference Example 44 and the compound (0.100 g) obtained in Reference Example 13-(3).
¹H-NMR (400 MHz, CDCl₃) δ: 1.44 (9H, s), 1.50 (6H, s), 2.31 (3H, s), 3.69 (2H, s), 3.71 (3H, s), 3.75 (2H, s), 3.92 (2H, s), 6.69 (1H, d, J=8.8 Hz), 6.77 (1H, dd, J=8.8, 2.9 Hz), 7.06 (1H, s), 7.17 (1H, d, J=2.9 Hz), 7.45-7.36 (3H, m), 7.62 (1H, s), 8.02-7.98 (2H, m).
MS m/z: 548(M+H)⁺.

### (2) 2-[4-Methoxy-3-[[(5-methyl-2-phenyloxazol-4-ylmethyl)-oxazol-2-ylmethylamino]methyl]phenoxy]-2-methylpropanoic acid

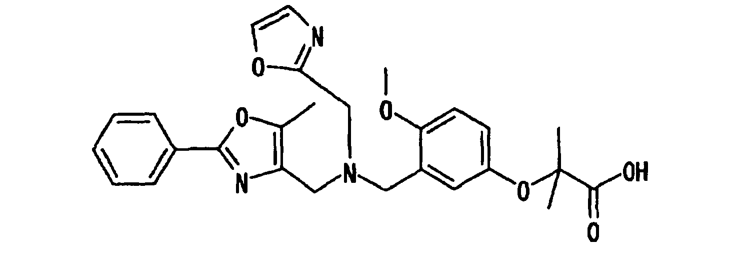

The compound (0.181 g) obtained in Example 53-(1) was dissolved in dichloromethane (3 ml), and 4 N hydrochloric acid-dioxane solution (5 ml) was added thereto and the resulting solution was stirred for a day at room temperature. The solvent was evaporated under reduced pressure, and the resulting residue was purified by silica gel column chromatography (methanol/chloroform = 1/19) to obtain the entitled compound (0.090 g) as a colorless solid.
¹H-NMR (400 MHz, CDCl₃) δ: 1.58 (6H, s), 2.35 (3H, s), 3.57 (2H, s), 3.75 (3H, s), 3.81 (2H, s), 3.86 (2H, s), 6.74 (1H, d, J=8.6 Hz), 6.86 (1H, d, J=7.6 Hz), 7.11 (1H, s), 7.49-7.43 (3H, m), 7.64 (1H, s), 7.72 (1H, d, J=2.7 Hz), 8.05-7.99 (2H, m).
MS m/z: 492(M+H)⁺.

| Elementary Analysis, as C₂₇H₂₉N₃O₆•1/2H₂O | | | |
|---|---|---|---|
| Calculated: | C,64.79; | H,6.04; | N,8.39. |
| Found: | C,64.66; | H,6.03; | N,8.09. |

### [Example 54]

### (1) Ethyl 2-[2,6-dimethyl-4-[[(3-methyl-[1,2,4]oxadiazol-5-ylmethyl)-(5-methyl-2-p-tolyl-oxazol-4-ylmethyl)amino]methyl]phenoxy]-2-methylpropanoate

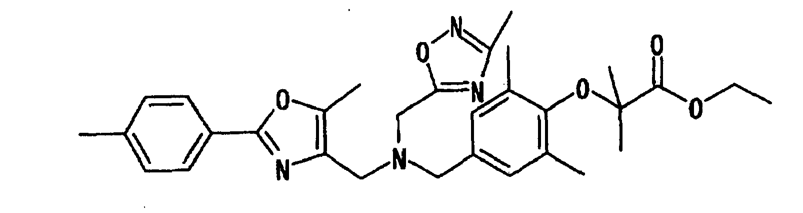

In the same manner as in Example 31-(1), the entitled compound (0.090 g) was obtained as a pale yellow oily substance from the compound (0.150 g) obtained in Reference Example 45-(4) and 4-chloromethyl-5-methyl-2-p-tolyloxazole (0.075 g).
¹H-NMR (400 MHz, CDCl₃) δ: 1.35 (3H, t, J=7.1 Hz), 1.45 (6H, s), 2.17 (6H, s), 2.26 (3H, s), 2.39 (6H, br s), 3.66 (2H, s), 3.69 (2H, s), 4.02 (2H, s), 4.28 (2H, q, J=7.1 Hz), 6.99 (2H, s), 7.23 (2H, d, J=8.5 Hz), 7.88 (2H, d, J=8.1 Hz).
MS m/z: 547(M+H)⁺.

### (2) 2-[2,6-Dimethyl-4-[[(3-methyl-[1,2,4]oxadiazol-5-ylmethyl)-(5-methyl-2-p-tolyl-oxazol-4-ylinethyl)amino]methyl]phenoxy]-2-methylpropanoic acid

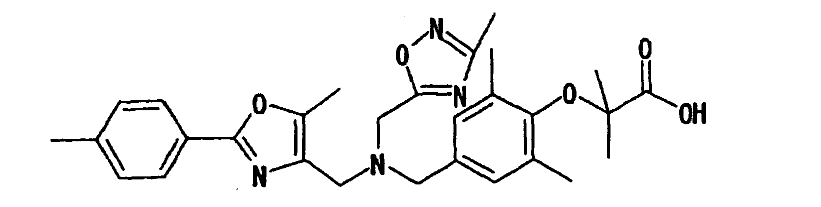

In the same manner as in Example 31-(2), the entitled compound (0.047 g) was obtained as a colorless oily substance from the compound (0.090 g) obtained in Example 45-(1).
¹H-NMR (400 MHz, CDCl₃) δ: 1.50 (6H, s), 2.22 (6H, s), 2.28 (3H, s), 2.39 (3H, s), 2.41 (3H, s), 3.69 (2H, s), 3.71 (2H, s), 4.03 (2H, s), 7.04 (2H, s), 7.22-7.24 (2H, m), 7.87-7.90 (2H, m).
MS m/z: 519(M+H)⁺.

### [Example 55]

### Ethyl 2-[2,6-dimethyl-4-[[[5-methyl-2-(5-methylthiophen-2-yl)oxazol-4-ylmethyl]-(3-methyl-[1,2,4]oxadiazol-5-ylmethyl)amino]methyl]phenoxy]-2-methylpropanoate

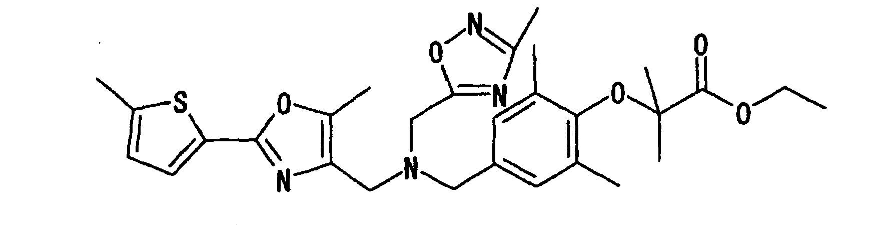

In the same manner as in Example 31-(1), the entitled compound (0.055 g) was obtained as a pale yellow oily substance from the compound (0.150 g) obtained in Example 45-(4) and the compound (0.077 g) obtained in Reference Example 32.
¹H-NMR (400 MHz, CDCl₃) δ: 1.35 (3H, t, J=7.2 Hz), 1.45 (6H, s), 2.18 (6H, s), 2.23 (3H, s), 2.41 (3H, s), 2.51 (3H, s), 3.64 (2H, s), 3.66 (2H, s), 4.01 (2H, s), 4.28 (2H, q, J=7.2 Hz), 6.73 (1H, dd, J=3.7, 1.2 Hz), 6.99 (2H, s), 7.39 (1H, d, J=3.7 Hz).
MS m/z: 553(M+H)⁺.

### [Example 56]

### (1) Ethyl 2-[4-[[cyclopropylcarbamoylmethyl-(5-methyl-2-phenyloxazol-4-ylmethyl)amino]methyl]-2,6-dimethylphenoxy]-2-methylpropanoate

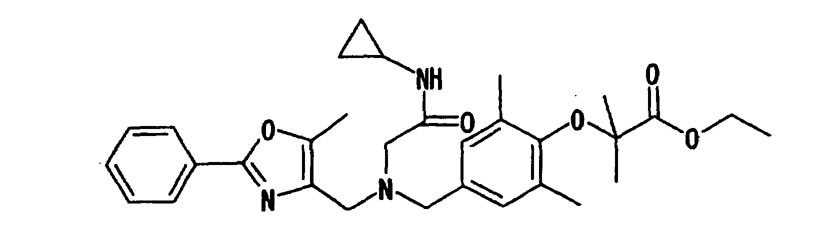

In the same manner as in Example 14-(1), the entitled compound (0.110 g) was obtained as a pale yellow oily substance from the compound (0.150 g) obtained in Reference Example 14-(3) and cyclopropylamine (0.030 ml).
¹H-NMR (400 MHz, CDCl₃) δ: 0.48-0.52 (2H, m), 0.71-0.76 (2H, m), 1.26 (1H, br s), 1.35 (3H, t, J=7.1 Hz), 1.44 (6H, s), 2.16 (6H, s), 2.26 (3H, s), 3.18 (2H, s), 3.48 (2H, s), 3.57 (2H, s), 4.28 (2H, q, J=7.1 Hz), 6.86 (2H, s), 7.44-7.48 (3H, m), 7.89 (1H, br s), 8.00-8.02 (2H, m).
MS m/z: 534(M+H)⁺.

### (2) 2-[4-[[Cyclopropylcarbamoylmethyl-(5-methyl-2-phenyloxazol-4-ylmethyl)amino]methyl]-2,6-dimethylphenoxy]-2-methylpropanoic acid

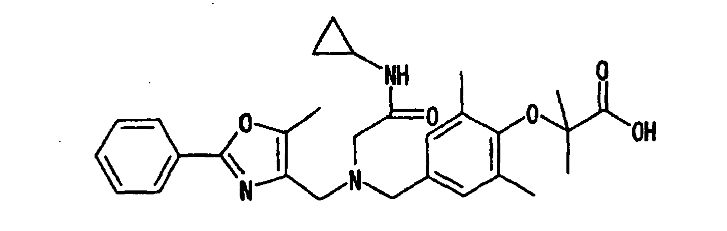

In the same manner as in Example 31-(2), the entitled compound (0.076 g) was obtained as a colorless solid from the compound (0.110 g) obtained in Example 56-(1).
¹H-NMR (400 MHz, DMSO-d₆) δ: 0.36-0.40 (2H, m), 0.59-0.63 (2H, m), 1.32 (6H, s), 2.11 (6H, s), 2.25 (3H, s), 2.66-2.68 (1H, m), 3.06(2H, s), 3.53 (2H, s), 3.57 (2H, s), 6.94 (2H, s), 7.50-7.55 (3H, m), 7.83 (1H, d, J=4.2 Hz), 7.95-7.92 (2H, m).
MS m/z: 506(M+H)⁺.

| Elementary Analysis, as C₂₉H₃₅N₃O₅•3/4H₂O | | | |
|---|---|---|---|
| Calculated: | C,67.10; | H,7.09; | N,8.09. |
| Found: | C,67.21; | H,6.60; | N,7.93. |

### [Example 57]

### (1) Ethyl 2-[4-[[[(cyclopropylmethylcarbamoyl)methyl]-(5-methyl-2-phenyloxazol-4-ylmethyl)amino]methyl]-2,6-dimethylphenoxy]-2-methylpropanoate

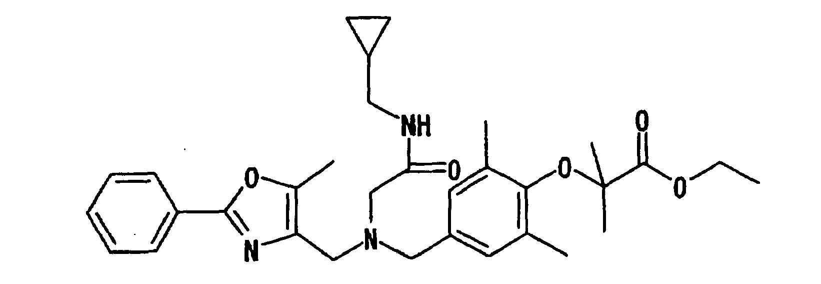

In the same manner as in Example 14-(1), the entitled compound (0.075 g) was obtained as a pale yellow oily substance from the compound (0.150 g) obtained in Reference Example 14-(3) and cyclopropylmethylamine (0.040 ml).
¹H-NMR (400 MHz, CDCl₃) δ: 0.19-0.21 (2H, m), 0.44-0.48 (2H, m), 0.92-0.97 (1H, m), 1.35 (3H, t, J=7.1 Hz), 1.45 (6H, s), 2.17 (6H, s), 2.27 (3H, s), 3.10-3.13 (2H, m), 3.22 (2H, s), 3.53 (2H, s), 3.60 (2H, s), 4.28 (2H, q, J=7.1 Hz), 6.93 (2H, s), 7.42-7.46 (3H, m), 7.81 (1H, br s), 8.01-7.98 (2H, m).
MS m/z: 548(M+H)⁺.

### (2) 2-[-4-[[[(Cyclopropylmethylcarbamoyl)methyl]-(5-methyl-2-phenyloxazolylmethyl)amino]methyl]-2,6-dimethylphenoxy]-2-methylpropanoic acid

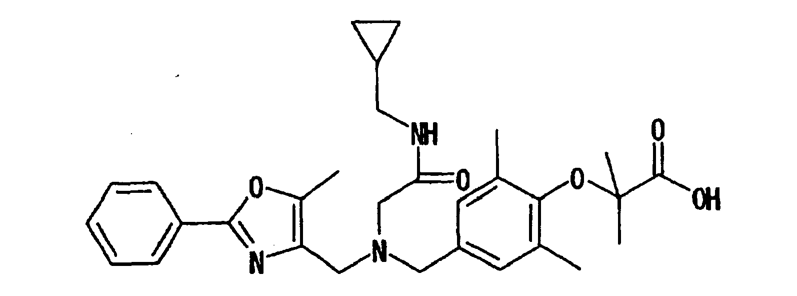

In the same manner as in Example 31-(2), the entitled compound (0.058 g) was obtained as a colorless solid from the compound (0.075 g) obtained in Example 57-(1).
¹H-NMR (400 MHz, DMSO-d₆) δ: 0.12-0.16 (2H, m), 0.34-0.39 (2H, m), 1.32 (6H, s), 2.13 (6H, s), 2.26 (3H, s), 2.66-2.68 (1H, m), 2.97 (2H, t, J=6.3 Hz), 3.10 (2H, s), 3.54 (2H, s), 3.58 (2H, s), 7.00 (2H, s), 7.49-7.55 (3H, m), 7.85 (1H, t, J=5.6 Hz), 7.95-7.92 (2H, m).
MS m/z: 520(M+H)⁺.

| Elementary Analysis, as C₃₀H₃₇N₃O₅•1/2H₂O | | | |
|---|---|---|---|
| Calculated: | C,68.16; | H,7.25; | N,7.95. |
| Found: | C,68.22; | H,6.93; | N,7.86. |

### [Example 58]

### (1) Ethyl 2-[4-[[butylcarbamoylmethyl-(5-methyl-2-phenyloxazol-4-ylmethyl)amino]methyl]-2,6-dimethylphenoxy]-2-methylpropanoate

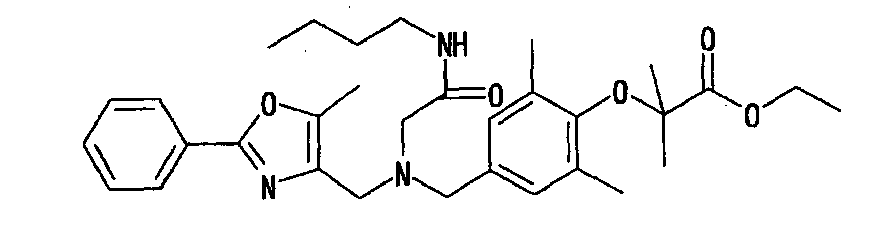

In the same manner as in Example 14-(1), the entitled compound (0.120 g) was obtained as a pale yellow oily substance from the compound (0.150 g) obtained in Reference Example 14-(3) and butylamine (0.042 ml).
¹H-NMR (400 MHz, CDCl₃) δ: 0.88 (3H, t, J=7.4 Hz), 1.30-1.36 (5H, m), 1.44 (6H, s), 1.47-1.53 (2H, m), 2.17 (6H, s), 2.27 (3H, s), 3.19-3.25 (4H, m), 3.51 (2H, s), 3.59 (2H, s), 4.28 (2H, q, J=7.1 Hz), 6.90 (2H, s), 7.43-7.46 (3H, m), 7.78 (1H, br s), 8.01-7.99 (2H, m).
MS m/z: 550(M+H)⁺.

### (2) 2-[4-[[Butylcarbamoytmethyl-(5-methyl-2-phenyloxazol-4-ylmethyl)amino]methyl]-2,6-dimethylphenoxy]-2-methylpropanoic acid

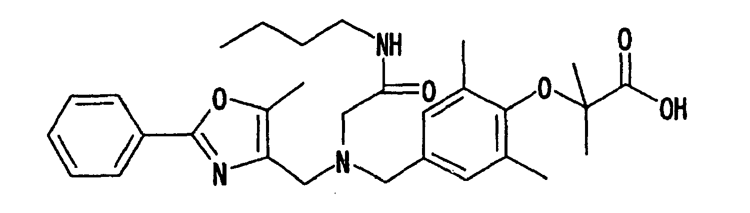

In the same manner as in Example 31-(2), the entitled compound (0.069 g) was obtained as a colorless solid from the compound (0.120 g) obtained in Example 58-(1).
¹H-NMR (400 MHz, DMSO-d₆) δ: 0.81 (3H, t, J=7.2 Hz), 1.19-1.28 (2H, m), 1.32 (6H, s), 1.33-1.41 (2H, m), 2.12 (6H, s), 2.26 (3H, s), 3.04-3.09 (4H, m), 3.54 (2H, s), 3.58 (2H, s), 6.98 (2H, s), 7.50-7.55 (3H, m), 7.79 (1H, t, J=6.1 Hz), 7.92-7.95 (2H, m).
MS m/z: 522(M+H)⁺.

| Elementary Analysis, as C₃₀H₃₉N₃O₅•1/2H₂O | | | |
|---|---|---|---|
| Calculated: | C,67.90; | H,7.60; | N,7.92. |
| Found: | C,68.09; | H,7.23; | N,7.87. |

### [Example 59]

### (1) Ethyl 2-[2,6-dimethyl-4-[[(5-methyl-2-phenyloxazol-4-ylmethyl)-(5-pyridin-3-yl-[1,3,4]oxadiazol-2-ylmethyl)amino]methyl]phenoxy]-2-methylpropanoate

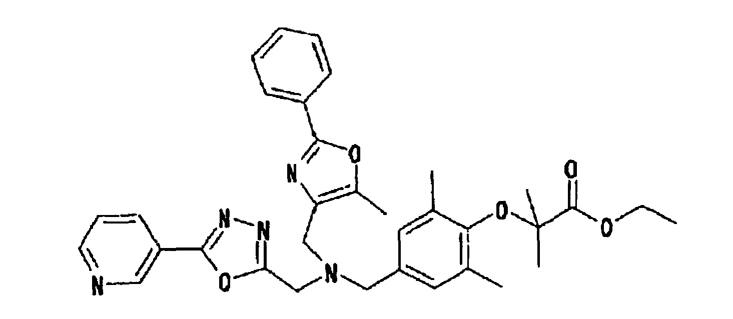

In the same manner as in Example 18, the entitled compound was obtained as a mixture with triphenylphosphine oxide (0.62 g) from the compound (0.170 g) obtained in Reference Example 46.
¹H-NMR (400 MHz, CDCl₃) δ: 1.35 (3H, t, J = 7.2 Hz), 1.45 (6H, s), 2.18 (6H, s), 2.31 (3H, s), 3.75 (6H, br s), 4.28 (2H, q J = 7.1 Hz), 7.03 (2H, s), 7.40-7.42 (3H, m), 7.96-7.99 (2H, m), 8.33-8.30 (1H, m), 8.76 (1H, dd, J = 4.7, 1.7 Hz), 9.26 (1H, d, J = 1.5 Hz).
MS m/z: 596(M+H)⁺.

### (2) 2-[2,6-Dimethyl-4-[[(5-methyl-2-phenyloxazol-4-ylmethyl)-(5-pyridin-3-yl-[1,3,4]oxadiazol-2-ylmethyl)amino]methyl]phenoxy]-2-methylpropanoic acid

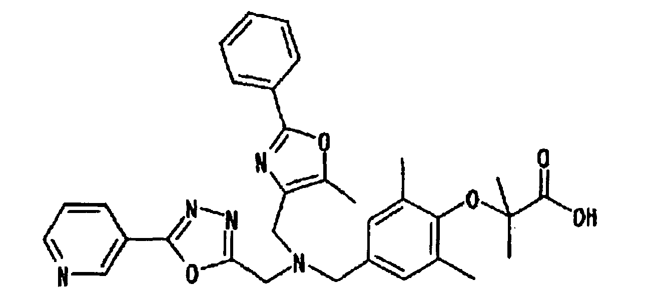

In the same manner as in Example 31-(2), the entitled compound (0.065 g) was obtained as a colorless solid from the mixture (0.62 g) obtained in Example 59-(1).
¹H-NMR (400 MHz, DMSO-d₆) δ: 1.28 (6H, s), 2.10 (6H, s), 2.29 (3H, s), 3.69 (2H, s), 3.71 (2H, s), 4.06 (2H, s), 7.00 (2H, s), 7.44-7.46 (3H, m), 7.59 (1H, dd, J = 8.0, 4.8 Hz), 7.86-7.84 (2H, m), 8.28 (1H, d, J = 7.8 Hz), 8.76 (1H, d, J = 3.2 Hz), 9.09 (1H, s).
MS m/z: 568(M+H)⁺.

### [Example 60]

### 2-[4-[[Carbamoylmethyl-(5-methyl-2-phenyloxazol-4-ylmethyl)amino]rnethyl]-2,6-dimethylphenoxy]-2-methylpropionic acid

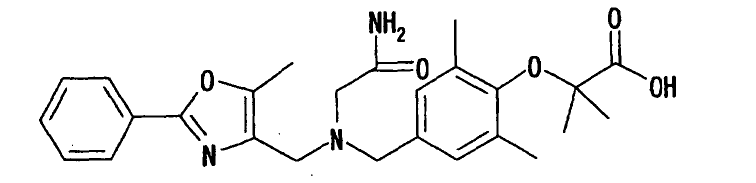

In the same manner as in Example 31-(2), the entitled compound (0.080 g) was obtained as a colorless solid from the compound (0.119 g) of Example 19-(1).
¹H-NMR (400 MHz, DMSO-d₆) δ: 1.32 (6H, s), 2.13 (6H, s), 2.24 (3H, s), 3.07 (2H, s), 3.53 (2H, s), 3.57 (2H, s), 7.15 (1H, br s), 7.31 (1H, br s), 7.49-8.53 (3H, m), 7.94-7.91 (2H, m).
MS m/z: 466(M+H)⁺.

### [Experimental Example 1]

### GAL4-hPPAR Transactivation Assay

(a) Plasmids:
   Plasmids for the expression of fusion protein for GAL4 DNA binding region-PPAR ligand binding region, pFA-hPPARα/GAL4 and pFA-hPPARγ/GAL4, were constructed by inserting the LBD cDNA of hPPARα and hPPARγ, respectively, into a commercially-available expression vector (pFA trans-activator plasmid, by STRATAGENE) having a GAL4 DNA binding region (GAL4 DBD) of yeast, under CMV promoter. For the expression of a reporter protein, used were a commercially-available plasmid (pFR-SEAP, by STRATAGENE) having a GAL4 responsive region (GAL4 UAS) in the upstream of cDNA of a secreted alkaline phosphatase (SEAP).

(b) Cell Culture and Transactivation Assay:
   HEK293T cells were suspended in a high-glucose Dulbecco's modified Eagle's medium (DMEM) containing 10% fetal calf serum (by Hyclone), 100 U/mL penicillin G and 100 mg/mL streptomycin sulfate, and seeded in a 24-well cell culture plate in a density of 8 × 10⁴ cells/well. The cells were cultured in a humidified atmosphere of 5% CO₂ at 37°C for 24 hours, and then subjected to transfection using Lipofectamine (by Invitrogen) and Plus reagent (by Invirogen) according to the manufacture's instruction in the absence of serum. Concretely, the cells were incubated in 225 µL of a transfection medium (OPTI-MEM, by Invitrogen) containing 0.48 µL of Lipofectamine, 0.030 µg of pFA-PPAR/GAL4 expression plasmid and 0.13 µg of pFR-SEAP, in a 5% CO₂ atmosphere at 37°C for 5 hours. Next, the same volume of a fresh high-glucose DMEM containing 10% fetal calf serum, 100 U/mL penicillin G, 100 mg/mL streptomycin sulfate and a test compound of two times the designated concentration was added to it, and the cells were incubated for about 48 hours. After the compound was solubilized in DMSO, control cells were incubated along with an equivalent concentration of DMSO. The final DMSO concentration was at most 0.1 %, and it is known that the concentration does not have any influence on the transactivation activity. After the incubation, the culture supernatant was collected, and using a kit (reporter assay kit - SEAP, by TOYOBO), the SEAP activity was determined according to the manufacturer's instruction. Concretely, the same amount of an intrinsic alkaline phosphatase inhibitor was added to 5 µL of the culture supernatant and incubated at 37°C for 30 minutes, and then 100 µL of a chemoluminescent substrate (Lumiphos PLUS, by Lumigen) was added thereto and incubated at 37°C for 15 minutes, and thereafter the luminescence level was determined using a multi-label counter (ARVOsx, by Perkin Elmer). The values obtained as a result of the above operation and the concentrations of the test compound were plotted to obtain the EC₅₀ value.

### <Test Result>

As in the Table below, the compounds of the invention show a strong GAL4-hPPAR transactivation activity.

**Table 1**

| Example No. | GAL4-hPPAR Transactivation Activity (µM) | |
|---|---|---|
| | PPARα | PPARγ |
| Example 1-(2) | 0.013 | 0.59 |
| Example 4-(2) | 0.0048 | 0.18 |
| Example 5-(2) | 0.0026 | 0.22 |
| Example 13-(2) | 0.0037 | 0.0055 |
| Example 16-(2) | 0.0024 | 0.0088 |
| Example 18-(2) | 0.0029 | 0.033 |
| Example 19-(3) | 0.0027 | 0.0069 |
| SKB-compd | 0.044 | 0.048 |

### [Experimental Example 2] Determination of Solubility

### <Test Method>

One mg of a test compound was put into a test tube, and 1 ml of the first fluid for Japanese Pharmacopoeia Disintegration Test Method (synthetic gastric juice, pH 1.2: hereinafter referred to as JP-fluid 1) or the second fluid for Japanese Pharmacopoeia Disintegration Test Method (synthetic intestinal juice, pH 6.8: hereinafter referred to as JP-fluid 2) was added thereto and shaken, and then statically left at room temperature for 12 hours or more. After thus left, the sample was filtered through a 0.45-µm membrane filter, and the filtrate was suitably diluted with a mixture of DMSO-purified water (1/1) to prepare solutions for solubility determination.
Apart from the above, 1 mg of the test compound was put into a 20-mL messflask, and dissolved in a mixture of DMSO-purified water (1/1) to prepare a standard solution of 50 µg/mL. The standard solution was stepwise diluted to give serial dilutions, and using the dilutions, a calibration curve was formed.
The above-mentioned solutions for solubility determination with the JP-fluid 1 or the JP-fluid 2 were analyzed through HPLC, and based on the calibration curve formed in the above, the solubility of the test compound was computed.

The HPLC condition is as follows:
HPLC System for Alliance Assay (by Waters, USA),
Feed Pump: 2795 separation module,
UV Detector: 2996 photodiode array detector,
Column: ODS-type C18 column (3.5 µm, 3.0 mm ID × 30 mm),
Column Temperature: 60°C,
Mobile Phase A: pH 4.5, 10 mM acetate buffer,
Mobile Phase B: 50% acetic acid-acetonitrile mixture (1/999),
Gradient Condition: mobile phase A/B ratio, 95/5 to 10/90,
Flow Rate: 1.5 mL/min,
Sample Temperature: 25°C,
Sample Amount: 5 µL,
Detection Wavelength: maximum absorption wavelength within a range of 220 to 420 nm.

### <Test Result>

As in the Table below, the compounds of the invention had good solubility in JP-fluid 1 and JP-fluid 2.

**Table 2**

| Example No. | Solubility | (µg/mL) |
|---|---|---|
| | JP-fluid 1 | JP-fluid 2 |
| Example 1-(2) | 490 | > 1000 |
| Example 4-(2) | 930 | < 3 |
| Example 5-(2) | 520 | 680 |
| Example 13-(2) | > 870 | > 870 |
| Example 16-(2) | > 490 | 480 |
| Example 18-(2) | > 710 | > 710 |
| Example 19-(3) | 650 | > 710 |
| SKB-compd | 240 | 10 |

### [Experimental Example 3] Lipid Solubility

### <Test Method>

An equi-volume mixture of n-octanol (hereinafter referred to as octanol) and pH 7.4 isotonic phosphate buffer (hereinafter referred to as aqueous phase) was shaken in a suitable vessel, then statically left for 24 hours or more, and the upper layer (water-saturated octanol) and the lower layer (octanol-saturated water) were separately collected.
1 mg of a test compound was put into a test tube, and 5 ml of the water-saturated octanol was added thereto, ultrasonicated for 10 minutes and then stirred for about 30 seconds. In case where an insoluble substance was seen, then the liquid was filtered through a 0.45-µm membrane filter. 2 mL of the water-saturated octanol solution of the test compound was taken into a test tube, 2 mL of the octanol-saturated water was added thereto and shaken for 30 minutes. Then, this was centrifuged to separate into an upper layer (octanol phase) and a lower layer (aqueous phase), which were then separately collected.
The octanol phase and the aqueous phase were suitably diluted with DMSO, and the concentration of the test compound therein was determined through UV-detection HPLC or MS-detection HPLC.
From the concentration measured in each phase and the dilution ratio of each phase, the partition coefficient was derived through computation.
In case where the solubility in water of the test compound is estimated to be high, then the test compound is dissolved in the octanol-saturated water and then the water-saturated octanol is added thereto, shaken and centrifuged, and the upper layer (octanol phase) and the lower layer (aqueous phase) are separately collected to determine the concentration of the test compound.

The details of the HPLC apparatus and its operation condition are as follows:

### <UV Detection HPLC System>

HPLC System for Alliance Assay (by Waters, USA),
Feed Pump: 2795 separation module,
UV Detector: 2996 photodiode array detector.

### <MS Detection HPLC System>

High Throughput LC/MS System (by Agilent, USA),
Feed Pump: 1100 series/binary pump,
MS Detector: 1100 series LC/MSD SL.

### <Test Condition>

Column: ODS-type C18 column (3.5 µm, 3.0 mm ID × 30 mm),
Column Temperature: 60°C,
Mobile Phase A: pH 4.5, 10 mM acetate buffer,
Mobile Phase B: 50% acetic acid-acetonitrile mixture (1/999),
Gradient Condition: mobile phase A/B ratio, 95/5 to 10/90,
Flow Rate: 1.5 mL/min,
Sample Temperature: 25°C,
Sample Amount: 5 µL,
Detection Wavelength: maximum absorption wavelength within a range of 220 to 420 nm.

The MS detection condition is as follows:
Mode: SIM,
Drying Gas Flow: 12 L/min,
Nebulizer Pressure: 55 psig.,
Capillary Voltage: 3000 V,
Drying Gas Temperature: 350°C,
Fragmenter voltage: 100V.

### <Test Result>

As in the following Table, the compounds of the invention had good lipid solubility favorable for drugs.

**Table 3**

| Example No. | log D (pH 7.4) |
|---|---|
| Example 1-(2) | 2.7 |
| Example 4-(2) | 2.5 |
| Example 5-(2) | 2.3 |
| Example 13-(2) | 1.8 |
| Example 16-(2) | 2.6 |
| Example 18-(2) | 1.1 |
| Example 19-(3) | 1.8 |
| SKB-compd | 4.4 |

The invention has been described in detail with reference to specific embodiments thereof, and it is obvious to those skilled in the art that various changes and modifications may be given thereto not overstepping the spirit and the scope of the invention.
This application is based on Japanese Patent Application (Tokugan 2004-99201) filed March 30, 2004 and Japanese Patent Application (Tokugan 2005-15954) filed January 24, 2005, and the entire contents thereof being incorporated herein by reference.

### Industrial Applicability

The compounds, their salts and their solvates of the invention have excellent PPAR α/γ agonist effects, and are useful as preventives/remedies for diabetes.

## Claims

1. A compound of the general formula (I): 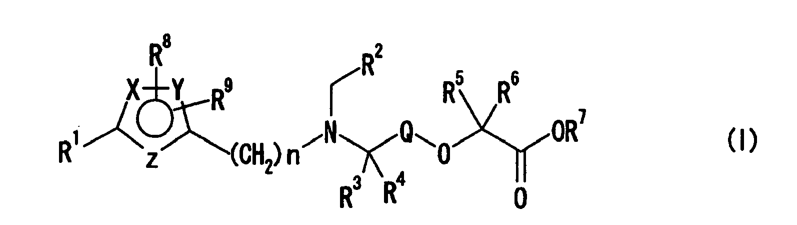 (wherein;
Q represents a benzene ring or a pyridine ring which may be substituted with one or two, the same or different groups selected from a hydroxyl group, a halogen atom, a lower alkenyl group, a lower alkoxy group, a substituted or unsubstituted lower alkyl group, a substituted or unsubstituted amino group, a pyrrolidinyl group, a piperidinyl group, a piperazinyl group, a morpholinyl group, a substituted or unsubstituted phenyl group and a substituted or unsubstituted pyridyl group;
R¹ represents a phenyl group, a pyridyl group, a pyrimidinyl group, a pyridazinyl group, a pyrazinyl group, a thienyl group, a furyl group, a pyrrolyl group, an imidazolyl group, a pyrazolyl group, a thiazolyl group, an oxazolyl group, an isothiazolyl group, an isoxazolyl group, an oxadiazolyl group or a triazolyl group, which may be substituted with one or two, the same or different groups selected from a halogen atom, a lower alkenyl group, a lower alkoxy group, a phenoxy group, a substituted or unsubstituted lower alkyl group and a substituted or unsubstituted amino group;
R² represents a pyridyl group, a pyrimidinyl group, a pyrazinyl group, a pyridazinyl group, an imidazolyl group, a pyrazolyl group, a thiazolyl group, an oxazolyl group, an isothiazolyl group, an isoxazolyl group, an oxadiazolyl group, a triazolyl group, a quinolyl group, an isoquinolyl group, a quinazolyl group, a cinnolyl group, a quinoxalyl group, a phthalazinyl group, a naphthyridinyl group, an indolyl group, a benzimidazolyl group, an indazolyl group, a benzothiazolyl group, a benzoxazolyl group, a benzisothiazolyl group, a benzisoxazolyl group or a benzotriazolyl group, which may be substituted with one or two, the same or different groups selected from a hydroxyl group, a halogen atom, a lower alkenyl group, a lower alkoxy group, a substituted or unsubstituted lower alkyl group, a substituted or unsubstituted amino group, a substituted or unsubstituted phenyl group and a substituted or unsubstituted pyridyl group, or represents a carbamoyl group which may be substituted with one or two, the same or different lower alkyl groups;
X, Y and Z each independently represent C, O, S or N (provided that at least any one of X, Y and Z is O, S or N);
R³ to R⁶ each independently represent a hydrogen atom or a lower alkyl group, or R³ and R⁴, or R⁵ and R⁶ may together form a 3- to 6-membered saturated ring along with the carbon atom substituted with them;
R⁷, R⁸ and R⁹ each independently represent a hydrogen atom or a lower alkyl group; n indicates an integer of from 0 to 3), a salt thereof, or a solvate thereof.

2. A compound of the general formula (I) wherein the 5-membered ring containing X, Y and Z is an oxazole ring, a thiazole ring or an oxadiazole ring, a salt thereof, or a solvate thereof.

3. The compound, a salt thereof, or a solvate thereof as claimed in claim 1 or 2, wherein Q is a benzene ring which may be substituted with one or two, the same or different groups selected from a hydroxyl group, a halogen atom, a lower alkenyl group, a lower alkoxy group, a substituted or unsubstituted lower alkyl group, a substituted or unsubstituted amino group, a pyrrolidinyl group, a piperidinyl group, a piperazinyl group, a morpholinyl group, a substituted or unsubstituted phenyl group and a substituted or unsubstituted pyridyl group.

4. The compound, a salt thereof, or a solvate thereof as claimed in claim 1 or 2, wherein Q is a benzene ring which may be substituted with one or two, the same or different groups selected from a halogen atom, a lower alkenyl group, a lower alkoxy group and a substituted or unsubstituted lower alkyl group.

5. The compound, a salt thereof, or a solvate thereof as claimed in any of claims 1 to 4, wherein R¹ is a phenyl group which may be substituted with one or two, the same or different groups selected from a halogen atom, a lower alkenyl group, a lower alkoxy group, a phenoxy group, a substituted or unsubstituted lower alkyl group and a substituted or unsubstituted amino group.

6. The compound, a salt thereof, or a solvate thereof as claimed in any of claims 1 to 4, wherein R¹ is a phenyl group which may be substituted with one or two, the same or different groups selected from a halogen atom and a substituted or unsubstituted lower alkyl.

7. The compound, a salt thereof, or a solvate thereof as claimed in any of claims 1 to 6, wherein R² is a pyridyl group, an imidazolyl group, a pyrazolyl group, a thiazolyl group, an oxazolyl group, an isothiazolyl group, an isoxazolyl group, an oxadiazolyl group, a benzimidazolyl group, an indazolyl group, a benzothiazolyl group, a benzoxazolyl group, a benzisothiazolyl group or a benzisoxazolyl group which may be substituted with one or two, the same or different groups selected from a hydroxyl group, a halogen atom, a lower alkenyl group, a lower alkoxy group, a substituted or unsubstituted lower alkyl group, a substituted or unsubstituted amino group, a substituted or unsubstituted phenyl group and a substituted or unsubstituted pyridyl group.

8. The compound, a salt thereof, or a solvate thereof as claimed in any of claims 1 to 6, wherein R² is a pyridyl group, an imidazolyl group, a pyrazolyl group, a thiazolyl group, an oxazolyl group, an isothiazolyl group, an isoxazolyl group, an oxadiazolyl group, a benzimidazolyl group, a benzothiazolyl group or a benzoxazolyl group which may be substituted with one or two, the same or different groups selected from a halogen atom, a lower alkoxy group, a substituted or unsubstituted lower alkyl group and a substituted or unsubstituted phenyl group.

9. The compound, a salt thereof, or a solvate thereof as claimed in any of claims 1 to 6, wherein R² is a carbamoyl group which may be substituted with one or two, the same or different lower alkyl groups.

10. A medicament comprising, as the active ingredient thereof, a compound of the general formula (I), a salt thereof, or a solvate thereof as described in claim 1.

11. A pharmaceutical composition comprising a compound of the general formula (I), a salt thereof, or a solvate thereof as described in claim 1, and a pharmaceutically acceptable carrier.

12. Use of a compound of the general formula (I), a salt thereof, or a solvate thereof as described in claim 1, for production of a medicament.
